(19) Europäisches Patentamt
European Patent Office
Office européen des brevets

(11) Publication number: 0 099 121 B1

(12) # EUROPEAN PATENT SPECIFICATION

(45) Date of publication of patent specification: 25.10.89

(51) Int. Cl.$^4$: **A 61 K 37/64,** A 61 K 45/06 // (A61K37/64, 31:41)

(21) Application number: 83106846.5

(22) Date of filing: 12.07.83

(54) **Pharmaceutical compositions.**

(30) Priority: 12.07.82 US 397271

(43) Date of publication of application: 25.01.84 Bulletin 84/04

(45) Publication of the grant of the patent: 25.10.89 Bulletin 89/43

(84) Designated Contracting States: AT BE CH DE FR GB IT LI LU NL SE

(56) References cited:
GB-A-2 067 987
US-A- 101 650

(73) Proprietor: Bristol-Myers Company
345 Park Avenue
New York New York 10154 (US)

(72) Inventor: Buyniski, Joseph P.
302 Rugby Road
Syracuse New York (US)
Inventor: Cavanagh, Robert L.
7684 Stonehedge Lane
Manlius New York (US)
Inventor: Gordon, Maxwell
513 Standish Drive
Syracuse New York (US)

(74) Representative: Kinzebach, Werner, Dr.
Patentanwälte Reitstötter, Kinzebach und Partner Sternwartstrasse 4 Postfach 86 06 49
D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 099 121 B1

**Description**

In the treatment of peptic ulcers in warm-blooded animals, the concomitant administration of the pepsin-complexing agent, pepstatin, and an histamine $H_2$-receptor antagonist of the formula I:

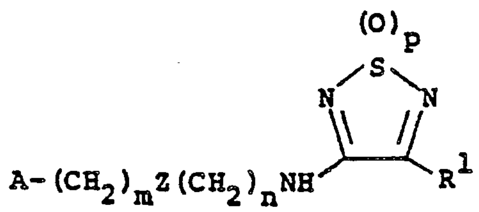

I

wherein A, m, Z, n, p and $R^1$ are as defined below, provides enhanced antiulcer activity, reduces the amount of the compound of Formula I necessary for effective treatment and thereby reduces the side effect liability of the compound of formula I. This invention relates to pharmaceutical compositions containing pepstatin and at least one compound of Formula I for treating peptic ulcers in warm-blooded animals.

The precise cause of peptic ulceration in man is unknown although gastric acid is considered to be one of the essential factors in the etiology of this disease. It recently was discovered that gastric acid secretion is mediated, at least in part, by histamine $H_2$-receptors located on parietal cells in the gastric mucosa and that gastric acid output induced by and secretagogues could be antagonized by specific antagonists of these receptors [Black, J. W. *et al.,* Nature, *236,* 385—390 (1972); Brimblecombe, R. W. *et al.,* J. Int. Med. Res., *3,* 86—92 (1975)]. The first successful commercial histamine $H_2$-receptor antagonist, cimetidine (II),

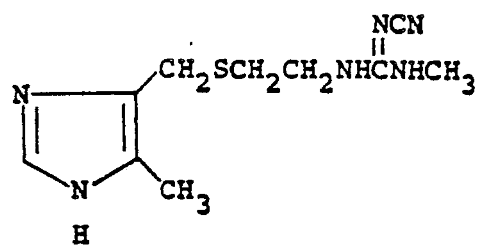

II

is now in widespread use as an antiulcer agent. A more recently introduced histamine $H_2$-receptor antagonist, ranitidine (III),

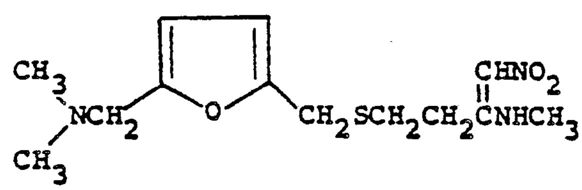

III

is now being sold and used in several countries of the world.

The role of the proteolytic enzyme, peptin, in the etiology of ulceration is not completely understood. Pepsin has been shown to play a major role in the development of experimentally induced ulcers in animals, but this may be due to lesion enlargement by means of pepsin digestion of necrotic tissue rather than by causing the initial damage. It is also possible that pepsin is entirely responsible for the erosions and that the acid produces pain and retards healing.

1) Umezawa, H. *et al.,* in J. Antibiotics, *23,* 259—262 (1970), disclose the pentapeptide, pepstatin, which has the structure

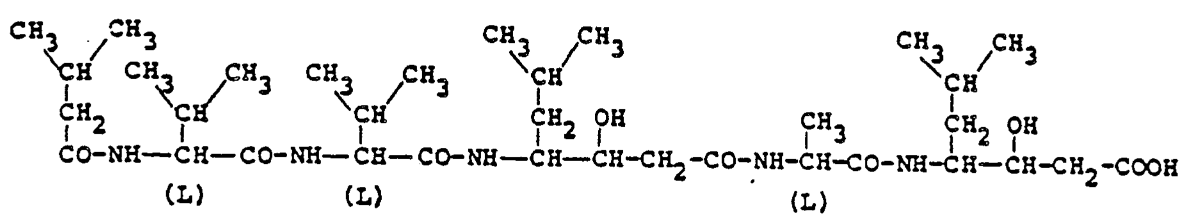

and which is a specific complexing agent for the enzyme pepsin. Pepstatin was found to prevent the formation of stomach ulcers in the pylorus ligated (Shay) rat.

2) Miyawaki, *et al.,* in Nagano-ken Noygo Sogo Shikenjo Chikusan Shikenjo Kenkyu Hokoku, *14,* 14—25 (1977) [as reported in Chemical Abstracts, *90,* 34373X (1979)] report that the addition of pepstatin to the

feed at >50 ppm inhibited pepsin activity in the gastric juice of swine and prevented the occurrence of ulcers.

3) Bonnevie, O. *et al.*, in Gut, *20,* 624—628 (1979), report the results of a double-blind randomized clinical trial of pepstatin versus placebo in patients having duodenal ulcers. Pepstatin was administered in 100 mg doses, given seven times a day, this dosage being sufficient to inhibit the peptic activity of gastric juice for 18 hours a day. They found no significant difference betwen pepstatin and placebo in the healing or symptomatology of duodenal ulcer.

4) Svendsen, L. B. *et al.*, in Scand. J. Gastroent., *14,* 929—932 (1979), report the results of a double-blind randomized clinical trial of pepstatin versus placebo in patients having gastric ulcer. Pepstatin was administered in 100 mg doses seven times a day. They were not able to detect any influence of pepstatin either on the healing or on the symptomatology of gastric ulcer.

5) Strauss, R. J. *et al.*, in Surg. Forum, *28,* 361—363 (1977), disclose that, in stress ulceration tests in rats, two or more days of pretreatment with either cimetidine or carbenoxolone significantly decreased ulcer formation. When the two agents were given together, significant ulcer reduction was found after only one-half day of predosing. Carbenoxolone is not an antisecretory or anti-pepsin agent, but acts by stimulating gastric mucus synthesis.

6) Dajani, E. Z. *et al.*, in J. Pharmacol. Exp. Ther., *210,* 373—377 (1979), disclose that, in stress ulceration tests in rats, a combination of cimetidine and propantheline bromide produced synergistic antiulcer activity and that a combination of cimetidine and thiopropazate hydrochloride produced additive antiulcer activity. Propantheline bromide (an anticholinergic agent) and thiopropazate hydrochloride (a tranquilizer) each act by inhibiting gastric secretion.

7) British Medical Journal, 95—96 (1980) reviews the results obtained in a large number of studies with various new antiulcer agents. With regard to pepsin antagonists, it states:

> "The results of using pepsin antagonists have been uniformly disappointing. Amylopectin showed no significant benefit in patients with duodenal ulcer, and sucralfate showed none in those with gastric ulcer. Even pepstatin, the most potent in-vitro and in-vivo pepsin antagonist, was ineffective in a formal controlled trial in healing duodenal ulcer and in preventing recurrent bleeding in patients admitted with haematemesis and melaena".

8) US—A—4,101,650 discloses long-acting pepstatin floating minicapsules comprising center particles of sodium bicarbonate coated with a water-soluble film-coating agent, which are further coated with pepstatin and a water-soluble film coating agent. Because of the release of carbon dioxide in gastric juice, these minicapsules float in the stomach and provide pepsin suppression for 3—5 hours as compared with about 1 hour for plain pepstatin.

9) Published United Kingdom Patent Application GB—A—2,067,987 discloses the synthesis of a large number of the histamine $H_2$-receptor antagonists of Formula I herein which are utilized in the methods and compositions disclosed and claimed in the present invention. However, it does not disclose that such compounds may be administered concomitantly with pepstatin and thereby provide enhanced antiulcer activity.

This invention relates to a pharmaceutical composition comprising a mixture of the pepsin-inhibiting agent, pepstatin, and at least one histamine $H_2$-receptor antagonist of the formula

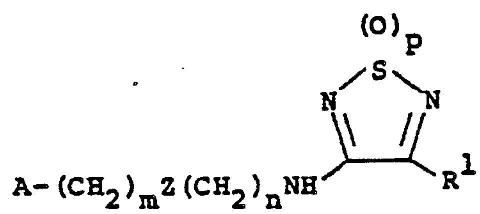

I

wherein

p is 1 or 2;

$R^1$ is hydroxy or $NR^2R^3$;

$R^2$ and $R^3$ each are independently hydrogen, $C_1$—$C_6$-alkyl, $C_2$—$C_6$-alkenyl, $C_2$—$C_6$-alkynyl, cyclo-$C_3$—$C_7$-alkyl-$C_1$—$C_6$-alkyl, hydroxy-$C_1$—$C_6$-alkyl, $C_1$—$C_6$-alkoxy-$C_1$—$C_6$-alkyl, $C_1$—$C_6$-alkylthio-$C_1$—$C_6$-alkyl, 2-fluoroethyl, 2,2,2-trifluoroethyl or cyano-$C_1$—$C_6$-alkyl, or, when $R^2$ is hydrogen, $R^3$ may also be cyclo-$C_3$—$C_7$-alkyl, amino-$C_1$—$C_6$-alkyl, $C_1$—$C_6$-alkylamino-$C_1$—$C_6$-alkyl, di-$C_1$—$C_6$-alkylamino-$C_1$—$C_6$-alkyl, pyrrolidino-$C_1$—$C_6$-alkyl, piperidino-$C_1$—$C_6$-alkyl, morpholino-$C_1$—$C_6$-alkyl, piperazino-$C_1$—$C_6$-alkyl, pyridyl-$C_1$—$C_6$-alkyl, substituted pyridyl-$C_1$—$C_6$-alkyl wherein the pyridyl ring may contain one substituent selected from $C_1$—$C_6$-alkyl, $C_1$—$C_6$-alkoxy, hydroxy, amino and halogen, amino, $C_1$—$C_6$-alkylamino, di-$C_1$—$C_6$-alkylamino, hydroxy, $C_1$—$C_6$-alkoxy, 2,3-dihydroxypropyl, cyano, amidino, $C_1$—$C_6$-alkylamidino, $A'$—$(CH_2)_{m'}Z'(CH_2)_{n'}$—, phenyl, phenyl-$C_1$—$C_6$-alkyl, substituted phenyl or substituted phenyl-$C_1$—$C_6$-alkyl, wherein the phenyl ring may contain one or two substituents independently selected from $C_1$—$C_6$-alkyl, hydroxy, $C_1$—$C_6$-alkoxy and halogen or one substituent selected from methylenedioxy, trifluoromethyl and di-$C_1$—$C_6$-alkylamino; or $R^2$ and $R^3$, taken together, may be —$CH_2CH_2X(CH_2)_r$—;

r is an integer of from 1 to 3, inclusive;

X is methylene, sulfur, oxygen or N—$R^4$, provided that, when r is 1, X is methylene;

$R^4$ is hydrogen, $C_1$—$C_6$-alkyl, $C_2$—$C_6$-alkenyl, $C_2$—$C_6$-alkynyl, $C_1$—$C_6$-alkanoyl or benzoyl;

m and m′ each are independently an integer of from zero to 2, inclusive;

n and n′ each are independently an integer of from 2 to 4, inclusive;

Z and Z′ each are independently sulfur, oxygen or methylene;

A and A′ each are independently phenyl, imidazolyl, thiazolyl, isothiazolyl, oxazolyl, isoxazolyl, triazolyl, thiadiazolyl, oxadiazolyl, furyl, thienyl or pyridyl; provided that A and A′ independently may contain one or two substituents, the first substituent being selected from $C_1$—$C_6$-alkyl, hydroxy, trifluoromethyl, halogen, amino, hydroxymethyl, $C_1$—$C_6$-alkoxy,

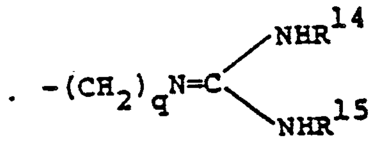 and $-(CH_2)_qNR^5R^6$,

and the second substituent being selected from $C_1$—$C_6$-alkyl, hydroxy, trifluoromethyl, halogen, amino, hydroxymethyl and $C_1$—$C_6$-alkoxy;

q is an integer of from 0 to 6, inclusive; $R^{14}$ and $R^{15}$ independently are hydrogen or $C_1$—$C_6$-alkyl, or, if $R^{14}$ is hydrogen, $R^{15}$ also may be $C_1$—$C_6$-alkanoyl or benzoyl, or $R^{14}$ and $R^{15}$, taken together, may be ethylene; and

$R^5$ and $R^6$ each are independently hydrogen, $C_1$—$C_6$-alkyl, $C_2$—$C_6$-alkenyl, $C_2$—$C_6$-alkynyl, $C_1$—$C_6$-alkoxy-$C_1$—$C_6$-alkyl, cyclo-$C_3$—$C_7$-alkyl, phenyl or phenyl-$C_1$—$C_6$-alkyl, provided that $R^5$ and $R^6$ may not both be cyclo-$C_3$—$C_7$-alkyl or phenyl; or $R^5$ and $R^6$, taken together with the nitrogen atom to which they are attached, may be pyrrolidino, methylpyrrolidino, dimethylpyrrolidino, morpholino, thiomorpholino, piperidino, methylpiperidino, dimethylpiperidino, hydroxypiperidino, N-methylpiperazino, homopiperidino, heptamethyleneimino or octamethyleneimino;

or a nontoxic, pharmaceutically acceptable salt, hydrate or solvate thereof.

The compounds of the present invention are useful in treating peptic ulcers in a warm-blooded animal. Such treatment comprises concomitantly administering to said animal a peptic activity-inhibiting amount of pepstatin and an effective antiulcerogenic amount of at least one compound of Formula I, or a pharmaceutically acceptable acid addition salt, hydrate or solvate thereof. Thus a further aspect of the present invention concerns the use of at least one compound of formula I in combination with pepstatin for preparing a pharmaceutical composition for the treatment of peptic ulcers.

The improvement in the treatment of peptic ulcers in a warm-blooded animal by the administration to said animal of an effective antiulcerogenic amount of at least one compound of Formula I, or a pharmaceutically acceptable acid addition salt, hydrate or solvate thereof, comprises reducing the amount of the compound of Formula I necessary for effective treatment by concomitantly administering to said animal a peptic activity-inhibiting amount of pepstatin.

The compounds of Formula I are not themselves our invention, but are the invention of our colleagues, Aldo A. Algieri and Ronnie R. Crenshaw.

The compounds of Formula I are relatively nontoxic substances, as demonstrated by pharmacological studies in animals of some of the compounds. These studies showed a toxicity profile substantially the same as that of the commercial $H_2$-antagonist cimetidine. Although widespread human usage of cimetidine has demonstrated it to be a relatively safe drug with a low incidence of side-effects, it would, of course, be desirable to even further reduce the side-effect liability of such a drug. Some of the preferred compounds of Formula I have been shown by various animal studies to be up to 400 times as potent as cimetidine as inhibitors of gastric secretion, depending on the animal model and route of administration. The preferred compounds of Examples 86 and 88 are 30 and 15, respectively, times more potent than cimetidine by the oral route. Based on this potency difference, the oral dosage of the compounds of Examples 86 and 88 would be about 1/30 and 1/15, respectively, that of cimetidine, thus reducing the expected incidence of side effects. The usual oral dosage of cimetidine is 300 mg, given four times a day, while the usual dosage of the compounds of Examples 86 and 88 is 10 mg and 20 mg, respectively, given four times a day. It was an object of this invention to further reduce the necessary dosage of the compounds of Formula I by the concomitant administration of a peptic activity-inhibitory amount of pepstatin. As will be shown below, such concomitant administration provides about a two- to three-fold increase in potency compared with the administration of an equal amount of the compound of Formula I alone, thus permitting a further two- to three-fold reduction in the dosage of the compound of Formula I.

Pepstatin has also been shown by pharmacological studies in animals to be a relatively nontoxic substance; its $LD_{50}$ exceeded 3000 mg/kg in all animal species studied [Svendsen, L. B. *et al.*, Scand. J. Gastroent., *11,* 459—463 (1976)]. It is essentially unabsorbed upon oral administration; no side effects were observed in human patients with ulcer dyspepsia receiving daily oral doses of 700 mg of pepstatin for up to three months [Svendsen, L. B. *et al.* (1976) supra]. Pepstatin does not inhibit the production of pepsin but inhibits peptic activity by forming a 1:1 pepsin-pepstatin complex which is devoid of proteolytic activity.

In patients with ulcer dyspepsia, it has been demonstrated that pepstatin inhibits gastric peptic activity, but has no effect on the gastric acidity [Svendsen, L. B. *et al.,* Scan. J. Gastroent., *11,* 459—463 (1976)]. In

contrast, the histamine $H_2$-receptor antagonist cimetidine has been shown to antagonize both basal and stimulated gastric acid secretion in normal volunteers [Burland, W. L. *et al.,* Brit. J. Clin. Pharmacol., *2,* 481—486 (1975)] and in patients with duodenal ulcer [Longstreth, G. F. *et al.,* New England J. Med., *294,* 801—804 (1976)], but its effect on pepsin secretion is less marked [Binder, H. J. and Donaldson Jr., R. M., Gastroenterology, *74,* 371—375 (1978)]. The results of those studies indicate that pepstatin and cimetidine act by different mechanisms. The inhibitory effect of the compounds of Formula I on gastric acid secretion is also significantly greater than on pepsin activity, and in this respect its pharmacological profile is similar to that of cimetidine.

In the tests described below, gastric erosions produced in rats by the oral instillation of 1.0 mL of 0.75 N HCl were compared with those in rats which had been pretreated with the most preferred compound of Formula I (of Example 1) or with that compound and pepstatin. Comparison tests utilizing pretreatment with the newer commercial product, ranitidine, and with ranitidine and pepstatin, are also shown. Ranitidine is about 4 and 6 times as potent as cimetidine as an inhibitor of gastric secretion, by the oral route, in the rat and dog, respectively.

## Experimental Methods

A modification of the method of Robert *et al.,* [Gastroenterology *77,* 433—443 (1979)] was employed to produce gastric erosions. Adult male, Long Evans rats weighing 280—300 g (Blue Spruce Farms, Alton, New York) were used. The animals were individually caged and food and water were removed 24 and 18 hours, respectively, prior to testing. On the following day, the test compound was administered orally to the animals 30 minutes before 1.0 mL of 0.75 N HCl was instilled into the stomach by gavage. Animals treated with the combination of the test compound and pepstatin received the test compound 30 minutes before, and a fixed amount of pepstatin (20 mg/kg po) 10 minutes before, the hydrochloric acid was administered. Previous studies in our laboratories had shown that this dose of pepstatin (20 mg/kg po) completely inhibited pepsin activity and antagonized ulcer formation in the 18 hour pylorus ligated rat. One hour after receiving the HCl solution, the animals were sacrificed with an intraperitoneal injection of 0.2 mL of T-61®, a euthanasia solution (National Laboratories Corp.).

The stomachs were removed from the animals, cut along the greater curvature, opened, rinsed with saline and pinned out flat in a standard position for macroscopic examination and scoring of erosions. The stomachs were photographed with a Polaroid® Close Up camera (Polaroid Corporation) and scoring was determined from the photographs. For scoring purposes, only those erosions with a minimum length of 1 mm were considered. The severity of gastric ulceration was defined for each animal as the sum of the maximum continuous lengths (in mm) of the erosions satisfying the above criteria. Percent inhibition of lesion formation was defined as

$$\frac{\text{(mm erosion, vehicle control)} - \text{(mm erosion, test agent)}}{\text{(mm erosion, vehicle control)}} \times 100.$$

Data were analyzed using the t-test for unpaired data and $ED_{50}$ values were calculated from the dose response data using probit analysis [Finney, Probit Analysis, 3rd ed., University Press, Cambridge, England (1971)].

The compound of Example 1 and ranitidine (synthesized by the Medicinal Chemistry Research Department of Bristol Laboratories, Division of Bristol-Myers Company) were dissolved in one equivalent of HCl and the pH adjusted to 5.5 with NaOH. A suspension of pepstatin (Banyu Pharmaceutical Co. Ltd.) in water was made by homogenizing the compound with a few drops of Tween-80® (Atlas Chemical Industries). Each of these compounds were administered orally by gavage in a volume of 2 mL/kg.

## Test Results

The instillation of HCl to untreated rats caused extensive gastric erosions consisting of elongated bands 1—10 mm long by 1—3 mm wide. These erosions were located primarily in the corpus (portion of the stomach which secretes acid and pepsin), while the antrum was not as severely affected and no lesions were observed in the forestomach (the non-secretory portion). These findings are similar to those reported by Robert *et al.,* in their initial description of this procedure.

Pretreatment of the rats with 25, 50 or 75 mg/kg of the compound of Example 86 prior to instillation of the HCl decreased the formation of gastric erosions in a dose-related manner. Pretreatment of the rats with the above amounts of the compound of Example 86 plus 20 mg/kg of pepstatin significantly enhanced the inhibitory effect of the compound of Example 86 when the latter was given at 25 mg/kg. An enhancement was also seen at 50 mg/kg, but the significance level was between .05 and .10. A small, non-significant enhancement was observed at 75 mg/kg. Figure 1 shows, in graphic form, the percent of reduction in gastric erosions over that of the control rats (no pretreatment) which was obtained at each of the dosage levels of the compound of Example 86 alone and with pepstatin. When the data shown in Figure 1 were analyzed by probit analysis according to Finney, it was shown that the response to the compound of Example 86 was linear with respect to the log of the dose administered and that the addition of pepstatin shifted the dose response to the left in a parallel manner. These data are shown in Figure 2. It may be seen

from Figure 2 that the $ED_{50}$ values for the compound of Example 86 alone and with pepstatin were found to be 82 and 46 mg/kg, respectively, thus showing that the combination had approximately twice the potency of the compound of Example 86 alone.

Pretreatment of the rats with a 25, 50, 100 or 200 mg/kg dose of the compound of Example 88 prior to instillation of the HCl also decreased the formation of gastric erosions in a dose-related manner. Pretreatment of rats with the above amounts of the compound of Example 88 plus 20 mg/kg of pepstatin significantly enhanced the inhibitory effect of the compound of Example 88 when the latter was dosed at 25 or 50 mg/kg. No further enhancement of the inhibitory effect over that of the compound of Example 88 alone occurred when pepstatin and 100 mg/kg or 200 mg/kg of the compound of Example 88 were used for pretreatment. Figure 3 shows, in graphic form, the percent of reduction in gastric erosions over that of the control rats (no pretreatment) which was obtained at each of the dosage levels of the compound of Example 88 alone and with pepstatin. When the data shown in Figure 3 were analyzed by probit analysis according to Finney, it was shown that the response to the compound of Example 88 was linear with respect to the log of the dose administered. Furthermore, with concomitant administration of pepstatin, the response was also linear with respect to the log dose of the compound of Example 88, but the two lines were not parallel. These data are shown in Figure 4. It may be seen from Figure 4 that the $ED_{50}$ values for the compound of Example 88 alone and with pepstatin were found to be 63 and 25 mg/kg, respectively. At the $ED_{50}$ level, the combination was 2.5 times as potent as the compound of Example 88 alone.

Pretreatment of rats with a 25, 50, 75 or 100 mg/kg dose of ranitidine prior to instillation of the HCl similarly decreased the formation of gastric erosions in a dose-related manner. No enhancement of the inhibitory effect over that of ranitidine alone occurred when pepstatin was given along with ranitidine. Figure 5 shows, in graphic form, the percent reduction in gastric erosions over that of the control rats (no pretreatment), which was obtained at each of the dosage levels of ranitidine alone and ranitidine plus pepstatin. When the data shown in Figure 5 were analyzed by probit analysis according to Finney, it was shown that the response to ranitidine was linear with respect to the log dose of the compound and that the addition of pepstatin shifted the dose response to the left in a parallel manner. These data are shown in Figure 6. It may be seen from Figure 6 that the $ED_{50}$ values for ranitidine alone and the ranitidine-pepstatin combination were found to be 123 and 104 mg/kg, respectively. The ratio of potencies of the combination to ranitidine alone was 1.18, which was not statistically different from unity.

The data from these tests are summarized in Table 1. It may be seen that the $ED_{50}$'s for the compounds of Examples 86 and 88, given alone, were 1.5 and 2 times, respectively, more potent than ranitidine given alone. When each of these compounds were administered with pepstatin, the compounds of Examples 86 and 88 were about 2.3 and 4.2 times, respectively, more potent than ranitidine plus pepstatin.

Table 1

Comparison of the Compound of Example 1 and Ranitidine in Inhibiting HCl-Induced Formation of Gastric Lesions, Alone or in Combination with Pepstatin

| | $ED_{50}$ (mg/kg) for Inhibition of Ulcer Formation | | |
|---|---|---|---|
| Compound | Alone | with Pepstatin | Potency Ratio[a] |
| Compound of Example 88 | 63 | 25 | 2.5[b] |
| Compound of Example 86 | 82 | 46 | 1.8 |
| Ranitidine | 123 | 104 | 1.18 |

[a]Ratio of potency (cpd. alone) to potency of combination (cpd. plus pepstatin).

[b]Defined as ratio of $ED_{50}$ (cpd. of Example 88 alone) to $ED_{50}$ (cpd. of Example 88 plus pepstatin); dose-response lines were nonparallel.

Antagonism of $H_2$-receptors and the subsequent anti-secretory effect probably is not the mechanism of the antiulcer effect in this test, since exogenous HCl is being supplied. Pepstatin is only poorly absorbed following oral administration as animal studies have shown that more than 90 percent of the compound is excreted in the feces within 72 hours. Therefore, the inhibition of proteolytic activity following oral administration of pepstatin is due primarily to a local effect of this compound.

In order to obtain the maximum benefit of the present invention, it is desirable that the dosage of pepstatin be such that there is substantially complete inhibition of gastric pepsin activity for as long a period of the day as practical. When pepstatin was administered to ulcer patients in 100 mg doses seven times a day (with meals, two hours after meals and at bedtime), pepsin activity was inhibited for 18 hours a day.

In one preferred embodiment of this invention, pepstatin is administered in dosages of about 100 mg seven times a day. In another preferred embodiment of this invention, pepstatin is administered in dosages of 175 mg four times a day. In a more preferred embodiment of this invention the pepstatin is administered in the form of floating minicapsules as described in U.S. Patent, i.e. US—A—4,101,650. The pepstatin floating minicapsules provide pepsin suppression for about 3—5 times as long as plain pepstatin and, in this form, may be administered, for example, four times a day in a dosage of floating minicapsules containing about 100 mg of pepstatin.

The dosage of the compounds of Formula I to be administered concomitantly with pepstatin will depend not only on such factors as the weight of the patient, but also on the degree of gastric acid inhibition desired and the potency of the particular compound being utilized. The decision as to the particular dosage to be employed is within the discretion of the physician. In the Heidenhain Pouch Dog test described below, cimetidine has an oral $ED_{50}$ of approximately 3.3 μmoles/kg. The usual human adult oral dose of cimetidine is 300 mg, given four times a day. The usual human adult starting oral dosages of the compounds of Formula I (without pepstatin) are readily determined from their oral $ED_{50}$ in this same test. Thus, if the oral $ED_{50}$ of a particular compound of Formula I is 0.33 μmoles/kg, the usual starting dosage (without pepstatin) would be approximately 30 mg, given four times a day, etc. When administered concomitantly with pepstatin, the usual starting dose would be approximately 15 mg, given four times a day. Similar calculations may be made for parenteral dosages. These starting dosages (and the number of times administered per day) may, of course, be varied by titration of the dosage to the particular circumstances of the specific patient.

It will be appreciated by those skilled in the art that, to obtain the benefits of the present invention, it is not necessary to physically combine the compound of Formula I and pepstatin in a single unitary dosage form. Not only may the two active ingredients be taken separately, but they may even be given by different routes of administration. Although pepstatin provides its effects by local action in the stomach and must be given orally, the compound of Formula I may be given orally or parenterally. For convenience, however, it usually is preferred to administer it orally.

The treatment of peptic ulcers in a warm-blooded animal in need of such treatment comprises concomitantly administering to said animal a peptic activity-inhibiting amount of pepstatin and an effective antiulcerogenic amount of at least one compound of Formula I, or a pharmaceutically acceptable acid addition salt, hydrate or solvate thereof. In man, the usual dosage of the preferred compounds of Formula I is from 2 to 100 mg (and most preferably from 4 to 50 mg), given three or four times (and most preferably four times) a day. With particularly preferred compounds of Formula I, the usual dosage is from 2 to 50 mg, and preferably from 4 to about 25 mg. The preferred dosage of pepstatin in man is from 100 mg when administered about seven times a day, to 175 mg when administered about four times a day. However, in a more preferred embodiment of this invention, when the pepstatin is in the form of pepstatin floating minicapsules, the preferred dosage is that amount of minicapsules containing 100 mg of pepstatin, administered about four times a day.

The improvement in the treatment of peptic ulcers in a warm-blooded animal by administering to said animal an effective antiulcerogenic amount of at least one compound of Formula I or a pharmaceutically acceptable acid addition salt, hydrate or solvate thereof, comprises reducing the amount of the compound of Formula I necessary for effective treatment by concomitantly administering a peptic activity-inhibiting amount of pepstatin. The preferred dosage of pepstatin and of pepstatin floating minicapsules in man is as described in the preceding paragraph.

There is provided by the present invention a pharmaceutical composition useful in the treatment of peptic ulcers which comprises a peptic activity-inhibiting amount of pepstatin and an effective antiulcerogenic amount of at least one compound of Formula I, or a pharmaceutically acceptable acid addition salt, hydrate or solvate thereof, and a pharmaceutically acceptable carrier. In a preferred embodiment the composition contains from 2 to 100 mg (and most preferably from about 4 to 50 mg) of one of the preferred compounds of Formula I and from 100 to 175 mg of pepstatin. In particularly preferred embodiments, the composition contains from 2 to 50 (and most preferably from 4 to 25) mg of the compound of Example 1, plus from 100 to 175 mg of pepstatin. Preferably the compositions according to the invention are in unitary dosage form.

As used herein, reference to a pharmaceutically acceptable acid addition salt of a compound of Formula I means the mono- or di-salt with a nontoxic pharmaceutically acceptable organic or inorganic acid. Such acids are well known and include hydrochloric, hydrobromic, sulfuric, sulfamic, phosphoric,

nitric, maleic, fumaric, succinic, oxalic, benzoic, methanesulfonic, ethanedisulfonic, benzenesulfonic, acetic, propionic, tartaric, citric, camphorsulfonic or levulinic. The salts are made by methods known in the art.

The present invention includes within its scope the use of all possible tautomeric forms, geometric isomers, optical isomers and zwitterionic forms of the compounds of Formula I, as well as mixtures thereof.

In practicing the present invention, a wide variety of pharmaceutical forms may be employed for the administration of the compound of Formula I and pepstatin, or the pharmaceutical composition containing both entities. Thus, if a solid carrier is used, the preparations may be tableted, placed in a hard gelatin capsule in powder or pellet form, or in the form of a troche or lozenge. If a liquid carrier is used, the preparations may be in the form of a soft gelatin capsule, syrup, emulsion, aqueous or non-aqueous suspension or, in the case of the compounds of Formula I, a sterile solution or suspension for injection. The pharmaceutical dosage forms are prepared by conventional techniques.

In a preferred embodiment of the invention the compounds of Formula I have the structure

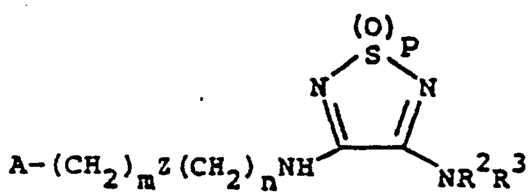

Id

wherein

p is 1 or 2;

$R^2$ and $R^3$ each are independently hydrogen, $C_1$—$C_6$-alkyl, $C_2$—$C_6$-alkenyl, $C_2$—$C_6$-alkynyl or cyclo-$C_3$—$C_7$-alkyl-$C_1$—$C_6$-alkyl, or, when $R^2$ is hydrogen, $R^3$ also may be pyridyl-$C_1$—$C_6$-alkyl, substituted pyridyl-$C_1$—$C_6$-alkyl wherein the pyridyl ring may contain one substituent selected from $C_1$—$C_6$-alkyl, $C_1$—$C_6$-alkoxy, hydroxy, amino and halogen, $A'$—$(CH_2)_{m'}Z'(CH_2)_{n'}$—, phenyl-$C_1$—$C_6$-alkyl or 3,4-methylenedioxybenzyl;

m and m' each are independently zero or 1;

n and n' each are independently 2 or 3;

Z and Z' each are independently sulfur, oxygen or methylene;

A and A' each are independently phenyl, imidazolyl, thiazolyl, furyl, thienyl or pyridyl; provided that A and A' independently may contain one or two substituents, the first substituent being selected from $C_1$—$C_6$-alkyl,

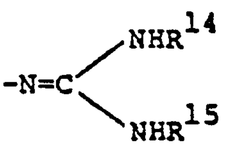

and $-CH_2NR^5R^6$,

and the second substituent being selected from $C_1$—$C_6$-alkyl;

$R^{14}$ and $R^{15}$ independently are hydrogen or $C_1$—$C_6$-alkyl, or $R^{14}$ and $R^{15}$, taken together, may be ethylene; and

$R^5$ and $R^6$ each are independently hydrogen or $C_1$—$C_6$-alkyl; or $R^5$ and $R^6$, taken together with the nitrogen atom to which they are attached, may be pyrrolidino, methylpyrrolidino, dimethylpyrrolidino, morpholino, thiomorpholino, piperidino, methylpiperidino, dimethylpiperidino, hydroxypioperidino, N-methylpiperazino, homopiperidino, heptamethyleneimino or octamethyleneimino;

or a nontoxic, pharmaceutically acceptable salt, hydrate or solvate thereof.

In another preferred embodiment of the invention the compounds of Formula I have the structure

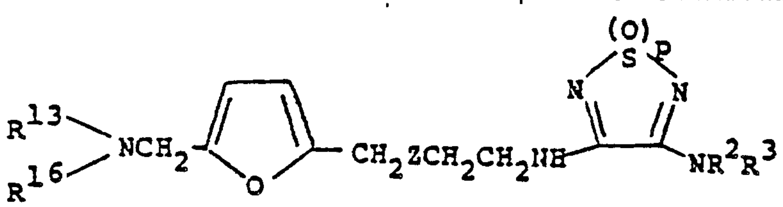

Ie

wherein p is 1 or 2; Z is sulfur or methylene; $R^2$ and $R^3$ each are independently hydrogen or $C_1$—$C_6$-alkyl, or, when $R^2$ is hydrogen, $R^3$ also may be $C_2$—$C_6$-alkenyl, $C_2$—$C_6$-alkynyl, phenyl-$C_1$—$C_6$-alkyl, cyclo-$C_3$—$C_7$-alkyl-$C_1$—$C_6$-alkyl, pyridylmethyl or

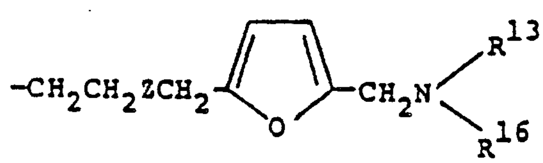

;

$R^{16}$ is methyl and $R^{13}$ is hydrogen or methyl, or $R^{16}$ and $R^{13}$, taken together with the nitrogen atom to which they are attached, may be piperidino; or a nontoxic pharmaceutically acceptable salt, hydrate or solvate thereof.

In another preferred embodiment of the invention the compounds of Formula I have the structure

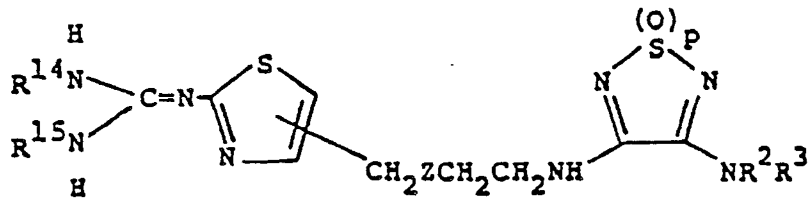

If

wherein p is 1 or 2; Z is sulfur or methylene; $R^{14}$ and $R^{15}$ independently are hydrogen or methyl, or, $R^{14}$ and $R^{15}$, taken together, may be ethylene; and $R^2$ and $R^3$ each are independently hydrogen or $C_1$—$C_6$-alkyl, or, when $R^2$ is hydrogen, $R^3$ also may be $C_2$—$C_6$-alkenyl, $C_2$—$C_6$-alkynyl, pyridylmethyl,

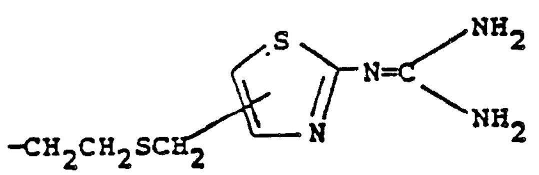

or

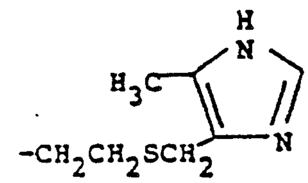

;

or a nontoxic pharmaceutically acceptable salt, hydrate or solvate thereof.

In another preferred embodiment of the invention the compounds of Formula I have the structure

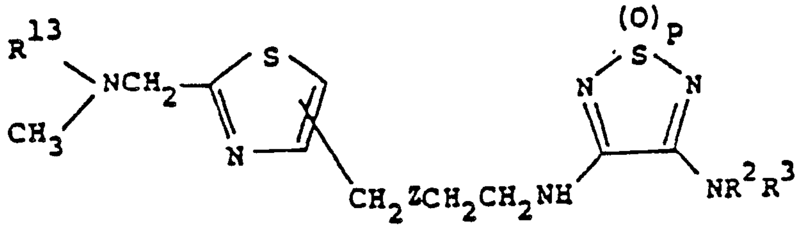

Ig

wherein p is 1 or 2; Z is sulfur or methylene; $R^2$ and $R^3$ each are independently hydrogen or $C_1$—$C_6$-alkyl, or when $R^2$ is hydrogen, $R^3$ also may be $C_2$—$C_6$-alkenyl, $C_2$—$C_6$-alkynyl or

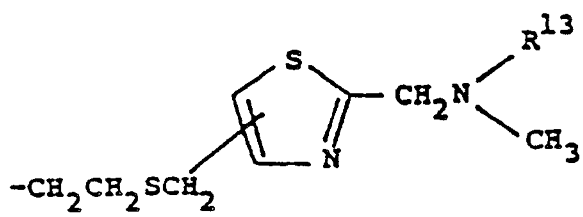

;

and $R^{13}$ is hydrogen or methyl; or a nontoxic pharmaceutically acceptable salt, hydrate or solvate thereof.

In another preferred embodiment of the invention the compounds of Formula I have the structure

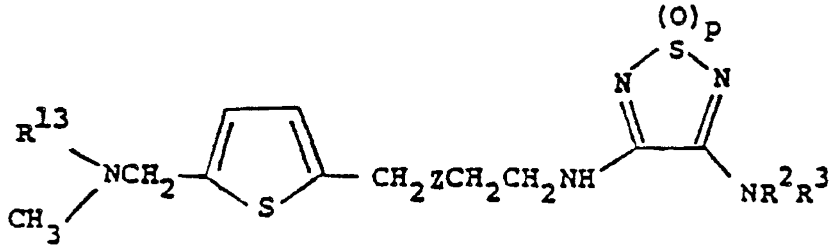

Ih

wherein p is 1 or 2; Z is sulfur or methylene; $R^2$ and $R^3$ each are independently hydrogen or $C_1$—$C_6$-alkyl, or, when $R^2$ is hydrogen, $R^3$ also may be $C_2$—$C_6$-alkenyl, $C_2$—$C_6$-alkynyl, phenyl-$C_1$—$C_6$-alkyl, pyridylmethyl, 3,4-methylenedioxybenzyl or

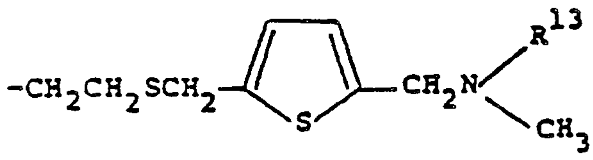

;

and $R^{13}$ is hydrogen or methyl; or a nontoxic pharmaceutically acceptable salt, hydrate or solvate thereof.

In another preferred embodiment of the invention the compounds of Formula I have the structure

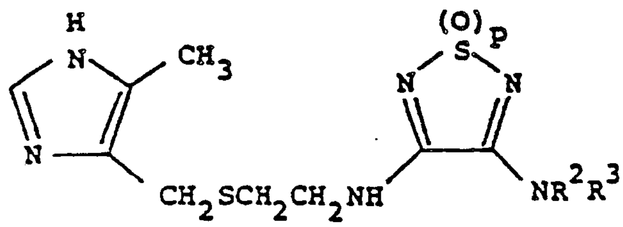

Ii

wherein p is 1 or 2; and $R^2$ and $R^3$ each are independently hydrogen or $C_1$—$C_6$-alkyl, or, when $R^2$ is hydrogen, $R^3$ also may be $C_2$—$C_6$-alkenyl, $C_2$—$C_6$-alkynyl or

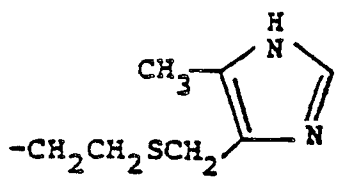

;

or a nontoxic pharmaceutically acceptable salt, hydrate, or solvate thereof.

In another preferred embodiment of the invention the compounds of Formula I have the structure

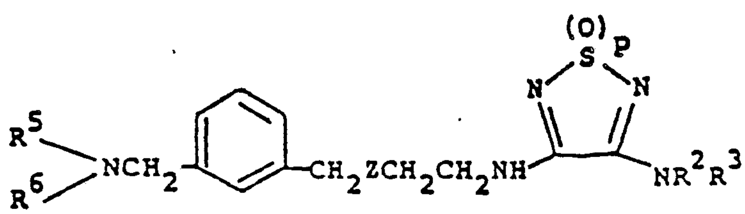

Ij

wherein p is 1 or 2; is sulfur or methylene; $R^2$ and $R^3$ each are independently hydrogen or $C_1$—$C_6$-alkyl, or, when $R^2$ is hydrogen, $R^3$ also may be $C_2$—$C_6$-alkenyl, $C_2$—$C_6$-alkynyl or

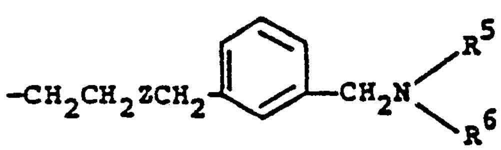

;

and $R^5$ and $R^6$ each are independently hydrogen or $C_1$—$C_6$-alkyl, or, $R^5$ and $R^6$, taken together with the nitrogen atom to which they are attached, may be piperidino; or a nontoxic, pharmaceutically acceptable salt, hydrate or solvate thereof.

In another preferred embodiment of the invention the compounds of Formula I have the structure

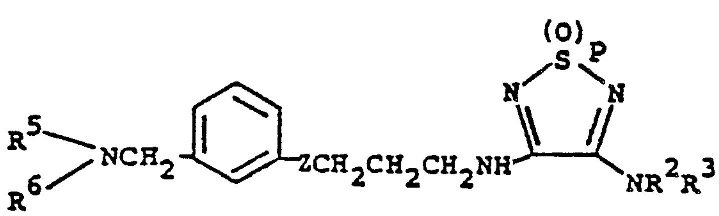

Ik

wherein p is 1 or 2; Z is oxygen or sulfur; $R^2$ and $R^3$ each are independently hydrogen or $C_1$—$C_6$-alkyl, or, when $R^2$ is hydrogen, $R^3$ also may be $C_2$—$C_6$-alkenyl, $C_2$—$C_6$-alkynyl, pyridylmethyl or

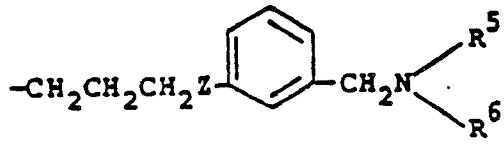

;

and $R^5$ and $R^6$ each are independently hydrogen or $C_1$—$C_6$-alkyl, or, when $R^5$ is hydrogen, $R^6$ also may be $C_2$—$C_6$-alkenyl or $C_2$—$C_6$-alkynyl; or $R^5$ and $R^6$, taken together with the nitrogen to which they are attached, may be pyrrolidino, methylpyrrolidino, morpholino, thiomorpholino, piperidino, methylpiperidino, dimethylpiperidino, homopiperidino or heptamethyleneimino; or a nontoxic pharmaceutically acceptable salt, hydrate or solvate thereof.

In another preferred embodiment of the invention the compounds of Formula I have the structure

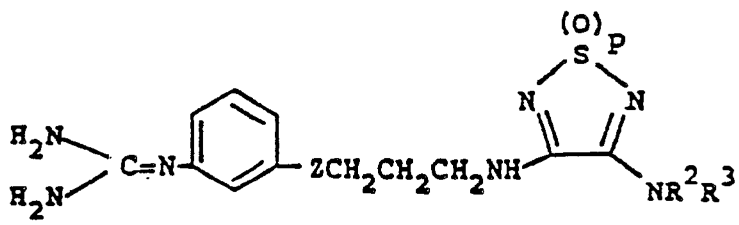

I1

wherein p is 1 or 2; Z is oxygen or sulfur; $R^2$ and $R^3$ each are independently hydrogen or $C_1$—$C_6$-alkyl, or, when $R^2$ is hydrogen, $R^3$ may be $C_2$—$C_6$-alkenyl, $C_2$—$C_6$-alkynyl, pyridylmethyl or

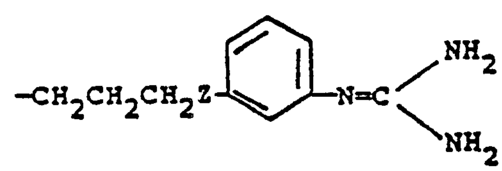

or a nontoxic pharmaceutically acceptable salt, hydrate or solvate thereof.

As presently envisaged, the particularly preferred compounds of Formula I utilized in this invention are

a) 3-{2-[(5-Dimethylaminomethyl-2-furyl)methylthio]ethylamino}-4-methylamino-1,2,5-thiadiazole 1,1-dioxide,

b) 3-{2-[(5-Dimethylaminomethyl-2-furyl)methylthio]ethylamino}-4-methylamino-1,2,5-thiadiazole 1-oxide,

c) 3-{2-[(5-Dimethylaminomethyl-2-furyl)methylthio]ethylamino}-4-ethylamino-1,2,5-thiadiazole 1-1-dioxide,

d) 3-{2-[(5-Dimethylaminomethyl-2-furyl)methylthio]ethylamino}-4-(n-propyl)amino-1,2,5-thiadiazole 1,1-dioxide,

e) 3-Allylamino-4-{2-[(5-dimethylaminomethyl-2-furyl)methylthio]ethylamino}-1,2,5-thiadiazole 1,1-dioxide,

f) 3-{2-[(5-Dimethylaminomethyl-2-furyl)methylthio]ethylamino}-4-(2-propynyl)amino-1,2,5-thiadiazole 1,1,-dioxide,

g) 3-{2-[(5-Dimethylaminomethyl-2-furyl)methylthio]ethylamino}-4-amino-1,2,5-thiadiazole 1,1-dioxide,

h) 3-{2-[(5-Dimethylaminomethyl-2-furyl)methylthio]ethylamino}-4-amino-1,2,5-thiadiazole 1-oxide,

i) 3-Amino-4-[3-(3-pyrrolidinomethylphenoxy)propylamino]-1,2,5-thiadiazole 1,1-dioxide,

j) 3-{4-[(5-Dimethylaminomethyl-2-furyl)butylamino}-4-methylamino-1,2,5-thiadiazole 1,1-dioxide,

k) 3-Methylamino-4-[3-(3-pyrrolidinomethylphenoxy)propylamino]-1,2,5-thiadiazole 1,1-dioxide,

l) 3-{2-[(2-Guanidinothiazol-4-yl)methylthio]ethylamino}-4-methylamino-1,2,5-thiadiazole 1,1-dioxide,

m) 3-{2-[(2-Guanidinothiazol-4-yl)methylthio]ethylamino}-4-(2-propynyl)amino-1,2,5-thiadiazole 1,1-dioxide,

n) 3-Amino-4-[3-(2-pyrrolidinomethylphenoxy)propylamino]-1,2,5-thiadiazole 1-oxide,

o) 3-{2-[(2-Guanidinothiazol-4-yl)methylthio]ethylamino-4-amino-1,2,5-thiadiazole 1,1-dioxide,

p) 3-{2-[(2-Dimethylaminomethyl-4-thiazolyl)methylthio]ethylamino}-4-methylamino-1,2,5-thiadiazole 1,1-dioxide,

q) 3-{2-[(5-Dimethylaminomethyl-2-thienyl)methylthio]ethylamino}-4-methylamino-1,2,5-thiadiazole 1,1-dioxide,

r) 3-{2-[(5-Dimethylaminomethyl-2-furyl)methylthio]ethylamino}-4-ethylamino-1,2,5-thiadiazole 1-oxide,

s) 3-Amino-4-{3-[3-(4-methylpiperidinomethyl)phenoxy]propylamino}-1,2,5-thiadiazole 1,1-dioxide,

t) 3-Amino-4-{2-[(2-guanidinothiazol-4-yl)methylthio]ethylamino}-1,2,5-thiadiazole 1-oxide,

u) 3-Benzylamino-4-{2-[(5-dimethylaminomethyl-2-furyl)methylthio]ethylamino}-1,2,5-thiadiazole 1,1-dioxide,

v) 3-{2-[(3-{Dimethylaminomethyl}phenyl)methylthio]ethylamino}-4-methylamino-1,2,5-thiadiazole 1,1-dioxide,

w) 3-Amino-4-{2-[(3-{dimethylaminomethyl}phenyl)methylthio]ethylamino}-1,2,5-thiadiazole 1-oxide,

x) 3-{2-[(5-Dimethylaminomethyl-2-thienyl)methylthio]ethylamino}-4-methylamino-1,2,5-thiadiazole 1-oxide,

y) 3-Amino-4-{4-(5-dimethylaminomethyl-2-furyl)butylamino}-1,2,5-thiadiazole 1,1-dioxide,

z) 3-Amino-4-{2-[(2-dimethylaminomethyl-4-thiazolyl)methylthio]ethylamino}-1,2,5-thiadiazole 1,1-dioxide,

aa) 3-Butylamino-4-{2-[(5-dimethylaminomethyl-2-furyl)methylthio]ethylamino}-1,2,5-thiadiazole 1,1-dioxide,

bb) 3-Cyclopropylmethylamino-4-{2-[(5-dimethylaminomethyl-2-furyl)methylthio]ethylamino}-1,2,5-thiadiazole 1,1-dioxide,

cc) 3-{2-[(5-Dimethylaminomethyl-2-furyl)methylthio]ethylamino}-4-[(2-pyridyl)methylamino]-1,2,5-thiadiazole 1,1-dioxide,

dd) 3-Amino-4-{3-[3-(4-methylpiperidinomethyl)phenoxy]propylamino}-1,2,5-thiadiazole 1-oxide,

ee) 4-{2-[(5-Dimethylaminomethyl-2-thienyl)methylthio]ethylamino}-3-(1-propylamino)-1,2,5-thiadiazole 1,1-dioxide,

ff) 3-{2-[(2-Guanidinothiazol-4-yl)methylthio]ethylamino}-4-methylamino-1,2,5-thiadiazole 1-oxide,

gg) 3-{3-[3-(hexamethyleneiminomethyl)phenoxy]propylamino}-4-methylamino-1,2,5-thiadiazole 1,1-dioxide,

hh) 3-[3-(3-dimethylaminomethylphenoxy)propylamino]-4-methylamino-1,2,5-thiadiazole 1,1-dioxide,

ii) 3-Amino-4-{3-[3-(hexamethyleneiminomethyl)phenoxy]propylamino}-1,2,5-thiadiazole 1-oxide,

jj) 4-{2-[(5-Dimethylaminomethyl-2-thienyl)methylthio]ethylamino}-3-(3-pyridyl)methylamino-1,2,5-thiadiazole 1,1-dioxide,

kk) 3-Amino-4-[3-(3-morpholinomethylphenoxy)propylamino]-1,2,5-thiadiazole 1,1-dioxide,

ll) 3-Methylamino-4-[3-(3-morpholinomethylphenoxy)propylamino]-1,2,5-thiadiazole 1,1-dioxide,

mm) 3-Amino-4-[3-(3-dimethylaminomethylphenoxy)propylamino]-1,2,5-thiadiazole 1-oxide,

nn) 3-Amino-4-{3-[3-(heptamethyleneiminomethyl)phenoxy]propylamino}-1,2,5-thiadiazole 1-oxide,

oo) 3-[(3-Pyridyl)methylamino]-4-[3-(3-piperidinomethylphenoxy)propylamino]-1,2,5-thiadiazole 1-oxide,

pp) 3-Amino-4-{2-[(2-{2-methylguanidino}thiazol-4-yl)methylthio]ethylamino}-1,2,5-thiadiazole 1-oxide,

qq) 3-Methylamino-4-[3-(3-piperidinomethylphenoxy)propylamino]-1,2,5-thiadiazole 1,1-dioxide,

rr) 3-Amino-4-[3-(3-piperidinomethylphenoxy)propylamino]-1,2,5-thiadiazole 1-oxide,

ss) 3-{2-[(5-Dimethylaminomethyl-2-thienyl)methylthio]ethylamino}-4-ethylamino-1,2,5-thiadiazole 1,1-dioxide,

tt) 3-Amino-4-[3-(3-piperidinomethylphenoxy)propylamino]-1,2,5-thiadiazole 1,1-dioxide,

uu) 3-Amino-4-[3-(3-guanidinophenoxy)propylamino]-1,2,5-thiadiazole 1-oxide.

Histamine $H_2$-receptor antagonists have been shown to be effective inhibitors of gastric secretion in animals, including man, Brimblecombe *et al., J. Int. Med. Res., 3,* 86 (1975). Clinical evaluation of the histamine $H_2$-receptor antagonist cimetidine has shown it to be an effective therapeutic agent in the treatment of peptic ulcer disease, Gray *et al., Lancet, 1,* 8001 (1977). Two of the standard animal models for determining gastric antisecretory activity of histamine $H_2$-antagonists are the Gastric Fistula Rat and the Heidenhain Pouch Dog. The $ED_{50}$'s for some of the compounds of Formula I in these two animal models are given in Tables 2 and 3, below.

Determination of Gastric Antisecretory Activity in the Gastric Fistula Rat

Male Long Evans rats weighing about 240—260 grams at the time of cannula implantation are used. The design and implantation of the stainless steel cannula into the anterior wall of the fore-stomach are carried out essentially as described by Pare *et al.* [Laboratory Animal Science, *27,* 244 (1977)]. The fistula components are designed and the operative procedure is carried out exactly as described in the above reference. Post operatively the animals are individually housed in solid bottom cages with sawdust and are allowed food and water *ad libitum* throughout the entire recovery period. Animals are not used for test purposes for at least 15 days after the operative procedure.

The animals are fasted but allowed water *ad libitum* for 20 hours before the testing procedure is to begin. Immediately prior to collection, the cannula is opened and the stomach gently with 30—40 mL of warm saline or distilled water to remove any residual contents. The catheter is then screwed into the cannula in place of the plugging screw and the rat is placed in a clear plastic rectangular cage measuring 40 cm long, 15 cm wide and 13 cm high. The bottom of the cage has a slit approximately 1.5 cm wide and 25 cm long running down the center to accommodate the catheter which hangs through it. In this way the rat is not restricted and can move freely about the cage during collection periods. The remainder of the assay is carried out as described by Ridley *et al.* [Research Comm. Chem. Path. Pharm., *17,* 365 (1977)].

Gastric secretions collected during the first hour after washing the stomach are discarded as they may

be contaminated. For oral evaluation, the catheter is then removed from the cannula and replaced with the plugging screw. Water (2 mL/kg) is administered orally via gastric intubation and the animal is returned to the cage for 45 minutes. After this time the plugging screw is removed and replaced with a catheter to which a small plastic vial has been attached to collect the gastric secretions. A two hour sample is collected (this represents the control secretion), the catheter removed and replaced with the plugging screw. The test drug is now administered orally in a volume of 2 mL/kg via gastric intubation. Forty-five minutes later the plugging screw is again removed, replaced with the catheter attached to a small plastic vial and another 2 hour sample is collected. The secretions in the second sample are compared to those of the control sample in order to determine the effects of the test drug.

When test compounds are to be evaluated parenterally, the animal is injected ip or sc with the test compound vehicle in a volume of 2 mL/kg immediately after discarding the initial 60 minute collection. A two hour sample is collected (control secretion) and the animals are injected either ip or sc with the test compound in a volume of 2 mL/kg. An additional two hour sample is collected and its secretions are compared to those of the control period to determine drug effects.

The samples are centrifuged in a graduated tube for volume determination. Titratable acidity is measured by titrating a one mL sample of pH 7.0 with 0.02N NaOH, using an Autoburet and an electrometric pH meter (Radiometer). Titratable acid output is calculated in microequivalents by multiplying the volume in milliliters by the acid concentration in milliequivalents per liter.

Results are expressed as percent inhibition relative to control readings. Dose response curves are constructed and $ED_{50}$ values are calculated by regression analyses. At least three rats are used at each dosage level and a minimum of three dosage levels are utilized for determination of a dose response curve.

Determination of Gastric Antisecretory Activity in the Heidenhain Pouch Dog

Prior to surgery, hematology and blood chemistry profiles are obtained and an assessment made as to the general health of selected female dogs. Dogs are vaccinated with Tissue Vax 5 (DHLP — Pitman-Moore) and housed in general animal quarters for four weeks' observation so incipient diseases may become apparent. Dogs are fasted with water *ad libitum* 24 hours prior to surgery.

Anesthesia is induced with Sodium Pentothal (Abbott) 25—30 mg/kg iv. Subsequent anesthesia is maintained with methoxyflurane (Pitman-Moore). A mid-line linea alba incision from xiphoid to umbilicus provides good exposure and ease of closure. The stomach is pulled up into the operative field, the greater curvature stretched out at multiple points and clamps placed along the selected line of incision. The pouch is made from the corpus of the stomach so that true parietal cell juice is obtained. About 30% of the corpus volume is resected. The cannula is made of light-weight, biologically-inert material such as nylon or Delrin with dimensions and attachments after DeVito and Harkins [J. Appl. Physiol., *14,* 138 (1959)]. Post operatively, dogs are medicated with antibiotics and an analgesic. They are allowed 2—3 months for recovery. Experiments are carried out in the following way: Dogs are fasted overnight (~18 hours) with water *ad libitum* prior to each experiment. The dogs are placed in a sling and a saphenous vein cannulated for drug administration. Histamine as the base (100 µg/kg/hr) and chlorpheniramine maleate (0.25 mg/kg/hr) are infused continuously (in a volume of 6 mL/hr) with a Harvard infusion pump.

Ninety minutes' infusion are allowed for the dogs to reach a steady state of acid output. At this time the drug or normal saline (control) is administered concomitantly with the secretagogue in a volume of 0.5 mL/kg over a 30 second period. When oral studies are to be carried out, the drug is administered via gastric gavage in a volume of 5 mL/kg. Infusion of the secretagogue is continued and 15 minute samples of the gastric juice are taken for 4.5 hours. Each sample is measured to the nearest 0.5 mL and titratable acidity is determined by titrating a 1 mL sample to pH 7.0 with 0.2N NaOH, using an Autoburet and an electrometric pH meter (Radiometer). Titratable acid output is calculated in microequivalents by multiplying the volume in milliliters by the acid concentration in milliequivalents per liter.

Results are expressed as percent inhibition relative to control readings. Dose response curves are constructed and $ED_{50}$ values are calculated by regression analyses. From 3 to 5 dogs are used at each dose level and a minimum of three dosage levels are utilized for determination of a dose response curve.

Table 2

Effect of Compounds of Formula I on Gastric Acid Output in the Two-Hour Pylorus Ligated Rat

| Compound of Example No. | Route of Administration | $ED_{50}$ * µmoles/kg |
|---|---|---|
| 1 | i.p. | 12.5 (4.90-33.0) |
| 2 | s.c. | ∿100 |
| 3 | i.p. | 0.46 (0.26-0.74) |
| 7 | i.p. | 31.1 (11.1-82.8) |
| 11 B | i.p. | 0.69 (0.31-1.33) |
| 11 C | s.c. | 0.20 (0.03-2.9) |
| 12 | i.p. | 0.28 (0.11-0.69) |
| 13 | s.c. | 0.46 (0.02-3.1) |
| 14 | s.c. | ∿25 |
| 17 | s.c. | 33 (8.7-141) |
| 18 | s.c. | 0.38 (0.02-5.33) |
| 19 | s.c. | 0.34 (0.15-0.81) |
| 20 A | s.c. | 1.15 (0.32-3.7) |
| 21 | s.c. | 0.30 (0.09-1.0) |
| 28 | s.c. | 1.39 (0.39-4.91) |
| 31 | i.p. | 0.41 (0.19-0.81) |
| 32 | i.p. | 0.08 (0.03-0.15) |
| 33 | s.c. | 0.57 (0.16-1.84) |
| 35 | s.c. | 0.08 (0.02-0.22) |
| 36 | s.c. | 1.59 (0.48-6.46) |
| 51 | s.c. | 55 (8.8-930) |
| 52 | s.c. | ∿350 |
| 65 | s.c. | 0.07 (0.02-0.32) |
| 84 | s.c. | 0.15 (0.02-0.53) |
| 85 | s.c. | 0.14 (0.05-0.41) |
| 86 | s.c. | 0.04 (0.015-0.12) |
| 87 | s.c. | 0.02 (0.006-0.04) |
| 88 | s.c. | 0.08 (0.04-0.22) |
| 89 | s.c. | 0.25 (0.07-0.84) |

Table 2 (cont.)

| | | |
|---|---|---|
| 90 | s.c. | 0.86 (0.24-2.69) |
| 91 | s.c. | 1.3 (0.36-3.9) |
| 92 | s.c. | 0.24 (0.09-0.71) |
| 93 | s.c. | 0.14 (0.07-0.32) |
| 94 | s.c. | 0.44 (0.08-1.9) |
| 95 | s.c. | ∿15 |
| 96 | s.c. | ∿15 |
| 97 | s.c. | ∿3 |
| 98 | s.c. | 0.52 (0.08-2.33) |
| 99 | s.c. | 32 (5.7-200) |
| 100 | s.c. | 1.6 (0.38-5.5) |
| 101 | s.c. | 68 (10-750) |
| 102 | s.c. | ∿15 |
| 103 | s.c. | 0.54 (0.21-1.4) |
| 104 | s.c. | 0.61 (0.15-1.88) |
| 105 | s.c. | 1.65 (0.45-4.45) |
| 106 | s.c. | ∿80 |
| 107 | s.c. | 23 (5.1-110) |
| 108 | s.c. | 2.2 (0.54-8.9) |
| 109 | s.c. | 1.4 (0.51-3.9) |
| 110 | s.c. | 0.05 (0.03-0.14) |
| 111 | s.c. | 0.64 (0.17-2.5) |
| 112 | s.c. | 1.2 (0.47-2.9) |
| 113 | s.c. | 0.07 (0.03-0.14) |
| 114 | s.c. | ∿15 |
| 115 | s.c. | 0.57 (0.20-1.6) |
| 116 | s.c. | >10 |
| 117 | s.c. | ∿0.5 |
| 118 | s.c. | 0.066 (0.018-0.19) |
| 119 | s.c. | >10 |
| 120 | s.c. | ∿5 |
| 121 | s.c. | 0.19 (0.055-0.56) |
| 122 | s.c. | ∿10.0 |
| 123 | s.c. | >10 |
| 124 | s.c. | >10 |

Table 2 (cont.)

| | | |
|---|---|---|
| 125 | s.c. | ~10 |
| 127 | s.c. | >10 |
| 128 | s.c. | ~10 |
| 129 | s.c. | ~1 |
| 130 | s.c. | 2.3 (0.79-14) |
| 131 | s.c. | ~0.5 |
| 132 | s.c. | 0.025 (0.007-0.069) |
| 133 | s.c. | 0.061 (0.019-0.24) |
| 134 | s.c. | 0.024 (0.011-0.050) |
| 135 | s.c. | 0.57 (0.29-1.12) |
| 144 a | s.c. | 0.095 (0.033-0.30) |
| 144 b | s.c. | 0.025 (0.0087-0.065) |
| 144 c | s.c. | 0.14 (0.034-0.44) |
| 144 d | s.c. | 0.91 (0.36-3.2) |
| 145 a | s.c. | 0.056 (0.021-0.18) |
| 145 b | s.c. | ~0.06 |
| 145 c | s.c. | 0.025 (0.0098-0.057) |
| 145 d | s.c. | 0.05 (0.005-0.2) |
| 146 a | s.c. | 0.023 (0.0091-0.046) |
| 146 d | s.c. | 0.28 (0.066-1.21) |
| 149 | s.c. | ~0.08 |
| 151 | s.c. | 0.9 (0.15-3.5) |

*Numbers in parentheses are the 95% confidence limits.

Table 3

Oral Gastric Antisecretory Activity of Compounds of Formula I in the Heidenhain Pouch Dog

| Compound of Example | Potency Ratio (cimetidine = 1.0) |
|---|---|
| 11 B | 11 |
| 12 | 10 |
| 18 | 18 |
| 31 | 13 |
| 35 | 20 |
| 65 | 10 |
| 86 | 33 |
| 87 | ∿30 |
| 88 | 13 |
| 118 | 10 |
| 133 | ∿10 |
| 145 c | 8 |
| 146 a | ∿20 |
| 149 | 10 |

The thiadiazole compounds of the present invention may be produced according to a process described in GB—A—2 067 987.

The following are representative compounds of formula I.

1) 3-{2-[(5-Methyl-1H-imidazol-4-yl)methylthio]ethylamino}-4-(2-propynyl)amino-1,2,5-thiadiazole 1,1-dioxide

mp 82—103°;

*Anal.* Calcd for $C_{12}H_{16}N_6O_2S_2\cdot\frac{1}{3}CH_3OH$: C, 42.19; H, 4.97; N, 23.95; S, 18.27.
Found: C, 42.05; H, 5.05; N, 24.01; S, 18.45.

2) 3-{2-[(5-Methyl-1H-imidazol-4-yl)methylthio]ethylamino}-4-methylamino-1,2,5-thiadiazole 1,1-dioxide

mp 98—110°. The NMR spectrum (100 MHz) in $d_6$ dimethyl sulfoxide gave the following resonances δ: 7.46 (s, 1H,); 3.70 (s, 2H); 2.53 (t, 2H); 2.86 (s, 3H); 2.72 (t, 2H); 2.15 (s, 3H).

*Anal.* Calcd for $C_{10}H_{16}N_6O_2S_2$: C, 37.96; H, 5.09; N, 26.56; S, 20.27.
Found (corr. for 1.60% $H_2O$): C, 37.79; H, 5.16; N, 26.52; S, 20.24.

3) 3-{2-[(2-Guanidinothiazol-4-yl)methylthio]ethylamino}-4-{2-[(5-methyl-1H-imidazol-4-yl)methylthio]ethylamino}-1,2,5-thiadiazole 1,1-dioxide

*Anal.* Calcd for $C_{16}H_{24}N_{10}O_2S_4\cdot\frac{2}{3}C_4H_8O_2$: C, 38.96; H, 5.14; N, 24.34; S, 22.29.
Found: C, 39.08; H, 4.96; N, 24.48; S, 22.26.

4) 3-{2-[(5-Methyl-1H-imidazol-4-yl)methylthio]ethylamino}-4-methylamino-1,2,5-thiadiazole 1-oxide

5) 3-{2-[(5-Hydroxymethyl-1H-imidazol-4-yl)methylthio]ethylamino}-4-methylamino-1,2,5-thiadiazole 1,1-dioxide

6a) 3-{2-[(5-bromo-1H-imidazol-4-yl)methylthio]ethylamino}-4-methylamino-1,2,5-thiadiazole 1,1-dioxide,

6b) 3-{2-[imidazol-4-ylmethylthio]ethylamino}-4-methylamino-1,2,5-thiadiazole 1,1-dioxide,

6c) 3-{2-[imidazol-2-ylmethylthio]ethylamino}-4-methylamino-1,2,5-thiadiazole 1,1-dioxide,

6d) 3-{2-[(1-methyl-imidazol-2-yl)methylthio]ethylamino}-4-methylamino-1,2,5-thiadiazole 1,1-dioxide,

6e) 3-{2-[(2-methyl-1H-imidazol-4-yl)methylthio]ethylamino}-4-methylamino-1,2,5-thiadiazole 1,1-dioxide,

6f) 3-{2-[(1-methyl-imidazol-4-yl)methylthio]ethylamino}-4-methylamino-1,2,5-thiadiazole 1,1-dioxide,

6g) 3-{2-[(1,5-dimethyl-imidazol-4-yl)methylthio]ethylamino}-4-methylamino-1,2,5-thiadiazole 1,1-dioxide,

6h) 3-{2-[(5-chloro-1-methyl-imidazol-4-yl)methylthio]ethylamino}-4-methylamino-1,2,5-thiadiazole 1,1-dioxide,

6i) 3-{2-[(5-trifluoromethyl-1H-imidazol-4-yl)methylthio]ethylamino}-4-methylamino-1,2,5-thiadiazole 1,1-dioxide,

6k) 3-{2-[(5-ethyl-1H-imidazol-4-yl)methylthio]ethylamino}-4-methylamino-1,2,5-thiadiazole 1,1-dioxide and

6l) 3-{2-[(2-amino-1H-imidazol-4-yl)methylthio]ethylamino}-4-methylamino-1,2,5-thiadiazole 1,1-dioxide,

7) 3-Hydroxy-4-{2-[(5-methyl-1H-imidazol-4-yl)methylthio]ethylamino}-1,2,5-thiadiazole 1,1-dioxide
mp 263—265° (dec).

| | | | | |
|---|---|---|---|---|
| *Anal.* Calcd for $C_9H_{13}N_5S_2O_3$: | C, 35.64; | H, 4.32; | N, 23.09; | S, 21.13. |
| Found: | C, 35.56; | H, 4.48; | N, 23.01; | S, 21.13. |

8) 3-{4-[(2-Guanidino-1H-imidazol-4-yl]butylamino}-4-methylamino-1,2,5-thiadiazole 1,1-dioxide,

9) 3-{2-[(5-Methyl-1H-imidazol-4-yl)methylthio]ethylamino}-4-(2-propylyl)amino-1,2,5-thiadiazole 1,1-dioxide,

10) 3-{2-[(5-Methyl-1H-imidazol-4-yl)methylthio]ethylamino}-4-methylamino-1,2,5-thiadiazole 1,1-dioxide,

11a) 3-{2-[(5-Dimethylaminomethyl-2-furyl)methylthio]ethylamino}-4-methylamino-1,2,5-thiadiazole 1,1-dioxide,
mp 82—90°;

| | | | | |
|---|---|---|---|---|
| *Anal.* Calcd for $C_{13}H_{21}N_5O_3S_2\cdot\frac{2}{3}CH_3OH$: | C, 43.10; | H, 6.26; | N, 18.38; | S, 16.83. |
| Found (corr. for 1.72% $H_2O$): | C, 43.30; | H, 6.12; | N, 18.57; | S, 16.96. |

11b) 3,4-Bis-{2-[(5-dimethylaminomethyl-2-furyl)methylthio]ethylamino}-1,2,5-thiadiazole 1,1-dioxide,
mp 92.5—96°.

| | | | | |
|---|---|---|---|---|
| *Anal.* Calcd for $C_{22}H_{34}N_6S_3O_4\cdot H_2O$: | C, 47.12; | H, 6.47; | N, 14.99; | S, 17.15. |
| Found: | C, 47.28; | H, 6.48; | N, 15.09; | S, 17.39. |

Calcd for $H_2O$ = 3.21%;
Found $H_2O$ = 3.32%

12) 3-{2-[(5-Dimethylaminomethyl-2-furyl)methylthio]ethylamino}-4-ethylamino-1,2,5-thiadiazole 1,1-dioxide,
mp 155—160° with variable sintering at 94—96°

| | | | | |
|---|---|---|---|---|
| *Anal.* Calcd for $C_{14}H_{23}N_5O_3S_2\cdot 0.8CH_3OH$: | C, 44.54; | H, 6.62; | N, 17.55; | S, 16.07. |
| Found: | C, 44.35; | H, 6.58; | N, 17.44; | S, 16.18. |

13) 3-{2-[(5-Dimethylaminomethyl-2-furyl)methylthio]ethylamino}-4-(2-propynyl)amino-1,2,5-thiadiazole 1,1-dioxide,
mp 92—100°;

| | | | | |
|---|---|---|---|---|
| *Anal.* Calcd for $C_{15}H_{21}N_5O_3S_2\cdot CH_3OH$: | C, 46.25; | H, 6.06; | N, 16.85; | S, 15.43. |
| Found: | C, 46.36; | H, 6.22; | N, 16.95; | S, 15.73. |

14) 3-Methylamino-4-{2-[(5-{[N-methyl-N-(2-propynyl)amino]methyl-2-furyl)methylthio]ethylamino}1,2,5-thiadiazole 1,1-dioxide,
mp 50—51°, clear melt 54—56°

| | | | | |
|---|---|---|---|---|
| *Anal.* Calcd for $C_{15}H_{21}N_5O_3S_2\cdot\frac{1}{4}C_3H_8O$: | C, 47.47; | H, 5.82; | N, 17.57; | S, 16.09. |
| Found: | C, 47.51; | H, 6.21; | N, 16.40; | S, 15.97. |

15) 3-{2-[(5-Dimethylaminomethyl-3-methyl-2-furyl)methylthio]ethylamino}-4-methylamino-1,2,5-thiadiazole 1,1-dioxide,

16) 3-{2-[(5-Dimethylaminomethyl-4-methyl-2-furyl)methylthio]ethylamino}-4-methylamino-1,2,5-thiadiazole 1,1-dioxide,

17) 3-{2-[(5-Dimethylaminomethyl-2-furyl)methylthio]ethylamino}-4-hydroxy-1,2,5-thiadiazole 1,1-dioxide, mp 109—122°;

| | | | | |
|---|---|---|---|---|
| *Anal.* Calcd for $C_{12}H_{18}N_4O_4S_2$: | C, 41.61; | H, 5.24; | N, 16.17; | S, 18.51. |
| Found (corr. for 1.15% $H_2O$): | C, 41.59; | H, 5.32; | N, 16.33; | S, 18.81. |

18) 3-{2-[(5-Dimethylaminomethyl-2-furyl)methylthio]ethylamino}-4-methylamino-1,2,5-thiadiazole 1-oxide,
mp 86—92°;

| | | | | |
|---|---|---|---|---|
| *Anal.* Calcd for $C_{13}H_{21}N_5O_2S_2$: | C, 45.46; | H, 6.16; | N, 20.39; | S, 18.67. |
| Found: | C, 45.24; | H, 6.24; | N, 20.41; | S, 18.90. |

19) 3-Allylamino-4-{2-[(5-dimethylaminomethyl-2-furyl)methylthio]ethylamino}-1,2,5-thiadiazole 1,1-dioxide,
mp 83—86°;

| | | | | |
|---|---|---|---|---|
| *Anal.* Calcd for $C_{15}H_{23}N_5O_3S_2 \cdot 0.9C_3H_8O$: | C, 48.36; | H, 6.92; | N, 15.93; | S, 14.59. |
| Found: | C, 48.46; | H, 6.96; | N, 16.13; | S, 14.58. |

20a) 3-Methylamino-4-{2-[(5-methylaminomethyl-2-furyl)methylthio]ethylamino}-1,2,5-thiadiazole 1,1-dioxide,
Rf = 0.50 (TLC-silica/$CH_3CN$:$CH_3OH$:$CH_3COOH$ (50:50:1)]
mp 50—56°.
The NMR spectrum (100 MHz) in $d_6$ dimethyl sulfoxide gave the following resonances δ 6.20 (m, 2H); 3.80 (s, 2H); 3.62 (s, 2H); 3.50 (t, 2H); 2.90 (s, 3H); 2.70 (t, 2H); 2.28 (s, 3H); it also showed the presence of approximately 0.2 mole of methanol.

| | | | | |
|---|---|---|---|---|
| *Anal.* Calcd for $C_{12}H_{19}N_5O_3S_2 \cdot 0.2\ CH_3OH$: | C, 41.65; | H, 5.65; | N, 19.96; | S, 18.28. |
| Found (corr. for 1.42% $H_2O$): | C, 41.98; | H, 5.69; | N, 19.54; | S, 18.54. |

20b) 3,4-Bis--{2-[(5-methylaminomethyl-2-furyl)methylthio]ethylamino}-1,2,5-thiadiazole 1,1-dioxide,
Rf = 0.07 [TLC-silica/$CH_3CN$:$CH_3OH$:$CH_3COOH$ (50:50:1)]
The NMR spectrum (100 MHz) in $d_6$ dimethyl sulfoxide gave the following resonances δ: 6.22 (m, 4H); 3.82 (s, 4H); 3.65 (s, 4H); 3.50 (t, 4H); 2.72 (t, 4H); 2.30 (s, 6H).

21) 3-{4-(5-Dimethylaminomethyl-2-furyl)butylamino}-4-methylamino-1,2,5-thiadiazole 1,1-dioxide,
mp 152—153°;

| | | | | |
|---|---|---|---|---|
| *Anal.* Calcd for $C_{14}H_{23}H_5O_3S$: | C, 49.25; | H, 6.79; | N, 20.51; | S, 9.39. |
| Found: | C, 49.41; | H, 6.87; | N, 20.61; | S, 9.28. |

22) 3-{2-[(5-Dimethylaminomethyl-2-furyl)methoxy]ethylamino}-4-methylamino-1,2,5-thiadiazole 1,1-dioxide,

23) 3-{2-[(5-Dimethylaminomethyl-2-furyl)methylthio]ethylamino}-4-ethylamino-1,2,5-thiadiazole 1,1-dioxide,

24) 3-{2-[(5-Dimethylaminomethyl-2-furyl)methylthio]ethylamino}-4-methylamino-1,2,5-thiadiazole 1-oxide,

25) 3-{2-[(5-Dimethylaminomethyl-2-furyl)methylthio]]ethylamino}-4-methylamino-1,2,5-thiadiazole 1,1-dioxide,
mp 92—96°;

26) 3-{2-[(5-Dimethylaminomethyl-2-furyl)methylthio]ethylamino}-4-methylamino-1,2,5-thiadiazole 1-oxide,

27) 3-{3-[(5-Dimethylaminomethyl-2-furyl)methylthio]propylamino}-4-ethylamino-1,2,5-thiadiazole 1,1-dioxide,

28) 3-{2-[(5-Dimethylaminomethyl-2-furyl)methylthio]ethylamino}-4-dimethylamino-1,2,5-thiadiazole 1,1-dioxide,
mp 139—142°;

*Anal.* Calcd for $C_{19}H_{24}N_5O_3S_2$: C, 44.90; H, 6.46; N, 18.70; S, 17.12.
Found (corr. for 0.51% $H_2O$): C, 44.77; H, 6.25; N, 18.89; S, 17.42.

29a) 3-{2-[(5-dimethylaminomethyl-2-furyl)methylthio]ethylamino}-4-(4-thiomorpholinyl)-1,2,5-thiadiazole 1,1-dioxide,

29b) 3-{2-[(5-dimethylaminomethyl-2-furyl)methylthio]ethylamino}-4-(1-piperazinyl)-1,2,5-thiadiazole 1,1-dioxide,

29c) 3-{2-[(5-dimethylaminomethyl-2-furyl)methylthio]ethylamino}-4-(4-acetyl-1-piperazinyl)-1,2,5-thiadiazole 1,1-dioxide,

29d) 3-{2-[(5-dimethylaminomethyl-2-furyl)methylthio]ethylamino}-4-(4-methyl-1-piperazinyl)-1,2,5-thiadiazole 1,1-dioxide,

29e) 3-{2-[(5-dimethylaminomethyl-2-furyl)methylthio]ethylamino}-4-(1-hexamethyleneimino)-1,2,5-thiadiazole 1,1-dioxide and

29f) 3-{2-[(5-dimethylaminomethyl-2-furyl)methylthio]ethylamino}-4-(1-homopiperazinyl)-1,2,5-thiadiazole 1,1-dioxide,

30a) 3-(cyclobutylamino)-4-{2-[(5-dimethylaminomethyl-2-furyl)methylthio]ethylamino}-1,2,5-thiadiazole 1,1-dioxide,

30b) 3-[(cyclobutyl)methylamino]-4-{2-[(5-dimethylaminomethyl-2-furyl)methylthio]ethylamino}-1,2,5-thiadiazole 1,1-dioxide,

30c) 3-{2-[(5-dimethylaminomethyl-2-furyl)methylthio]ethylamino}-4-(2-hydroxyethylamino)-1,2,5-thiadiazole 1,1-dioxide,

30d) 3-{2-[(5-dimethylaminomethyl-2-furyl)methylthio]ethylamino}-4-(2-methylthioethylamino)-1,2,5-thiadiazole 1,1-dioxide,

30e) 3-{2-[(5-dimethylaminomethyl-2-furyl)methylthio]ethylamino}-4-(2,2,2-trifluoroethylamino)-1,2,5-thiadiazole 1,1-dioxide,

30f) 3-{2-[(5-dimethylaminomethyl-2-furyl)methylthio]ethylamino}-4-(2-fluoroethylamino)-1,2,5-thiadiazole 1,1-dioxide,

30g) 3-(2-aminoethylamino)-4-{2-[(5-dimethylaminomethyl-2-furyl)methylthio]ethylamino}-1,2,5-thiadiazole 1,1-dioxide,

30h) 3-{2-[(5-dimethylaminomethyl-2-furyl)methylthio]ethylamino}-4-(2-methylaminoethylamino)-1,2,5-thiadiazole 1,1-dioxide,

30i) 3-{2-[(5-dimethylaminomethyl-2-furyl)methylthio]ethylamino}-4-(2-dimethylaminoeethylamino)-1,2,5-thiadiazole 1,1-dioxide,

30k) 3-{2-[(5-dimethylaminomethyl-2-furyl)methylthio]ethylamino}-4-(2,2-dimethylhydrazino)-1,2,5-thiadiazole 1,1-dioxide,

30l) 3-cyanoamino-4-{2-[(5-dimethylaminomethyl-2-furyl)methylthio]ethylamino}-1,2,5-thiadiazole 1,1-dioxide,

30m) 3-(3-cyanopropylamino)-4-{2-[(5-dimethylaminomethyl-2-furyl)methylthio]ethylamino}-1,2,5-thiadiazole 1,1-dioxide,

30n) 3-{2-[(5-dimethylaminomethyl-2-furyl)methylthio]ethylamino}-4-guanidino-1,2,5-thiadiazole 1,1-dioxide,

30o) 3-{2-[(5-dimethylaminomethyl-2-furyl)methylthio]ethylamino}-4-(N'-methyl)guanidino-1,2,5-thiadiazole 1,1-dioxide.

31) 3-{2-[(2-Guanidinothiazol-4-yl)methylthio]ethylamino}-4-methylamino-1,2,5-thiadiazole 1,1-dioxide, mp 196—198° (dec);

*Anal.* Calcd for $C_{10}H_{16}N_8O_2S_3$: C, 31.90; H, 4.28; N, 29.77; S, 25.55.
Found: C, 31.85; H, 4.24; N, 29.79; S, 25.45.

32) 3-{2-[(2-Guanidinothiazol-4-yl)methylthio]ethylamino}-4-(2-propynyl)amino-1,2,5-thiadiazole 1,1-dioxide,
mp 176—178°;
*Anal.* Calcd for $C_{12}H_{16}N_8O_2S_3$: C, 35.99; H, 4.03; N, 27.98; S, 24.02.
Found: C, 35.82; H, 4.12; N, 28.41; S, 24.28.

33) 3-{2-[(2-Dimethylaminomethyl-4-thiazolyl)methythio]ethylamino}-4-methylamino-1,2,5-thiadiazole 1,1-dioxide,
mp 144—148°
*Anal.* Calcd for $C_{12}H_{20}N_6O_2S_3$: C, 38.28; H, 5.35; N, 22.32; S, 25.55.
Found: C, 37.89; H, 5.43; N, 22.19; S, 25.40.

34) 3-{2-[(2-Dimethylaminomethyl-4-thiazolyl)methythio]ethylamino}-4-methylamino-1,2,5-thiadiazole 1-oxide,

35) 3-Amino-4-{2-[(2-guanidinothiazol-4-yl)methythio]ethylamino}-1,2,5-thiadiazole 1,1-dioxide,
mp 134—149° (foaming); the NMR spectrum (100 MHz) in $d_6$ dimethyl sulfoxide/$D_2O$/DCl gave the following resonances δ: 7.16 (s, 1H); 3.84 (s, 2H); 3.52 (t, 2H); 2.75 (t, 2H); and showed the presence of approximately 1.2 moles of methanol.
*Anal.* Calcd for $C_9H_{14}N_8O_2S_3 \cdot 1.2CH_3OH$: C, 30.56; H, 4.72; N, 27.95; S, 23.99.
Found (corr. for 1.31% $H_2O$): C, 30.19; H, 4.32; N, 27.91; S, 24.71.

36) 3-{2-[(2-Guanidinothiazol-4-yl)methylthio]ethylamino}-4-(2-hydroxyethylamino)-1,2,5-thiadiazole 1,1-dioxide,
mp = slowly resinified starting at 115°, decomposed starting at 175°.
*Anal.* Calcd for $C_{11}H_{18}N_8O_3S_3$: C, 32.50; H, 4.46; N, 27.57; S, 23.66.
Found (corr for 3.85% $H_2O$): C, 32.77; H, 4.21; N, 27.90; S, 24.39.

37) 3-(2,3-Dihydroxypropylamino)-4-{2-[(2-guanidinothiazol-4-yl)methylthio]ethylamino}-1,2,5-thiadiazole 1,1-dioxide,

38) 3-Methylamino-4-{2-[(thiazol-2-yl)methylthio]ethylamino}-1,2,5-thiadiazole 1,1-dioxide,

39a) 3-{2-[(2-aminothiazol-4-yl)methylthio]ethylamino}-4-methylamino-1,2,5-thiadiazole 1,1-dioxide,

39b) 3-{2-[(4,5-dimethylthiazol-2-yl)methylthio]ethylamino}-4-methylamino-1,2,5-thiadiazole 1,1-dioxide,

39c) 3-{2-[(2-methylthiazol-4-yl)methylthio]ethylamino}-4-methylamino-1,2,5-thiadiazole 1,1-dioxide,

39d 3-{2-[(2-chlorothiazol-4-yl)methylthio]ethylamino}-4-methylamino-1,2,5-thiadiazole 1,1-dioxide,

39e) 3-{2-[(2-methylthiazol-5-yl)methylthio]ethylamino}-4-methylamino-1,2,5-thiadiazole 1,1-dioxide,

39f) 3-{2-[(4-methylthiazol-5-yl)methylthio]ethylamino}-4-methylamino-1,2,5-thiadiazole 1,1-dioxide,

39g) 3-{2-[(thiazol-4-yl)methylthio]ethylamino}-4-methylamino-1,2,5-thiadiazole 1,1-dioxide and

39h) 3-{2-[(4-dimethylaminomethylthiazol-2-yl)methylthio]ethylamino}-4-methylamino-1,2,5-thiadiazole 1,1-dioxide,

40) 3-{3-[(2-Dimethylaminomethyl-4-thiazolyl)methylthio]propylamino}-4-methylamino-1,2,5-thiadiazole 1,1-dioxide,

41) 3-{2-[(2-Guanidinothiazol-4-yl)methylthio]propylamino}-4-amino-1,2,5-thiadiazole 1,1-oxide,

42) 3-{2-[(2-Guanidinothiazol-4-yl)methylthio]ethylamino}-4-methylamino-1,2,5-thiadiazole 1,1-dioxide,

43) 3-{2-[(2-Guanidinothiazol-4-yl)methylthio]ethylamino}-4-methylamino-1,2,5-thiadiazole 1,1-dioxide,

44) 3-{2-[(2-Guanidinothiazol-4-yl)methylthio]ethylamino}-4-hydroxy-1,2,5-thiadiazole 1,1-dioxide,

45) 3-{2-[(2-Dimethylaminomethyl-4-thiazol)methylthio]ethylamino}-4-methylamino-1,2,5-thiadiazole 1,1-dioxide,

46) 3-{2-[(2-Dimethylaminomethyl-4-thiazolyl)methylthio]ethylamino}-4-methylamino-1,2,5-thiadiazole 1,1-dioxide,

47) 3-{2-[(2-Dimethylaminomethyl-4-thiazolyl)methylthio]ethylamino}-4-hydroxy-1,2,5-thiadiazole 1,1-dioxide,

48) 3-Amino-4-{2-[(2-dimethylaminomethyl-4-thiazolyl)methylthio]ethylamino}-1,2,5-thiadiazole 1-oxide,

49) 3-{2-[(2-Dimethylaminomethyl-4-thiazolyl)methylthio]ethylamino}-4-methylamino-1,2,5-thiadiazole 1-oxide,

50) 3-Amino-4-[4-(2-guanidinothiazol-4-yl)butylamino]-1,2,5-thiadiazole 1-oxide,

51) 3-{2-[(5-Dimethylaminomethyl-2-furyl)methylthio]ethylamino}-4-hydrazino-1,2,5-thiadiazole 1,1-dioxide,
mp 170° (dec.)

52) 3-Methylamino-4-{2-[(2-pyridyl)methylthio]ethylamino}-1,2,5-thiadiazole 1,1-dioxide,
mp 168—171°;

| | | | | |
|---|---|---|---|---|
| *Anal.* Calcd for $C_{11}H_{15}N_5O_2S_2$: | C, 42.15; | H, 4.82; | N, 22.35; | S, 20.46. |
| Found: | C, 42.07; | H, 4.75; | N, 22.28; | S, 20.73. |

53) 3-{2-[(3-Chloro-2-pyridyl)methylthio]ethylamino}-4-methylamino-1,2,5-thiadiazole 1,1-dioxide,

54) 3-{2-[(6-Dimethylaminomethyl-2-pyridyl)methylthio]ethylamino}-4-methylamino-1,2,5-thiadiazole 1,1-dioxide,

55a) 3-{2-[(3-bromo-2-pyridyl)methylthio]ethylamino}-4-methylamino-1,2,5-thiadiazole 1,1-dioxide,

55b) 3-{2-[(3-cyano-2-pyridyl)methylthio]ethylamino}-4-methylamino-1,2,5-thiadiazole 1,1-dioxide,

55c) 3-{2-[(3-hydroxy-2-pyridyl)methylthio]ethylamino}-4-methylamino-1,2,5-thiadiazole 1,1-dioxide,

55d) 3-{2-[(3-methoxy-2-pyridyl)methylthio]ethylamino}-4-methylamino-1,2,5-thiadiazole 1,1-dioxide,

55e) 3-{2-[(3-ethoxy-2-pyridyl)methylthio]ethylamino}-4-methylamino-1,2,5-thiadiazole 1,1-dioxide,

55f) 3-{2-[(3-methyl-2-pyridyl)methylthio]ethylamino}-4-methylamino-1,2,5-thiadiazole 1,1-dioxide and

55g) 3-{2-[(3-amino-2-pyridyl)methylthio]ethylamino}-4-methylamino-1,2,5-thiadiazole 1,1-dioxide,

56) 3-{2-[(3-Chloro-2-pyridyl)methylthio]ethylamino}-4-{2-[(5-dimethylaminomethyl-2-furyl)methylthio]ethylamino}-1,2,5-thiadiazole 1,1-dioxide,

57) 3-{2-[(6-Dimethylaminomethyl-2-pyridyl)methylthio]ethylamino}-4-methylamino-1,2,5-thiadiazole 1-oxide,

58) 3-{2-[(4-Methyl-1,2,5-oxadiazol-3-yl)methylthio]ethylamino}-4-methylamino-1,2,5-thiadiazole 1,1-dioxide,

59) 3-{2-[(5-Dimethylaminomethyl-2-furyl)methylthio]ethylamino}-4-{2-[(4-methyl-1,2,5-oxadiazol-3-yl)methylthio]ethylamino}-1,2,5-thiadiazole 1,1-dioxide,

60) 3-{2-[(5-Methyl-1,2,4-oxadiazol-3-yl)methylthio]ethylamino}-4-methylamino-1,2,5-thiadiazole 1,1-dioxide,

61) 3-{2-[(2-Methyl-1,3,4-oxadiazol-5-yl)methylthio]ethylamino}-4-methylamino-1,2,5-thiadiazole 1,1-dioxide,

62) 3-{2-[(2-Dimethylamino-1,3,4-oxadiazol-5-yl)methylthio]ethylamino}-4-methylamino-1,2,5-thiadiazole 1,1-dioxide,

63) 3-{2-[(3-{Dimethylaminomethyl}phenyl)methylthio]ethylamino}-4-amino-1,2,5-thiadiazole 1,1-dioxide,

64) 3-{3-[3-(Dimethylaminomethyl)phenoxy]propylamino}-4-amino-1,2,5-thiadiazole 1,1-dioxide,

65) 3-{2-[(5-Dimethylaminomethyl-2-thienyl)methylthio]ethylamino}-4-methylamino-1,2,5-thiadiazole 1,1-dioxide,
mp 60.5—66°;

66) 3-{2-[(5-Dimethylaminomethyl-2-thienyl)methylthio]ethylamino}-4-ethylamino-1,2,5-thiadiazole 1-oxide,

67) 3-{2-[(5-Dimethylaminomethyl-2-thienyl)methylthio]ethylamino}-4-{2-[(4-methyl-1,2,5-oxadiazol-3-yl)methylthio]-ethylamino}-1,2,5-thiadiazole 1,1-dioxide,

68) 3-{4-[(2-Guanidino-4-oxazolyl]butylamino}-4-methylamino-1,2,5-thiadiazole 1,1-dioxide,

69) 3-{2-[(2-(2-Amino-5-oxazolyl)ethylthio]ethylamino}-4-methylamino-1,2,5-thiadiazole 1,1-dioxide,

70) 3-{2-[3-Isoxazolylmethylthio]ethylamino}-4-methylamino-1,2,5-thiadiazole 1,1-dioxide,

71a) 3-{2-[(5-methyl-3-isoxazolyl)methylthio]ethylamino}-4-methylamino-1,2,5-thiadiazole 1,1-dioxide,

71b) 3-{2-[(3,5-dimethyl-4-isoxazolyl)methylthio}-4-methylamino-1,2,5-thiadiazole 1,1-dioxide and

71c) 3-{2-[2-(5-methyl-4-isoxazolyl)ethylthio]ethylamino}-4-methylamino-1,2,5-thiadiazole 1,1-dioxide

72) 3-{2-[(5-Dimethylaminomethyl-2-furyl)methylthio]ethylamino}-4-{2-[3-isoxazolylmethylthio]ethylamino}-1,2,5-thiadiazole 1,1-dioxide,

73) 3-{2-[3-Isothiazolylmethylthio]ethylamino}-4-methylamino-1,2,5-thiadiazole 1,1-dioxide,

74a) 3-{2-[(3-methyl-4-isothiazolyl)methylthio]ethylamino}-4-methylamino-1,2,5-thiadiazole 1,1-dioxide,

74b) 3-{2-[(4-bromo-3-methyl-5-isothiazolyl)methylthio]ethylamino}-4-methylamino-1,2,5-thiadiazole 1,1-dioxide and

74c) 3-{2-[(3-methyl-5-isothiazolyl)methylthio]ethylamino}-4-methylamino-1,2,5-thiadiazole 1,1-dioxide,

75) 3-{2-[(5-Dimethylaminomethyl-2-furyl)methylthio]ethylamino}-4-{2-[3-isothiazolylmethylthio]-ethylamino}-1,2,5-thiadiazole 1,1-dioxide,

76) 3-{2-[(2-Amino-1,3,4-thiadiazol-5-yl)methylthio]ethylamino}-4-methylamino-1,2,5-thiadiazole 1,1-dioxide,

77a) 3-{3-[1,2,4-thiadiazol-3-ylthio]propylamino}-4-methylamino-1,2,5-thiadiazole 1,1-dioxide,

77b) 3-{2-[1,2,3-thiadiazol-4-yl)methylthio]ethylamino}-4-methylamino-1,2,5-thiadiazole 1,1-dioxide,

77c) 3-{2-[(3-hydroxy-1,2,5-thiadiazol-4-yl)methylthio]ethylamino}-4-methylamino-1,2,5-thiadiazole 1,1-dioxide and

77d) 3-{2-[(3-amino-1,2,5-thiadiazol-4-yl)methylthio]ethylamino}-4-methylamino-1,2,5-thiadiazole 1,1-dioxide,

78) 3-{2-[(5-Dimethylaminomethyl-2-furyl)methylthio]ethylamnino}-4-{2-[(3-hydroxy-1,2,5-thiadiazol-4-yl)methylthio]ethylamino}-1,2,5-thiadiazole 1,1-dioxide,

79) 3-{2-[(2-Amino-1,2,4-triazol-4-yl)methylthio]ethylamino}-4-methylamino-1,2,5-thiadiazole 1,1-dioxide,

80a) 3-{2-[(4-methyl-1,2,4-triazol-3-yl)methylthio]ethylamino}-4-methylamino-1,2,5-thiadiazole 1,1-dioxide,

80b) 3-{2-[(5-methyl-1,2,3-triazol-4-yl)methylthio]ethylamino}-4-methylamino-1,2,5-thiadiazole 1,1-dioxide and

80c) 3-methylamino-4-{2-[1,2,4-triazol-3-ylmethylthio]ethylamino}-1,2,5-thiadiazole 1,1-dioxide,

81) 3-{2-[(5-Dimethylaminomethyl-2-furyl)methylthio]ethylamino}-4-{2-[(5-methyl-1,2,3-triazol-4-yl)methylthio]ethylamino}-1,2,5-thiadiazole 1,1-dioxide,

82) 3-{2-[(2-Dimethylaminomethyl-4-thiazolyl)methylthio]ethylamino}-4-dimethylamino-1,2,5-thiadiazole 1-oxide,

83a) 3-{2-[(2-dimethylaminomethyl-4-thiazolyl)methylthio]ethylamino}-4-(1-pyrrolidinyl)-1,2,5-thiadiazole 1-oxide,

83b) 3-{2-[(2-dimethylaminomethyl-4-thiazolyl)methylthio]ethylamino}-4-(1-piperidinyl)-1,2,5-thiadiazole 1-oxide,

83c) 3-{2-[(2-dimethylaminomethyl-4-thiazolyl)methylthio]ethylamino}-4-(morpholinyl)-1,2,5-thiadiazole 1-oxide,

83d) 3-{2-[(2-dimethylaminomethyl-4-thiazolyl)methylthio]ethylamino}-4-(4-thiomorpholinyl)-1,2,5-thiadiazole 1-oxide,

83e) 3-{2-[(2-dimethylaminomethyl-4-thiazolyl)methylthio]ethylamino}-4-(1-piperazinyl)-1,2,5-thiadiazole 1-oxide,

83f) 3-{2-[(2-dimethylaminomethyl-4-thiazolyl)methylthio]ethylamino}-4-(4-acetyl-1-piperazinyl)-1,2,5-thiadiazole 1-oxide,

83g) 3-{2-[(2-dimethylaminomethyl-4-thiazolyl)methylthio]ethylamino}-4-(4-methyl-1-piperazinyl)-1,2,5-thiadiazole 1-oxide,

83h) 3-{2-[(2-dimethylaminomethyl-4-thiazolyl)methylthio]ethylamino}-4-(1-hexamethyleneimino)-1,2,5-thiadiazole 1-oxide,

83i) 3-{2-[(2-dimethylaminomethyl-4-thiazolyl)methylthio]ethylamino}-4-(1-homopiperazinyl)-1,2,5-thiadiazole 1-oxide,

84) 3-{2-[(5-Dimethylaminomethyl-2-furyl)methylthio]ethylamino}-4-ethylamino-1,2,5-thiadiazole 1-oxide, mp 68—74°;

*Anal.* Calcd for $C_{14}H_{23}N_5O_2S_2$: C, 47.04; H, 6.48; N, 19.59; S, 17.94.
Found (corr. for 1.24% $H_2O$): C, 46.54; H, 6.33; N, 19.37; S, 17.96.

85) 3-{2-[(5-Dimethylaminomethyl-2-furyl)methylthio]ethylamino}-4-propylamino-1,2,5-thiadiazole 1,1-dioxide

mp 164—166°; the NMR spectrum (100 MHz) in $d_6$ dimethyl sulfoxide showed the presence of approximately 0.9 moles of methanol.

*Anal.* Calcd for $C_{15}H_{25}N_5O_3S_2 \cdot 0.9CH_4O$: C, 45.86; H, 6.92; N, 16.82; S, 15.40.
Found: C, 45.60; H, 6.93; N, 17.03; S, 15.47.

86) 3-Amino-4-{2-[(5-dimethylaminomethyl-2-furyl)methylthio]ethylamino}-1,2,5-thiadiazole 1,1-oxide mp 139—142°;

*Anal.* Calcd for $C_{12}H_{19}N_5O_2S_2$: C, 43.75; H, 5.81; N, 21.26; S, 19.46.
Found: C, 43.71; H, 6.05; N, 21.32; S, 19.51.

87) 3-Amino-4-{2-[(5-dimethylaminomethyl-2-furyl)methylthio]ethylamino}-1,2,5-thiadiazole 1,1-dioxide mp 156—158°;

*Anal.* Calcd for $C_{12}H_{19}N_5O_3S_2$: C, 41.72; H, 5.54; N, 20.28; S, 18.56.
Found: C, 41.50; H, 5.52; N, 20.33; S, 18.74.

88) 3-Amino-4-{2-[(2-guanidinothiazol-4-yl)methylthio]ethylamino}-1,2,5-thiadiazole 1-oxide mp 167—170° (dec);

*Anal.* Calcd for $C_9H_{14}N_8OS_3$: C, 31.20; H, 4.07; N, 32.35; S, 27.76.
Found (corr. for 0.48% $H_2O$): C, 30.39; H, 3.97; N, 32.25; S, 27.91.

Recrystallization of the crude product from 95% ethanol yielded the title compound as a monohydrate, mp 136—138° (dec).

*Anal.* Calcd for $C_9H_{14}N_8OS_3 \cdot H_2O$: C, 29.66; H, 4.42; N, 30.75; S, 26.39.
Found: C, 29.92; H, 4.42; N, 30.84; S, 26.58.

A sample of the product as the free base was suspended in 95% ethanol, treated with one equivalent of aqueous 6.0N hydrochloric acid and filtered to yield the hydrochloride salt, mp 200—201°C (dec.)

*Anal.* Calcd for $C_9H_{15}ClN_8OS_3$: C, 28.23; H, 3.95; N, 29.26; Cl, 9.26.
Found (corr. for 1.02% $H_2O$): C, 28.26; H, 3.83; N, 29.41; Cl, 9.53.

89) 3-Benzylamino-4-{2-[(5-dimethylaminomethyl-2-furyl)methylthio]ethylamino}-1,2,5-thiadiazole 1,1-dioxide
mp 152° (dec);
*Anal.* Calcd for $C_{19}H_{25}N_5O_3S_2 \cdot CH_4O$: C, 51.37; H, 6.25; N, 14.98.
Found: C, 51.51; H, 6.05; N, 14.78.

90) 3-{2-[(3-{Dimethylaminomethyl)phenyl)methylthio]ethylamino}-4-methylamino-1,2,5-thiadiazole 1,1-dioxide
mp 152—158°
*Anal.* Calcd for $C_{15}H_{23}N_5O_2S_2 \cdot 0.6\ CH_4O$: C, 48.20; H, 6.59; N, 18.02; S, 16.49.
Found: C, 47.99; H, 6.78; N, 17.81; S, 16.09.

91) 3-Amino-4-{2-[(3-{dimethylaminomethyl}phenyl)methylthio]ethylamino}-1,2,5-thiadiazole 1-oxide
mp 122—125°;
*Anal.* Calcd for $C_{14}H_{21}N_5OS_2$: C, 49.53; H, 6.23; N, 20.63; S, 18.89.
Found: C, 49.18; H, 6.08; N, 20.93; S, 19.25.

92) 3-{2-[(5-Dimethylaminomethyl-2-thienyl)methylthio]ethylamino-4-methylamino-1,2,5-thiadiazole 1-oxide
mp 98.5—102°;
*Anal.* Calcd for $C_{13}H_{21}N_5OS_3$: C, 43.42; H, 5.89; N, 19.48; S, 26.76.
Found: C, 43.70; H, 5.58; N, 19.71; S, 26.79.

93) 3-Amino-4-{4-(5-dimethylaminomethyl-2-furyl)butylamino}-1,2,5-thiadiazole 1,1-dioxide
mp 154—156° (dec).;
*Anal.* Calcd for $C_{13}H_{21}N_5O_3S$: C, 47.69; H, 6.47; N, 21.39; S, 9.80.
Found: C, 47.73; H, 6.28; N, 21.43; S, 9.84.

94) 3-Amino-4-{2-[(2-dimethylaminomethyl-4-thiazolyl)methylthio]ethylamino}-1,2,5-thiadiazole 1,1-dioxide
mp 60—65°;
*Anal.* Calcd for $C_{11}H_{18}N_6S_3O_2 \cdot 0.15C_3H_8O$: C, 37.02; H, 5.21; N, 22.62; S, 25.89.
Found (corr. for 2.79% $H_2O$): C, 36.75; H, 5.13; N, 21.75; S, 25.03.

95) 3-{2-[(2-Guanidinothiazol-5-yl)methylthio]ethylamino}-4-methylamino-1,2,5-thiadiazole 1,1-dioxide
mp 225—226°;
*Anal.* Calcd for $C_{10}H_{16}N_8O_2S_3$: C, 31.90; H, 4.28; N, 29.76; S, 25.55.
Found: C, 32.07; H, 4.14; N, 29.91; S, 25.60.

96) 3-Amino-4-{2-[(2-guanidinothiazol-5-yl)methylthio]ethylamino}-1,2,5-thiadiazole 1-oxide
mp 85—132°;
*Anal.* Calcd for $C_9H_{14}N_8OS_3 \cdot 0.3C_2H_3N$: C, 32.24; H, 4.22; N, 32.41; S, 26.71.
Found (corr. for 1.84% $H_2O$): C, 32.63; H, 4.33; N, 32.55; S, 26.62.

97) 3-Cyclopropylamino-4-{2-[(5-dimethylaminomethyl-2-furyl)methylthio]ethylamino}-1,2,5-thiadiazole 1,1-dioxide
mp 194—195° (dec.);
*Anal.* Calcd for $C_{15}H_{23}N_5O_3S_2 \cdot C_3H_8O$: C, 48.52; H, 7.01; N, 15.72
Found: C, 48.36; H, 6.95; N, 14.87.

98) 3-Cyclopropylmethylamino-4-{2-[(5-dimethylaminomethyl-2-furyl)methylthio]ethylamino}-1,2,5-thiadiazole 1,1-dioxide
mp 86—89° (dec.); the NMR spectrum (100 MHz) in $d_6$ dimethyl sulfoxide showed the presence of approximately 1.25 moles of methanol.
*Anal.* Calcd for $C_{16}H_{25}N_5O_3S_2 \cdot 1.25\ CH_4O$: C, 47.13; H, 6.88; N, 15.93.
Found (corr. for 0.68% $H_2O$): C, 47.40; H, 6.49; N, 15.77.

99) 3-{2-[(5-Dimethylaminomethyl-2-furyl)methylthio]ethylamino}-4-morpholino-1,2,5-thiadiazole 1,1-dioxide
mp 122—127°

*Anal.* Calcd for $C_{16}H_{25}N_5O_4S_2$: C, 46.24; H, 6.06; N, 16.86.
Found (corr. for 0.61% $H_2O$): C, 45.82; H, 6.06; N, 16.62.

100) 3-{2-[(5-Dimethylaminomethyl-2-furyl)methylthio]ethylamino}-4-(2-methoxyethylamino)-1,2,5-thiadiazole 1,1-dioxide
mp 56—58°;
*Anal.* Calcd for $C_{15}H_{25}N_5O_4S_2 \cdot 0.6\ C_3H_8O$: C, 45.90; H, 6.83; N, 15.93.
Found (corr. for 0.74% $H_2O$): C, 45.50; H, 6.72; N, 15.63.

101) 3-{2-[(5-Dimethylaminomethyl-2-furyl)methylthio]ethylamino}-4-pyrrolidino-1,2,5-thiadiazole 1,1-dioxide
mp 151—152°;
*Anal.* Calcd for $C_{16}H_{25}N_5O_3S_2$: C, 48.09; H, 6.31; N, 17.53.
Found: C, 48.00; H, 6.10; N, 17.71.

102) 3-{2-[(5-Dimethylaminomethyl-2-furyl)methylthio]ethylamino}-4-piperidino-1,2,5-thiadiazole 1,1-dioxide
mp 106—108°;
*Anal.* Calcd for $C_{18}H_{27}N_5O_3S_2$: C, 49.37; H, 6.58; N, 16.94.
Found (corr. for 0.2% $H_2O$): C, 49.17; H, 6.52; N, 17.14.

103) 3-Butylamino-4-{2-[(5-dimethylaminomethyl-2-furyl)methylthio]ethylamino}-1,2,5-thiadiazole 1,1-dioxide
*Anal.* Calcd for $C_{16}H_{27}N_5O_3S_2$: C, 47.86; H, 6.78; N, 17.44.
Found (corr. for 1.34% $H_2O$): C, 47.60; H, 6.81; N, 17.81.

104) 3-{2-[(5-Dimethylaminomethyl-2-furyl)methylthio]ethylamino}-4-[(2-pyridyl)methylamino]-1,2,5-thiadiazole 1,1-dioxide
mp 43—45°. A sample was recrystallized from absolute ethanol and the solid was heated under vacuum at 60° for 6 hours to give a melt. The melt was dissolved in hot isopropyl alcohol, collected by filtration at ambient temperature and dried under high vacuum to yield the title compound, mp 45—47°; the NMR spectrum (100 MHz) in $d_6$ dimethyl sulfoxide showed the presence of approximately 1.25 moles of isopropyl alcohol.
*Anal.* Calcd for $C_{18}H_{24}N_6O_3S_2 \cdot 1.25\ C_3H_8O$: C, 51.05; H, 6.70; N, 16.42.
Found (corr. for 0.58% $H_2O$): C, 51.08; H, 6.32; N, 16.03.

105) 3-{2-[(5-Dimethylaminomethyl-2-furyl)methylthio]ethylamino}-4-hydroxylamino-1,2,5-thiadiazole 1,1-dioxide
mp 203—205°;
*Anal.* Calcd for $C_{12}H_{19}N_5O_4S_2$: C, 39.87; H, 5.30; N, 19.38; S, 17.74.
Found (corr. for 1.18% $H_2O$): C, 39.53; H, 5.04; N, 19.61; S, 17.62.

106) 3-{2-[(5-Dimethylaminomethyl-2-furyl)methylthio]ethylamino}-4-dodecylamino-1,2,5-thiadiazole 1,1-dioxide
mp 136—139°;
*Anal.* Calcd for $C_{24}H_{45}N_5O_3S_2$: C, 55.89; H, 8.79; N, 13.58; S, 12.43.
Found: C, 56.16; H, 8.57; N, 13.38; S, 12.61.

107) 3-{2-[(5-Dimethylaminomethyl-2-furyl)methylthio]ethylamino}-4-methoxyamino-1,2,5-thiadiazole 1,1-dioxide
mp 224—226 (dec.);
*Anal.* Calcd for $C_{13}H_{21}N_5O_4S_2$: C, 41.59; H, 5.64; N, 18.65; S, 17.08.
Found (corr. for 0.79% $H_2O$): C, 41.25; H, 5.54; N, 18.50; S, 17.16.

108) 3-{2-[(5-Dimethylaminomethyl-2-thienyl)methylthio]ethylamino}-4-propylamino-1,2,5-thiadiazole 1,1-dioxide
mp 194—196° (dec.);
*Anal.* Calcd for $C_{15}H_{25}N_5O_2S_3$: C, 44.64; H, 6.24; N, 17.35; S, 23.84.
Found: C, 44.66; H, 6.02; N, 17.88; S, 23.87.

109) 3-Amino-4-{2-[(5-dimethylaminomethyl-2-thienyl)methylthio]ethylamino}-1,2,5-thiadiazole 1-oxide
mp 149—152° (dec.);

110) 3-{2-[(5-Dimethylaminomethyl-2-thienyl)methylthio]ethylamino}-4-[(3-pyridyl)methylamino]-1,2,5-thiadiazole 1,1-dioxide

mp 143—146.5°;
*Anal.* Calcd for $C_{18}H_{26}Cl_2N_6O_2S_3$: C, 41.13; H, 4.99; N, 15.99; S, 18.30.
Found (corr. for 2.04% $H_2O$): C, 41.25; H, 4.90; N, 16.18; S, 18.52.

111) 3-Amino-4-{2-[(5-dimethylaminomethyl-2-thienyl)methylthio]ethylamino}-1,2,5-thiadiazole 1,1-dioxide

The NMR spectrum (100 MHz) in $d_6$ dimethyl sulfoxide gave the following resonances δ: 6.88 (d, 1H); 6.78 (d, 1H); 4.03 (s, 2H); 3.61 (s, 2H); 3.54 (t, 2H); 2.74 (t, 2H); 2.22 (s, 6H); it also showed the presence of approximately 2/3 mole of methanol.

112) 3-Benzylamino-4-{2-[(5-dimethylaminomethyl-2-thienyl)methylthio]ethylamino}-1,2,5-thiadiazole 1,1-dioxide
mp 203—205.5° (dec.);
*Anal.* Calcd for $C_{19}H_{25}N_5O_2S_3$: C, 50.53; H, 5.58; N, 15.51; S, 21.30.
Found: C, 50.79; H, 5.34; N, 15.78; S, 20.94.

113) 3-[3-(3-Dimethylaminomethylphenoxy)propylamino]-4-methylamino-1,2,5-thiadiazole 1,1-dioxide
mp 140—145°;
*Anal.* Calcd for $C_{15}H_{24}ClN_5O_3S$: C, 46.20; H, 6.20; N, 17.96; S, 8.22; Cl, 9.09.
Found (corr for 3.79% $H_2O$): C, 46.21 H, 6.06; N, 18.24; S, 8.38; Cl, 9.05.

114) 3-{2-[(2-Dimethylaminomethylthiazol-5-yl)-methylthio]ethylamino}-4-methylamino-1,2,5-thiadiazole 1,1-dioxide
mp 170—172°
*Anal.* Calcd for $C_{12}H_{20}N_6O_2S_3$: C, 38.28; H, 5.36; N, 22.33; S, 25.56.
Found: C, 38.31; H, 5.32; N, 22.13; S, 25.96.

115) 3-{2-[(2-Guanidinothiazol-4-yl)methylthio]ethylamino}-4-methylamino-1,2,5-thiadiazole 1-oxide
mp 175—177°;
*Anal.* Calcd for $C_{10}H_{16}N_8OS_3$: C, 33.32; H, 4.47; N, 31.09; S, 26.68.
Found: C, 33.10; H, 4.42; N, 31.00; S, 26.51.

116) 3-{2-[(2-Guanidinothiazol-4-yl)methylthio]ethylamino}-4-hydroxy-1,2,5-thiadiazole 1-oxide
mp 148—151°;
*Anal.* Calcd for $C_9H_{13}N_7O_2S_3$: C, 31.11; H, 3.77; N, 28.22; S, 27.69.
Found (corr. for 5.52% $H_2O$): C, 30.95; H, 3.76; N, 28.27; S, 28.11.

117) 3-Amino-4-{2-[(2-{2-methylguanidino}thiazol-4-yl)methylthio]ethylamino}-1,2,5-thiadiazole 1-oxide
mp 86—91°;
*Anal.* Calcd for $C_{10}H_{16}N_8OS_3 \cdot 0.8\ C_2H_6O$: C, 35.06; H, 5.28; N, 28.20; S, 24.21.
Found (corr. for 1.64% $H_2O$): C, 35.66; H, 5.05; N, 28.33; S, 23.96.

118) 3-Amino-4-[3-(3-dimethylaminomethylphenoxy)propylamino]-1,2,5-thiadiazole 1-oxide
mp 165.5—166.5° (dec.);
*Anal.* Calcd for $C_{14}H_{21}N_5O_2S$: C, 51.99; H, 6.55; N, 21.66; S, 9.92.
Found: C, 51.58; H, 6.49; N, 22.03; S, 10.19.

119) 3-Amino-4-{2-[(2-methylaminothiazol-4-yl)methylthio]ethylamino}-1,2,5-thiadiazole 1-oxide
mp 180—183° (dec.);
*Anal.* Calcd for $C_9H_{14}N_6OS_3$: C, 33.94; H, 4.43; N, 26.39; S, 30.21.
Found (corr. for 1.41% $H_2O$): C, 33.96; H, 4.11; N, 26.27; S, 30.44.

120) 3-Amino-4-{2-[(2-{2,3-dimethylguanidino}thiazol-4-yl)methylthio]ethylamino}-1,2,5-thiadiazole 1-oxide
mp 201—203° (dec.);
*Anal.* Calcd for $C_{11}H_{18}N_8OS_3$: C, 35.28; H, 4.84; N, 29.92; S, 25.69.
Found (corr. for 0.88% $H_2O$): C, 34.93; H, 4.56; N, 30.27; S, 25.92.

121) 3,4-Bis-{2-[(2-guanidinothiazol-4-yl)methylthio]ethylamino}-1,2,5-thiadiazole 1-oxide
*Anal.* Calcd for $C_{16}H_{24}N_{12}OS_5 \cdot 0.11C_2H_6O$: C, 34.42; H, 4.39; N, 29.71; S, 28.33.
Found (corr. for 1.86% $H_2O$): C, 34.95; H, 4.41; N, 29.04; S, 27.71.

122) 3-{2-[(2-Aminothiazol-4-yl)methylthio]ethylamino}-4-methylamino-1,2,5-thiadiazole 1,1-dioxide
mp 200—201°;
*Anal.* Calcd for $C_9H_{14}N_6O_2S_3$: C, 32.32; H, 4.32; N, 25.13; S, 28.76.
Found: C, 32.25; H, 4.20; N, 25.06; S, 29.14.

123) 3-Amino-4-{2-[(2-dimethylaminothiazol-5-yl)methylthio]ethylamino}-1,2,5-thiadiazole 1-oxide
mp 131—133°;
*Anal.* Calcd for $C_{11}H_{17}N_6OS_3$: C, 38.13; H, 5.24; N, 24.26; S, 27.76.
Found (corr. for 0.49% $H_2O$): C, 37.86; H, 5.06; N, 24.34; S, 27.68.

124) 3-Amino-4-{2-[(2-aminothiazol-4-yl)methylthio]ethylamino}-1,2,5-thiadiazole 1-oxide
*Anal.* Calcd for $C_8H_{12}N_6OS_3 \cdot 0.4C_2H_6O$: C, 32.74; H, 4.50; N, 26.03; S, 29.80.
Found (corr. for 1.39% $H_2O$): C, 32.39; H, 4.28; N, 28.39; S, 30.02.

125) 3-Methylamino-4-{2-[(2-{2,3-dimethylguanidino}thiazol-4-yl)methylthio]ethylamino}-1,2,5-thiadiazole 1,1-dioxide
mp 132—137°;
*Anal.* Calcd for $C_{12}H_{20}N_8O_2S_3$: C, 35.63; H, 4.98; N, 27.70; S, 23.78.
Found (corr. for 4.78% $H_2O$): C, 35.74; H, 5.04; N, 27.87; S, 23.56.

126) 3-{2-[(2-Dimethylaminothiazol-4-yl)methylthio]ethylamino}-4-amino-1,2,5-thiadiazole 1-oxide
mp 116—122°;
*Anal.* Calcd for $C_{10}H_{16}N_6OS_3 \cdot \frac{1}{3}C_2H_6O$: C, 36.83; H, 5.22; N, 24.16.
Found (corr. for 11.92% $H_2O$): C, 36.61; H, 4.06; N, 24.22.

127) 3-{2-[(2-Dimethylaminothiazol-4-yl)methylthio]ethylamino}-4-methylamino-1,2,5-thiadiazole 1,1-dioxide
mp 227—229°;

128) 3-{2-[(2-Imidazolidinyl}iminothiazol-4-yl)methylthio]ethylamino}-4-methylamino-1,2,5-thiadiazole 1,1-dioxide
mp 229—231°. Recrystallization from aqueous ethanol gave the title compound with mp 136—140° which resolidified with remelting at mp 219—224°.
*Anal.* Calcd for $C_{12}H_{18}N_8O_2S_3$: C, 35.81; H, 4.51; N, 27.84; S, 23.90.
Found (corr. for 4.59% $H_2O$): C, 35.51; H, 4.43; N, 27.98; S, 23.56.

129) 3-{2-[(5-Dimethylaminomethyl-2-thienyl)methylthio]ethylamino}-4-[(2-pyridyl)methylamino]-1,2,5-thiadiazole 1,1-dioxide
mp 162—164° (dec.);
*Anal.* Calcd for $C_{18}H_{24}N_6O_2S_3$: C, 47.76; H, 5.34; N, 18.57.
Found: C, 47.80; H, 5.32; N, 18.75.

130) 3-{2-[(5-Dimethylaminomethyl-2-thienyl)methylthio]ethylamino}-4-[(4-pyridyl)methylamino]-1,2,5-thiadiazole 1,1-dioxide
mp 88—90°;
*Anal.* Calcd for $C_{18}H_{24}N_6O_2S_3$: C, 47.76; H, 5.34; N, 18.57.
Found (corr. for 3.73% $H_2O$): C, 47.54; H, 5.32; N, 19.09.

131) 3-{2-[(5-Dimethylaminomethyl-2-thienyl)methylthio]ethylamino}-4-ethylamino-1,2,5-thiadiazole 1,1-dioxide
mp 246—247° (dec.);
*Anal.* Calcd for $C_{14}H_{24}ClN_5O_2S_3$: C, 39.47; H, 5.68; N, 16.44; Cl, 8.32.
Found: C, 39.81; H, 5.74; N, 16.62; Cl, 8.20.

132) 3-Methylamino-4-[3-(3-piperidinomethylphenoxy)propylamino]-1,2,5-thiadiazole 1,1-dioxide
mp 182—184°;
*Anal.* Calcd for $C_{18}H_{27}N_5O_3S$: C, 54.94; H, 6.92; N, 17.80; S, 8.15.
Found: C, 54.90; H, 7.07; N, 18.14; S, 8.29.

133) 3-Amino-4-[3-(3-piperidinomethylphenoxy)propylamino]-1,2,5-thiadiazole 1-oxide
mp 155—157° (dec.);
*Anal.* Calcd for $C_{17}H_{25}N_5O_2S$: C, 56.17; H, 6.93; N, 19.27; S, 8.82.
Found: C, 55.97; H, 7.04; N, 19.57; S, 8.63.

134) 3-Amino-4-[3-(3-piperidinomethylphenoxy)propylamino]-1,2,5-thiadiazole 1,1-dioxide,
The NMR spectrum (100 MHz) in $d_6$ dimethyl sulfoxide showed the following resonances δ: 7.2 (m, 1H); 6.9 (m, 3H); 4.1 (t, 2H); 3.5 (t, 2H); 3.4 (s, 2H); 2.3 (m, 4H); 2.0 (m, 2H); 1.4 (broad s, 6H).

135) 3-{2-[(5-Dimethylaminomethyl-2-thienyl)methylthio]ethylamino}-4-(3,4-methylenedioxybenzylamino)-1,2,5-thiadiazole 1,1-dioxide

mp 180—182°;

*Anal.* Calcd for $C_{20}H_{25}N_5O_4S_3$: C, 48.46; H, 5.08; N, 14.13.

Found (corr. for 0.38% $H_2O$): C, 48.92; H, 4.88; N, 14.52.

136) 3-Amino-4-{2-[(6-dimethylaminomethyl-2-pyridyl)methylthio]ethylamino}-1,2,5-thiadiazole 1-oxide

137) 3-Amino-4-{2-[(6-dimethylaminomethyl-2-pyridyl)methylthio]ethylamino}-1,2,5-thiadiazole 1,1-dioxide

138) 3-{2-[(5-Guanidino-1,2,4-thiadiazol-3-yl)methylthio]ethylamino}-4-methylamino-1,2,5-thiadiazole 1,1-dioxide

139) 3-Amino-4-{2-[(5-guanidino-1,2,4-thiadiazol-3-yl)methylthio]ethylamino}-1,2,5-thiadiazole 1,1-dioxide

140) 3-Amino-4-{2-[(5-guanidino-1,2,4-thiadiazol-3-yl)methylthio]ethylamino}-1,2,5-thiadiazole 1-oxide

141) 3-{2-[(5-Guanidino-1,2,4-oxadiazol-3-yl)methylthio]ethylamino}-4-methylamino-1,2,5-thiadiazole 1,1-dioxide

142) 3-Amino-4-{2-[(5-guanidino-1,2,4-oxadiazol-3-yl)methylthio]ethylamino}-1,2,5-thiadiazole 1,1-dioxide

143) 3-Amino-4-{2-[(5-guanidino-1,2,4-oxadiazol-3-yl)methylthio]ethylamino}-1,2,5-thiadiazole 1-oxide

144a) 3-Methylamino-4-[3-(3-pyrrolidinomethylphenoxy)propylamino]-1,2,5-thiadiazole 1,1-dioxide, mp 156—157°C,

144b) 3-Methylamino-4-{3-[3-(4-methylpiperidino)methylphenoxy]propylamino}-1,2,5-thiadiazole 1,1-dioxide, mp 186—189°C,

144c) 3-Methylamino-4-[3-(3-homopiperidinomethylphenoxy)propylamino]-1,2,5-thiadiazole 1,1-dioxide, mp 174—176°C as the hydrochloride,

144d) 3-Methylamino-4-[3-(3-morpholinomethylphenoxy)propylamino]-1,2,5-thiadiazole 1,1-dioxide, mp 162—163°C, and

144e) 3-Methylamino-4-{3-[3-(N-methylpiperazino)methylphenoxy]propylamino}-1,2,5-thiadiazole 1,1-dioxide,

145a) 3-Amino-4-[3-(3-pyrrolidinomethylphenoxy]propylamino]-1,2,5-thiadiazole 1-oxide, mp 168—170°C (dec.),

145b) 3-Amino-4-{3-[3-(4-methylpiperidino)methylphenoxy]propylamino}-1,2,5-thiadiazole 1-oxide, mp 157—159°C,

145c) 3-Amino-4-[3-(3-homopiperidinomethylphenoxy]propylamino]-1,2,5-thiadiazole 1-oxide mp 167—169°C,

145d) 3-Amino-4-{3-[3-(heptamethyleneiminomethyl)phenoxy]propylamino}-1,2,5-thiadiazole 1-oxide mp 154—157°C,

145e) 3-Amino-4-[3-(3-morpholinomethylphenoxy)propylamino]-1,2,5-thiadiazole 1-oxide and,

145f) 3-Amino-4-{3-[3-(N-methylpiperazino)methylphenoxy]propylamino}-1,2,5-thiadiazole 1-oxide,

146a) 3-Amino-4-[3-(3-pyrrolidinomethylphenoxy)propylamino]-1,2,5-thiadiazole 1,1-dioxide, mp 160—163°C (dec.),

146b) 3-Amino-4-{3-[3-(4-methylpiperazino)methylphenoxy]propylamino}-1,2,5-thiadiazole 1,1-dioxide,

146c) 3-Amino-4-[3-(3-homopiperidinomethylphenoxy)propylamino]-1,2,5-thiadiazole 1,1-dioxide,

146d) 3-Amino-4-[3-(3-morpholinomethylphenoxy)propylamino]-1,2,5-thiadiazole 1,1-dioxide, mp 172—174°C (dec.), and

146e) 3-Amino-4-{3-[3-(N-methylpiperazino)methylphenoxy]propylamino}-1,2,5-thiadiazole 1,1-dioxide,

147a) 3-Ethylamino-4-[3-(3-piperidinomethylphenoxy)propylamino]-1,2,5-thiadiazole 1,1-dioxide,

147b) 3-Propylamino-4-[3-(3-piperidinomethylphenoxy)propylamino]-1,2,5-thiadiazole 1,1-dioxide,

147c) 3-Butylamino-4-[3-(3-piperidinomethylphenoxy)propylamino]-1,2,5-thiadiazole 1,1-dioxide,

147d) 3-Allylamino-4-[3-(3-piperidinomethylphenoxy)propylamino]-1,2,5-thiadiazole 1,1-dioxide,

147e) 3-(2-Propynyl)amino-4-[3-(3-piperidinomethylphenoxy)propylamino]-1,2,5-thiadiazole 1,1-dioxide,

147f) 3-(Cyclopropylamino)-4-[3-(3-piperidinomethylphenoxy)propylamino]-1,2,5-thiadiazole 1,1-dioxide,

147g) 3-[(Cyclopropyl)methylamino)-4-[3-(3-piperidinomethylphenoxy)propylamino]-1,2,5-thiadiazole 1,1-dioxide,

147h) 3-(2-Hydroxyethylamino)-4-[3-(3-piperidinomethylphenoxy)propylamino]-1,2,5-thiadiazole 1,1-dioxide,

147i) 3-(2-Methoxyethylamino)-4-[3-(3-piperidinomethylphenoxy)propylamino]-1,2,5-thiadiazole 1,1-dioxide,

147k) 3-(2,2,2-trifluoroethylamino)-4-[3-(3-piperidinomethylphenoxy)propylamino]-1,2,5-thiadiazole 1,1-dioxide,

147l) 3-(2-Fluoroethylamino)-4-[3-(3-piperidinomethylphenoxy)propylamino]-1,2,5-thiadiazole 1,1-dioxide,

147m) 3-Hydroxyamino-4-[3-(3-piperidinomethylphenoxy)propylamino]-1,2,5-thiadiazole 1,1-dioxide,

147n) 3-(3-Cyanopropylamino)-4-[3-(3-piperidinomethylphenoxy)propylamino]-1,2,5-thiadiazole 1,1-dioxide,

147o) 3-Benzylamino)-4-[3-(3-piperidinomethylphenoxy)propylamino]-1,2,5-thiadiazole 1,1-dioxide,

147p) 3-(3-Methoxybenzylamino)-4-[3-(3-piperidinomethylphenoxy)propylamino]-1,2,5-thiadiazole 1,1-dioxide,

147q) 3-(4-Methoxybenzylamino)-4-[3-(3-piperidinomethylphenoxy)propylamino]-1,2,5-thiadiazole 1,1-dioxide,

147r) 3-(3,4-Dimethoxybenzylamino)-4-[3-(3-piperidinomethylphenoxy)propylamino]-1,2,5-thiadiazole 1,1-dioxide,

147s) 3-(3,4-Methylenedioxybenzylamino)-4-[3-(3-piperidinomethylphenoxy)propylamino]-1,2,5-thiadiazole 1,1-dioxide,

147t) 3-(4-Chlorobenzylamino)-4-[3-(3-piperidinomethylphenoxy)propylamino]-1,2,5-thiadiazole 1,1-dioxide,

147u) 3-[(2-Pyridyl)methylamino]-4-[3-(3-piperidinomethylphenoxy)propylamino]-1,2,5-thiadiazole 1,1-dioxide,

147v) 3-[(3-Pyridyl)methylamino]-4-[3-(3-piperidinomethylphenoxy)propylamino]-1,2,5-thiadiazole 1,1-dioxide, and

147w) 3-[(4-Pyridyl)methylamino]-4-[3-(3-piperidinomethylphenoxy)propylamino]-1,2,5-thiadiazole 1,1-dioxide,

148a) 3-Ethylamino-4-[3-(3-piperidinomethylphenoxy)propylamino]-1,2,5-thiadiazole 1-oxide,

148b) 3-Propylamino-4-[3-(3-piperidinomethylphenoxy)propylamino]-1,2,5-thiadiazole 1-oxide,

148c) 3-Butylamino-4-[3-(3-piperidinomethylphenoxy)propylamino]-1,2,5-thiadiazole 1-oxide,

148d) 3-Allylamino-4-[3-(3-piperidinomethylphenoxy)propylamino]-1,2,5-thiadiazole 1-oxide,

148e) 3-(2-Propynyl)amino-4-[3-(3-piperidinomethylphenoxy)propylamino]-1,2,5-thiadiazole 1-oxide,

148f) 3-(Cyclopropylamino)-4-[3-(3-piperidinomethylphenoxy)propylamino]-1,2,5-thiadiazole 1-oxide,

148g) 3-[(Cyclopropyl)methylamino]-4-[3-(3-piperidinomethylphenoxy)propylamino]-1,2,5-thiadiazole 1-oxide,

148h) 3-(2-Hydroxyethylamino)-4-[3-(3-piperidinomethylphenoxy)propylamino]-1,2,5-thiadiazole 1-oxide,

148i) 3-(2-Methoxyethylamino)-4-[3-(3-piperidinomethylphenoxy)propylamino]-1,2,5-thiadiazole 1-oxide,

148k) 3-(2,2,2-Trifluoroethylamino)-4-[3-(3-piperidinomethylphenoxy)propylamino]-1,2,5-thiadiazole 1-oxide,

148l) 3-(2-Fluoroethylamino)-4-[3-(3-piperidinomethylphenoxy)propylamino]-1,2,5-thiadiazole 1-oxide,

148m) 3-Hydroxyamino-4-[3-(3-piperidinomethylphenoxy)propylamino]-1,2,5-thiadiazole 1-oxide,

148n) 3-(3-Cyanopropylamino)-4-[3-(3-piperidinomethylphenoxy)propylamino]-1,2,5-thiadiazole 1-oxide,

148o) 3-Benzylamino-4-[3-(3-piperidinomethylphenoxy)propylamino]-1,2,5-thiadiazole 1-oxide,

148p) 3-(3-Methoxybenzylamino)-4-[3-(3-piperidinomethylphenoxy)propylamino]-1,2,5-thiadiazole 1-oxide,

148q) 3-(4-Methoxybenzylamino)-4-[3-(3-piperidinomethylphenoxy)propylamino]-1,2,5-thiadiazole 1-oxide,

148r) 3-(3,4-Dimethoxybenzylamino)-4-[3-(3-piperidinomethylphenoxy)propylamino]-1,2,5-thiadiazole 1-oxide,

148s) 3-(3,4-Methylenedioxybenzylamino)-4-[3-(3-piperidinomethylphenoxy)propylamino]-1,2,5-thiadiazole 1-oxide,

148t) 3-(4-Chlorobenzylamino)-4-[3-(3-piperidinomethylphenoxy)propylamino]-1,2,5-thiadiazole 1-oxide,

148u) 3-[(2-Pyridyl)methylamino]-4-[3-(3-piperidinomethylphenoxy)propylamino]-1,2,5-thiadiazole 1-oxide,

148v) 3-[(3-Pyridyl)methylamino]-4-[3-(3-piperidinomethylphenoxy)propylamino]-1,2,5-thiadiazole 1-oxide, mp 139.5—143°C, and

148w) 3-[(4-Pyridyl)methylamino]-4-[3-(3-piperidinomethylphenoxy)propylamino]-1,2,5-thiadiazole 1-oxide.

149) 3-[(3-Pyridyl)methylamino]-4-[3-(3-piperidinomethylphenoxy)propylamino]-1,2,5-thiadiazole 1-oxide, mp 139.5—143°;

| | | | | |
|---|---|---|---|---|
| *Anal.* Calcd for $C_{22}H_{30}N_6O_2S$: | C, 60.77; | H, 6.65; | N, 18.49; | S, 7.04. |
| Found: | C, 60.66; | H, 6.64; | N, 18.22; | S, 7.02. |

150) 3-Amino-4-[3-(3-guanidinophenoxy)propylamino]-1,2,5-thiadiazole 1-oxide, TLC [silica gel/$CH_2Cl_2$:$CH_3OH$ (4:1)] gave Rf=0.21.

The NMR spectrum (60 MHz) in $d_6$ dimethyl sulfoxide gave the following resonances δ: 9.33 (s, 1H); 8.43 (s, 2H); 7.52 (m, 4H); 7.43 (m, 1H); 6.83 (m, 3H); 4.13 (broad t, 2H); 3.51 (broad t, 2H); 2.10 (broad t, 2H).

151) 3-Amino-4-{2-[(5-piperidinomethyl-2-furyl)methylthio]ethylamino}-1,2,5-thiadiazole 1-oxide, mp 161—163°;

| | | | | |
|---|---|---|---|---|
| *Anal.* Calcd for $C_{15}H_{23}N_5O_2S_2$: | C, 48.76; | H, 6.27; | N, 18.96; | S, 17.36. |
| Found: | C, 48.86; | H, 6.16; | N, 19.66; | S, 17.63. |

**Claims for the Contracting States: BE CH DE FR GB IT LI LU NL SE**

1. A pharmaceutical composition useful in the treatment of peptic ulcers, which comprises a peptic activity-inhibiting amount of pepstatin and an effective anti-ulcerogenic amount of at least one compound of the formula I:

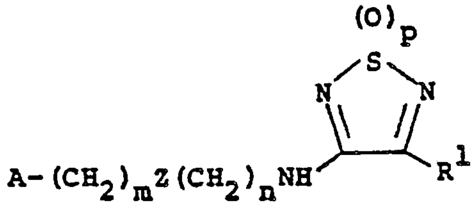

I

wherein

p is 1 or 2;

$R^1$ is hydroxy or $NR^2R^3$;

$R^2$ and $R^3$ are each independently hydrogen, $C_1$—$C_6$-alkyl, $C_2$—$C_6$-alkenyl, $C_2$—$C_6$-alkynyl, cyclo-$C_3$—$C_7$-alkyl-$C_1$—$C_6$-alkyl, hydroxy-$C_1$—$C_6$-alkyl, $C_1$—$C_6$-alkoxy-$C_1$—$C_6$-alkyl, $C_1$—$C_6$-alkylthio-$C_1$—$C_6$-alkyl, 2-fluoroethyl, 2,2,2-trifluoroethyl or cyano-$C_1$—$C_6$-alkyl, or, when $R^2$ is hydrogen, $R^3$ may also be cyclo-$C_3$—$C_7$-alkyl, amino-$C_1$—$C_6$-alkyl, $C_1$—$C_6$-alkylamino-$C_1$—$C_6$-alkyl, di-$C_1$—$C_6$-alkylamino-$C_1$—$C_6$-alkyl, pyrrolidino-$C_1$—$C_6$-alkyl, piperidino-$C_1$—$C_6$-alkyl, morpholino-$C_1$—$C_6$-alkyl, piperazino-$C_1$—$C_6$-alkyl, pyridyl-$C_1$—$C_6$-alkyl, substituted pyridyl-$C_1$—$C_6$-alkyl wherein the pyridyl ring may contain one substituent selected from $C_1$—$C_6$-alkyl, $C_1$—$C_6$-alkoxy, hydroxy, amino and halogen, amino, $C_1$—$C_6$-alkylamino, di-$C_1$—$C_6$-alkylamino, hydroxy, $C_1$—$C_6$-alkoxy, 2,3-dihydroxypropyl, cyano, amidino, $C_1$—$C_6$-alkylamidino, $A'$—$(CH_2)_{m'}Z'(CH_2)_{n'}$—, phenyl, phenyl-$C_1$—$C_6$-alkyl, substituted phenyl or substituted phenyl-$C_1$—$C_6$-alkyl, wherein the phenyl ring may contain one or two substituents independently selected from $C_1$—$C_6$-alkyl, hydroxy, $C_1$—$C_6$-alkoxy and halogen or one substituent selected from methylenedioxy, trifluoromethyl and di-$C_1$—$C_6$-alkylamino; or $R^2$ and $R^3$, taken together, may be —$CH_2CH_2X(CH_2)_r$—;

r is an integer of from 1 to 3, inclusive;

X is methylene, sulfur, oxygen or N—$R^4$, provided that, when r is 1, X is methylene;

$R^4$ is hydrogen, $C_1$—$C_6$-alkyl, $C_2$—$C_6$-alkenyl, $C_2$—$C_6$-alkynyl, $C_1$—$C_6$-alkanoyl or benzoyl;

m and m' each are independently an integer of from zero to 2, inclusive;

n and n' each are independently an integer of from 2 to 4, inclusive;

Z and Z' each are independently sulfur, oxygen or methylene;

A and A' each are independently phenyl, imidazolyl, thiazolyl, isothiazolyl, oxazolyl, isoxazolyl, triazolyl, thiadiazolyl, oxadiazolyl, furyl, thienyl or pyridyl; provided that A and A' independently may contain one or two substituents, the first substituent being selected from $C_1$—$C_6$-alkyl, hydroxy, trifluoromethyl, halogen, amino, hydroxymethyl, $C_1$—$C_6$-alkoxy,

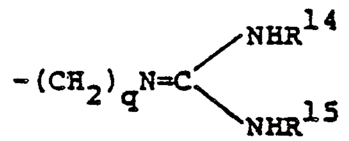

and $-(CH_2)_qNR^5R^6$,

and the second substituent being selected from $C_1$—$C_6$-alkyl, hydroxy, trifluoromethyl, halogen, amino, hydroxymethyl and $C_1$—$C_6$-alkoxy;

q is an integer of from 0 to 6, inclusive; $R^{14}$ and $R^{15}$ independently are hydrogen or $C_1$—$C_6$-alkyl, or, if $R^{14}$ is hydrogen, $R^{15}$ also may be $C_1$—$C_6$-alkanoyl or benzoyl, or $R^{14}$ and $R^{15}$, taken together, may be ethylene; and

$R^5$ and $R^6$ each are independently hydrogen, $C_1$—$C_6$-alkyl, $C_2$—$C_6$-alkenyl, $C_2$—$C_6$-alkynyl, $C_1$—$C_6$-alkoxy-$C_1$—$C_6$-alkyl, cyclo-$C_3$—$C_7$-alkyl, phenyl or phenyl-$C_1$—$C_6$-alkyl, provided that $R^5$ and $R^6$ may not both be cyclo-$C_3$—$C_7$-alkyl or phenyl; or $R^5$ and $R^6$, taken together with the nitrogen atom to which they are attached, may be pyrrolidino, methylpyrrolidino, dimethylpyrrolidino, morpholino, thiomorpholino, piperidino, methylpiperidino, dimethylpiperidino, hydroxypiperidino, N-methylpiperazino, homopiperidino, heptamethyleneimino or octamethyleneimino, or a nontoxic, pharmaceutically acceptable salt, hydrate or solvate thereof.

2. A composition of Claim 1 wherein in Formula I $R^1$ is $NR^2R^3$ and $R^2$ and $R^3$ are as defined in claim 1.

3. A composition of Claim 1 wherein the compound of Formula I has the structure

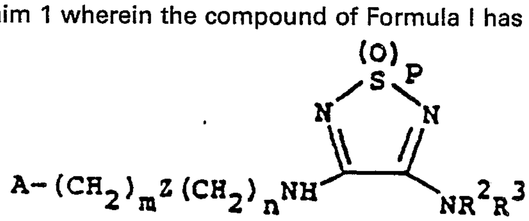

wherein p is 1 or 2;

$R^2$ and $R^3$ each are independently hydrogen, $C_1$—$C_6$-alkyl, $C_2$—$C_6$-alkenyl, $C_2$—$C_6$-alkynyl, cyclo-$C_3$—$C_7$-alkyl-$C_1$—$C_6$-alkyl, hydroxy-$C_1$—$C_6$-alkyl, $C_1$—$C_6$-alkoxy-$C_1$—$C_6$-alkyl, 2-fluoroethyl or 2,2,2-trifluoroethyl, or, when $R^2$ is hydrogen, $R^3$ also may be pyrrolidino-$C_1$—$C_6$-alkyl, piperidino-$C_1$—$C_6$-alkyl, morpholino-$C_1$—$C_6$-alkyl, piperazino-$C_1$—$C_6$-alkyl, pyridyl-$C_1$—$C_6$-alkyl, substituted pyridyl-$C_1$—$C_6$-alkyl wherein the pyridyl ring may contain one substituent selected from $C_1$—$C_6$-alkyl, $C_1$—$C_6$-alkoxy, hydroxy, amino and halogen, hydroxy, $A'$—$(CH_2)_{m'}Z'(CH_2)_{n'}$—, phenyl-$C_1$—$C_6$-alkyl or substituted phenyl-$C_1$—$C_6$-alkyl, wherein the phenyl ring may contain one or two substituents independently selected from $C_1$—$C_6$-alkyl, hydroxy, $C_1$—$C_6$-alkoxy and halogen or one substituent selected from methylenedioxy, trifluoromethyl and di-$C_1$—$C_6$-alkylamino;

m and m' each are independently an integer of from zero to 2, inclusive;

n and n' each are independently an integer of from 2 to 4, inclusive;

Z and Z' each are independently sulfur, oxygen or methylene;

A and A' each are independently phenyl, imidazolyl, thiazolyl, oxazolyl, thiadiazolyl, oxadiazolyl, furyl, thienyl or pyridyl; provided that A and A' independently may contain one or two substituents, the first substituent being selected from $C_1$—$C_6$-alkyl, hydroxy, trifluoromethyl, halogen, amino, hydroxymethyl, $C_1$—$C_6$-alkoxy,

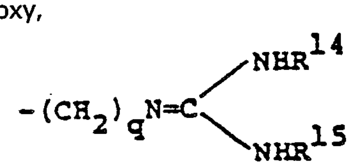

and $-(CH_2)_qNR^5R^6$,

and the second substituent being selected from $C_1$—$C_6$-alkyl, hydroxy, trifluoromethyl, halogen, amino, hydroxymethyl and $C_1$—$C_6$-alkoxy;

q is an integer of from 0 to 6, inclusive; $R^{14}$ and $R^{15}$ independently are hydrogen or $C_1$—$C_6$-alkyl, or $R^{14}$ and $R^{15}$, taken together, may be ethylene; and

$R^5$ and $R^6$ each are independently hydrogen, $C_1$—$C_6$-alkyl, $C_2$—$C_6$-alkenyl or $C_2$—$C_6$-alkynyl; or $R^5$ and $R^6$, taken together with the nitrogen atom to which they are attached, may be pyrrolidino, methylpyrrolidino, dimethylpyrrolidino, morpholino, thiomorpholino, piperidino, methylpiperidino, dimethylpiperidino, hydroxypiperidino, N-methylpiperazino, homopiperidino, heptamethyleneimino or octamethyleneimino, or a nontoxic pharmaceutically acceptable salt, hydrate or solvate thereof.

4. A composition of Claim 1 wherein the compound of Formula I has the structure

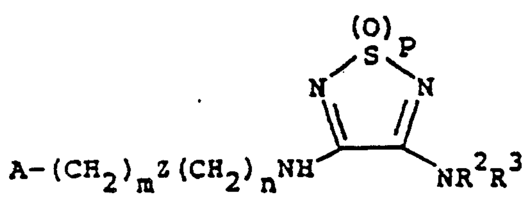

wherein p is 1 or 2;

$R^2$ and $R^3$ each are independently hydrogen, $C_1$—$C_6$-alkyl, $C_2$—$C_6$-alkenyl, $C_2$—$C_6$-alkynyl or cyclo-$C_3$—$C_7$-alkyl-$C_1$—$C_6$-alkyl, or, when $R^2$ is hydrogen, $R^3$ also may be pyridyl-$C_1$—$C_6$-alkyl, substituted pyridyl-$C_1$—$C_6$-alkyl wherein the pyridyl ring may contain one substituent selected from $C_1$—$C_6$-alkyl, $C_1$—$C_6$-alkoxy, hydroxy, amino and halogen, $A'$—$(CH_2)_{m'}Z'(CH_2)_{n'}$—, phenyl-$C_1$—$C_6$-alkyl or 3,4-methylenedioxybenzyl;

m and m' each are independently zero or 1;

n and n' each are independently 2 or 3;

Z and Z' each are independently sulfur, oxygen or methylene;

A and A' each are independently phenyl, imidazolyl, thiazolyl, furyl, thienyl or pyridyl; provided that A and A' independently may contain one or two substituents, the first substituent being selected from $C_1$—$C_6$-alkyl,

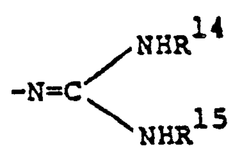

and $-CH_2NR^5R^6$,

and the second substituent being selected from $C_1$—$C_6$-alkyl;

$R^{14}$ and $R^{15}$ independently are hydrogen or $C_1$—$C_6$-alkyl, or $R^{14}$ and $R^{15}$, taken together, may be ethylene; and

$R^5$ and $R^6$ each are independently hydrogen or $C_1$—$C_6$-alkyl; or $R^5$ and $R^6$, taken together with the nitrogen atom to which they are attached, may be pyrrolidino, methylpyrrolidino, dimethylpyrrolidino, morpholino, thiomorpholino, piperidino, methylpiperidino, dimethylpiperidino, hydroxypiperidino, N-methylpiperazino, homopiperidino, heptamethyleneimino or octamethyleneimino, or a nontoxic, pharmaceutically acceptable salt, hydrate or solvate thereof.

5. A composition of Claim 1 wherein the compound of Formula I has the structure

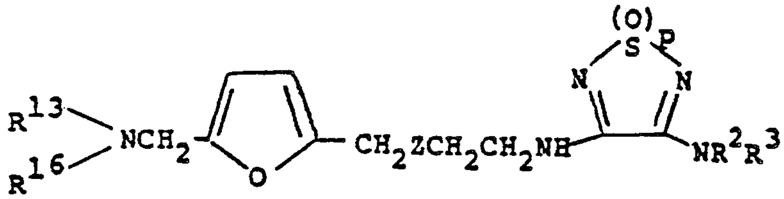

wherein p is 1 or 2; Z is sulfur or methylene; $R^2$ and $R^3$ each are independently hydrogen or $C_1$—$C_6$-alkyl, or, when $R^2$ is hydrogen, $R^3$ also may be $C_2$—$C_6$-alkenyl, $C_2$—$C_6$-alkynyl, phenyl-$C_1$—$C_6$-alkyl, cyclo-$C_3$—$C_7$-alkyl-$C_1$—$C_6$-alkyl, pyridylmethyl or

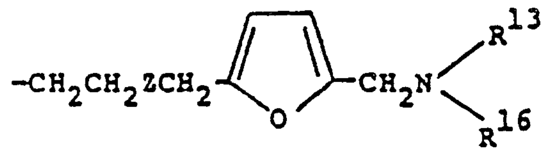 ;

$R^{16}$ is methyl and $R^{13}$ is hydrogen or methyl, or $R^{16}$ and $R^{13}$, taken together with the nitrogen atom to which they are attached, may be piperidino; or a nontoxic pharmaceutically acceptable salt, hydrate or solvate thereof.

6. A composition of Claim 1 wherein the compound of Formula I has the structure

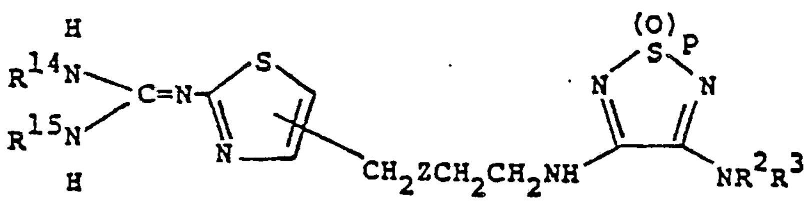

wherein p is 1 or 2; Z is sulfur or methylene; $R^{14}$ and $R^{15}$ independently are hydrogen or methyl, or, $R^{14}$ and $R^{15}$, taken together, may be ethylene; and $R^2$ and $R^3$ each are independently hydrogen or $C_1$—$C_6$-alkyl, or, when $R^2$ is hydrogen, $R^3$ also may be $C_2$—$C_6$-alkenyl, $C_2$—$C_6$-alkynyl, pyridylmethyl,

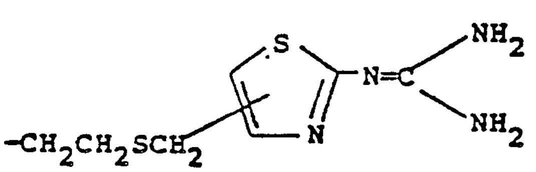 or 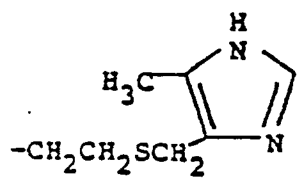 ;

or a nontoxic, pharmaceutically acceptable salt, hydrate or solvate thereof.

7. A composition of Claim 1 wherein the compound of Formula I has the structure

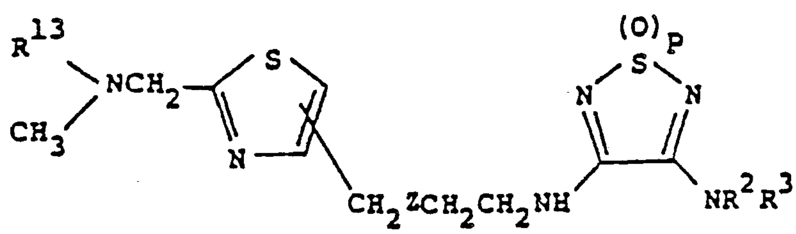

wherein p is 1 or 2; Z is sulfur or methylene; $R^2$ and $R^3$ each are independently hydrogen or $C_1$—$C_6$-alkyl, or when $R^2$ is hydrogen, $R^3$ also may be $C_2$—$C_6$-alkenyl, $C_2$—$C_6$-alkynyl or

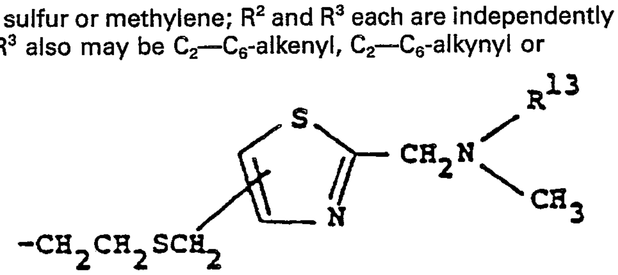 ;

and $R^{13}$ is hydrogen or methyl; or a nontoxic pharmaceutically acceptable salt, hydrate or solvate thereof.

8. A composition of Claim 1 wherein the compound of Formula I has the structure

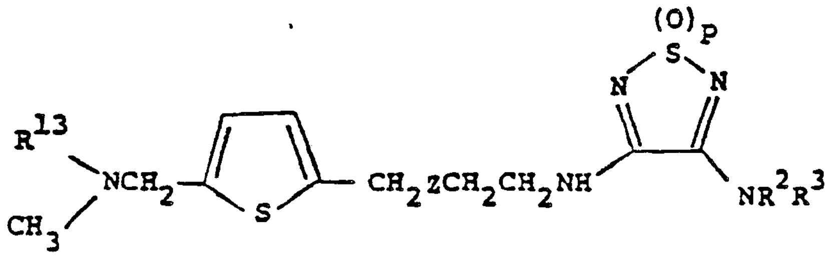

wherein p is 1 or 2; Z is sulfur or methylene; $R^2$ and $R^3$ each are independently hydrogen or $C_1$—$C_6$-alkyl, or, when $R^2$ is hydrogen, $R^3$ also may be $C_2$—$C_6$-alkenyl, $C_2$—$C_6$-alkynyl, phenyl-$C_1$—$C_6$-alkyl, pyridylmethyl, 3,4-methylenedioxybenzyl or

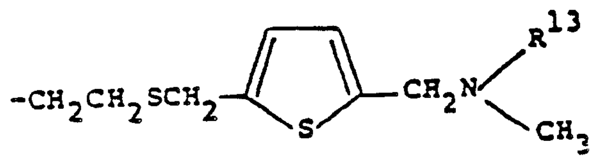 ;

and $R^{13}$ is hydrogen or methyl; or a nontoxic pharmaceutically acceptable salt, hydrate or solvate thereof.

9. A composition of Claim 1 wherein the compound of Formula I has the structure

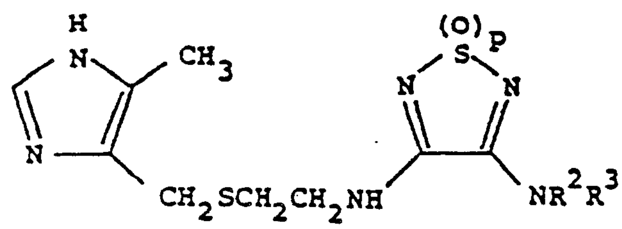

wherein p is 1 or 2; and $R^2$ and $R^3$ each are independently hydrogen or $C_1$—$C_6$-alkyl, or, when $R^2$ is hydrogen, $R^3$ also may be $C_2$—$C_6$-alkenyl, $C_2$—$C_6$-alkynyl or

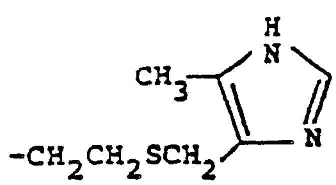 ;

or a nontoxic, pharmaceutically acceptable salt, hydrate or solvate thereof.

10. A composition of Claim 1 wherein the compound of Formula I has the structure

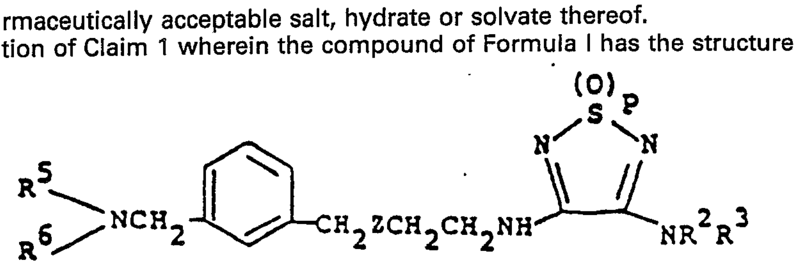

wherein p is 1 or 2; Z is sulfur or methylene; $R^2$ and $R^3$ each are independently hydrogen or $C_1$—$C_6$-alkyl, or, when $R^2$ is hydrogen, $R^3$ also may be $C_2$—$C_6$-alkenyl, $C_2$—$C_6$-alkynyl or

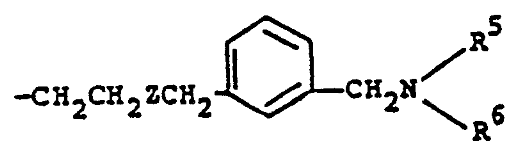 ;

and $R^5$ and $R^6$ each are independently hydrogen or $C_1$—$C_6$-alkyl, or, $R^5$ and $R^6$, taken together with the nitrogen atom to which they are attached, may be piperidino; or a nontoxic, pharmaceutically acceptable salt, hydrate or solvate thereof.

11. A composition of Claim 1 wherein the compound of Formula I has the structure

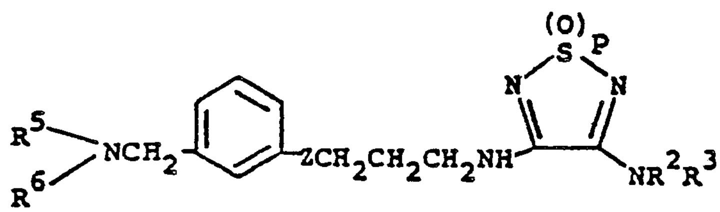

wherein p is 1 or 2; Z is oxygen or sulfur; $R^2$ and $R^3$ each are independently hydrogen or $C_1$—$C_6$-alkyl, or, when $R^2$ is hydrogen, $R^3$ also may be $C_2$—$C_6$-alkenyl, $C_2$—$C_6$-alkynyl, pyridylmethyl or

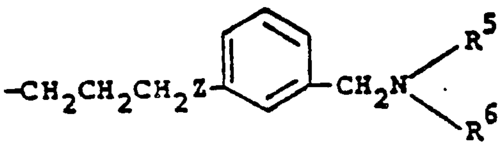 ;

and $R^5$ and $R^6$ each are independently hydrogen or $C_1$—$C_6$-alkyl, or, when $R^5$ is hydrogen, $R^6$ also may be $C_2$—$C_6$-alkenyl or $C_2$—$C_6$-alkynyl; or $R^5$ and $R^6$, taken together with the nitrogen to which they are attached, may be pyrrolidino, methylpyrrolidino, morpholino, thiomorpholino, piperidino, methylpiperidino, dimethylpiperidino, homopiperidino or heptamethyleneimino; or a nontoxic, pharmaceutically acceptable salt, hydrate or solvate thereof.

12. A composition of claim 1 wherein the compound of formula I has the structure

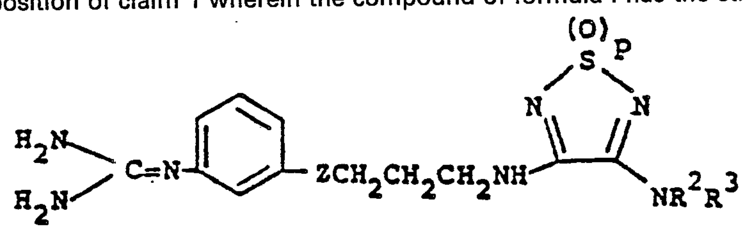

wherein p is 1 or 2; Z is oxygen or sulfur; $R^2$ and $R^3$ each are independently hydrogen or $C_1$—$C_6$-alkyl, or, when $R^2$ is hydrogen, $R^3$ may be $C_2$—$C_6$-alkenyl, $C_2$—$C_6$-alkynyl, pyridylmethyl or

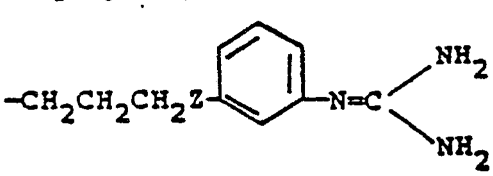

or a nontoxic pharmaceutically acceptable salt, hydrate or solvate thereof.

13. A composition of any one of Claims 1—12 wherein the dosage of pepstatin is from 100 to 175 mg and the dosage of the compound of Formula I is from 2 to 100 mg.

14. A composition of Claim 13 wherein the dosage of the compound of Formula I is from about 4 to 50 mg.

15. A pharmaceutical composition of claim 13 which comprises from 100 to 175 mg of pepstatin and from 2 to 15 mg of 3-amino-4-{2-[(5-dimethylaminomethyl-2-furyl)-methylthio]-ethylamino}-1,2,5-thiadiazole 1-oxid or a non-toxic pharmaceutically acceptable acid addition salt, hydrate or solvate thereof.

16. A pharmaceutical composition of claim 13 which comprises from 100 to 175 mg of pepstatin and from 2 to 15 mg of 3-amino-4-{2-[(2-guanidinothiazol-4-yl)methylthio]ethylamino}-1,2,5-thiadiazole 1-oxide or a nontoxic pharmaceutically acceptable acid addition salt, hydrate or solvate thereof.

17. A composition of Claims 13 to 16 wherein the pepstatin is in the form of pepstatin floating minicapsules.

18. A composition of any one of claims 1—17 in unit dosage form.

19. A composition of any one of the preceding claims comprising two parts, the first part comprising at least one compound of formula I as defined in claims 1 to 12, 15 and 16 and the second part comprising pepstatin.

20. Use of at least one compound of the formula I as defined in claims 1 to 12, 15 and 16 in combination with pepstatin for preparing a pharmaceutical composition for the treatment of peptic ulcers.

**Claims for the Contracting State: AT**

1. A pharmaceutical composition useful in the treatment of peptic ulcers, which comprises a peptic activity-inhibiting amount of pepstatin and an effective anti-ulcerogenic amount of at least one compound of the formula I:

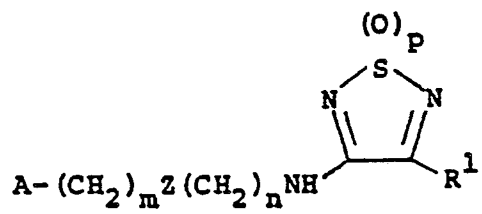

I

wherein

p is 1 or 2;

$R^1$ is hydroxy or $NR^2R^3$;

$R^2$ and $R^3$ are each independently hydrogen, $C_1$—$C_6$-alkyl, $C_2$—$C_6$-alkenyl, $C_2$—$C_6$-alkynyl, cyclo-$C_3$—$C_7$-alkyl-$C_1$—$C_6$-alkyl, hydroxy-$C_1$—$C_6$-alkyl, $C_1$—$C_6$-alkoxy-$C_1$—$C_6$-alkyl, $C_1$—$C_6$-alkylthio-$C_1$—$C_6$-alkyl, 2-fluoroethyl, 2,2,2-trifluoroethyl or cyano-$C_1$—$C_6$-alkyl, or, when $R^2$ is hydrogen, $R^3$ may also be cyclo-$C_3$—$C_7$-alkyl, amino-$C_1$—$C_6$-alkyl, $C_1$—$C_6$-alkylamino-$C_1$—$C_6$-alkyl, di-$C_1$—$C_6$-alkylamino-$C_1$—$C_6$-alkyl, pyrrolidino-$C_1$—$C_6$-alkyl, piperidino-$C_1$—$C_6$-alkyl, morpholino-$C_1$—$C_6$-alkyl, piperazino-$C_1$—$C_6$-alkyl, pyridyl-$C_1$—$C_6$-alkyl, substituted pyridyl-$C_1$—$C_6$-alkyl wherein the pyridyl ring may contain one substituent selected from $C_1$—$C_6$-alkyl, $C_1$—$C_6$-alkoxy, hydroxy, amino and halogen, amino, $C_1$—$C_6$-alkylamino, di-$C_1$—$C_6$-alkylamino, hydroxy, $C_1$—$C_6$-alkoxy, 2,3-dihydroxypropyl, cyano, amidino, $C_1$—$C_6$-alkylamidino, A'—$(CH_2)_{m'}Z'(CH_2)_{n'}$—, phenyl, phenyl-$C_1$—$C_6$-alkyl, substituted phenyl or substituted phenyl-$C_1$—$C_6$-alkyl, wherein the phenyl ring may contain one or two substituents independently selected from $C_1$—$C_6$-alkyl, hydroxy, $C_1$—$C_6$-alkoxy and halogen or one substituent selected from methylenedioxy, trifluoromethyl and di-$C_1$—$C_6$-alkylamino; or $R^2$ and $R^3$, taken together, may be —$CH_2CH_2X(CH_2)_r$—;

r is an integer of from 1 to 3, inclusive;

X is methylene, sulfur, oxygen or N—$R^4$, provided that, when r is 1, X is methylene;

$R^4$ is hydrogen, $C_1$—$C_6$-alkyl, $C_2$—$C_6$-alkenyl, $C_2$—$C_6$-alkynyl, $C_1$—$C_6$-alkanoyl or benzoyl;

m and m' each are independently an integer of from zero to 2, inclusive;

n and n' each are independently an integer of from 2 to 4, inclusive;

Z and Z' each are independently sulfur, oxygen or methylene;

A and A' each are independently phenyl, imidazolyl, thiazolyl, isothiazolyl, oxazolyl, isoxazolyl, triazolyl, thiadiazolyl, oxadiazolyl, furyl, thienyl or pyridyl; provided that A and A' independently may contain one or two substituents, the first substituent being selected from $C_1$—$C_6$-alkyl, hydroxy, trifluoromethyl, halogen, amino, hydroxymethyl, $C_1$—$C_6$-alkoxy,

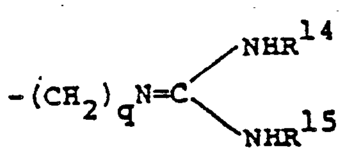

and

$-(CH_2)_qNR^5R^6$,

and the second substituent being selected from $C_1$—$C_6$-alkyl, hydroxy, trifluoromethyl, halogen, amino, hydroxymethyl and $C_1$—$C_6$-alkoxy;

q is an integer of from 0 to 6, inclusive; $R^{14}$ and $R^{15}$ independently are hydrogen or $C_1$—$C_6$-alkyl, or, if $R^{14}$ is hydrogen, $R^{15}$ also may be $C_1$—$C_6$-alkanoyl or benzoyl, or $R^{14}$ and $R^{15}$, taken together, may be ethylene; and

$R^5$ and $R^6$ each are independently hydrogen, $C_1$—$C_6$-alkyl, $C_2$—$C_6$-alkenyl, $C_2$—$C_6$-alkynyl, $C_1$—$C_6$-alkoxy-$C_1$—$C_6$-alkyl, cyclo-$C_3$—$C_7$-alkyl, phenyl or phenyl-$C_1$—$C_6$-alkyl, provided that $R^5$ and $R^6$ may not both be cyclo-$C_3$—$C_7$-alkyl or phenyl; or $R^5$ and $R^6$, taken together with the nitrogen atom to which they are attached, may be pyrrolidino, methylpyrrolidino, dimethylpyrrolidino, morpholino, thiomorpholino, piperidino, methylpiperidino, dimethylpiperidino, hydroxypiperidino, N-methylpiperazino, homopiperidino, heptamethyleneimino or octamethyleneimino, or a nontoxic, pharmaceutically acceptable salt, hydrate or solvate thereof.

2. A composition of Claim 1 wherein in Formula I $R^1$ is $NR^2R^3$ and $R^2$ and $R^3$ are as defined in claim 1.

3. A composition of Claim 1 wherein the compound of Formula I has the structure

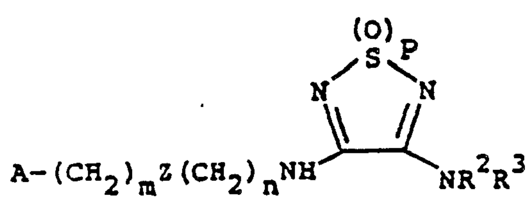

wherein p is 1 or 2;

$R^2$ and $R^3$ each are independently hydrogen, $C_1$—$C_6$-alkyl, $C_2$—$C_6$-alkenyl, $C_2$—$C_6$-alkynyl, cyclo-$C_3$—$C_7$-alkyl-$C_1$—$C_6$-alkyl, hydroxy-$C_1$—$C_6$-alkyl, $C_1$—$C_6$-alkoxy-$C_1$—$C_6$-alkyl, 2-fluoroethyl or 2,2,2-trifluoroethyl, or,

when $R^2$ is hydrogen, $R^3$ also may be pyrrolidino-$C_1$—$C_6$-alkyl, piperidino-$C_1$—$C_6$-alkyl, morpholino-$C_1$—$C_6$-alkyl, piperazino-$C_1$—$C_6$-alkyl, pyridyl-$C_1$—$C_6$-alkyl, substituted pyridyl-$C_1$—$C_6$-alkyl wherein the pyridyl ring may contain one substituent selected from $C_1$—$C_6$-alkyl, $C_1$—$C_6$-alkoxy, hydroxy, amino and halogen, hydroxy, A'—$(CH_2)_{m'}$Z'$(CH_2)_{n'}$—, phenyl-$C_1$—$C_6$-alkyl or substituted phenyl-$C_1$—$C_6$-alkyl, wherein the phenyl ring may contain one or two substituents independently selected from $C_1$—$C_6$-alkyl, hydroxy, $C_1$—$C_6$-alkoxy and halogen or one substituent selected from methylenedioxy, trifluoromethyl and di-$C_1$—$C_6$-alkylamino;

m and m' each are independently an integer of from zero to 2, inclusive;

n and n' each are independently an integer of from 2 to 4, inclusive;

Z and Z' each are independently sulfur, oxygen or methylene;

A and A' each are independently phenyl, imidazolyl, thiazolyl, oxazolyl, thiadiazolyl, oxadiazolyl, furyl, thienyl or pyridyl; provided that A and A' independently may contain one or two substituents, the first substituent being selected from $C_1$—$C_6$-alkyl, hydroxy, trifluoromethyl, halogen, amino, hydroxymethyl, $C_1$—$C_6$-alkoxy,

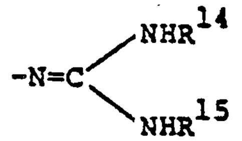 and $-CH_2NR^5R^6$,

and the second substituent being selected from $C_1$—$C_6$-alkyl, hydroxy, trifluoromethyl, halogen, amino, hydroxymethyl and $C_1$—$C_6$-alkoxy;

q is an integer of from 0 to 6, inclusive; $R^{14}$ and $R^{15}$ independently are hydrogen or $C_1$—$C_6$-alkyl, or $R^{14}$ and $R^{15}$, taken together, may be ethylene; and

$R^5$ and $R^6$ each are independently hydrogen, $C_1$—$C_6$-alkyl, $C_2$—$C_6$-alkenyl or $C_2$—$C_6$-alkynyl; or $R^5$ and $R^6$, taken together with the nitrogen atom to which they are attached, may be pyrrolidino, methylpyrrolidino, dimethylpyrrolidino, morpholino, thiomorpholino, piperidino, methylpiperidino, dimethylpiperidino, hydroxypiperidino, N-methylpiperazino, homopiperidino, heptamethyleneimino or octamethyleneimino, or a nontoxic pharmaceutically acceptable salt, hydrate or solvate thereof.

4. A composition of Claim 1 wherein the compound of Formula I has the structure

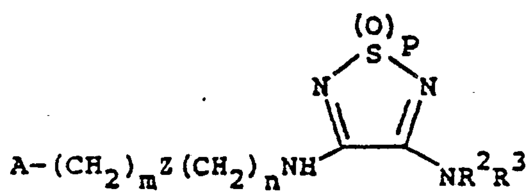

wherein p is 1 or 2;

$R^2$ and $R^3$ each are independently hydrogen, $C_1$—$C_6$-alkyl, $C_2$—$C_6$-alkenyl, $C_2$—$C_6$-alkynyl or cyclo-$C_3$—$C_7$-alkyl-$C_1$—$C_6$-alkyl, or, when $R^2$ is hydrogen, $R^3$ also may be pyridyl-$C_1$—$C_6$-alkyl, substituted pyridyl-$C_1$—$C_6$-alkyl wherein the pyridyl ring may contain one substituent selected from $C_1$—$C_6$-alkyl, $C_1$—$C_6$-alkoxy, hydroxy, amino and halogen, A'—$(CH_2)_{m'}$Z'$(CH_2)_{n'}$—, phenyl-$C_1$—$C_6$-alkyl or 3,4-methylenedioxybenzyl;

m and m' each are independently zero or 1;

n and n' each are independently 2 or 3;

Z and Z' each are independently sulfur, oxygen or methylene;

A and A' each are independently phenyl, imidazolyl, thiazolyl, furyl, thienyl or pyridyl; provided that A and A' independently may contain one or two substituents, the first substituent being selected from $C_1$—$C_6$-alkyl,

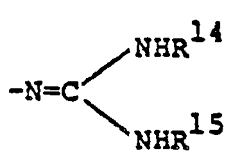 and $-CH_2NR^5R^6$,

and the second substituent being selected from $C_1$—$C_6$-alkyl;

$R^{14}$ and $R^{15}$ independently are hydrogen or $C_1$—$C_6$-alkyl, or $R^{14}$ and $R^{15}$, taken together, may be ethylene; and

$R^5$ and $R^6$ each are independently hydrogen or $C_1$—$C_6$-alkyl; or $R^5$ and $R^6$, taken together with the nitrogen atom to which they are attached, may be pyrrolidino, methylpyrrolidino, dimethylpyrrolidino, morpholino, thiomorpholino, piperidino, methylpiperidino, dimethylpiperidino, hydroxypiperidino, N-methylpiperazino, homopiperidino, heptamethyleneimino or octamethyleneimino, or a nontoxic, pharmaceutically acceptable salt, hydrate or solvate thereof.

5. A composition of Claim 1 wherein the compound of Formula I has the structure

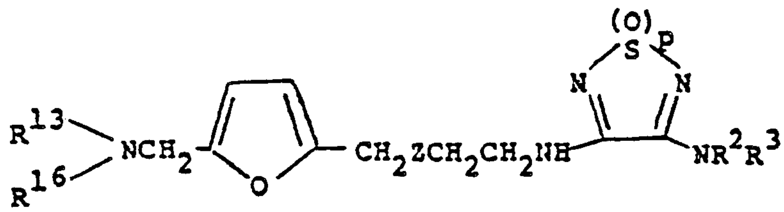

wherein p is 1 or 2; Z is sulfur or methylene; $R^2$ and $R^3$ each are independently hydrogen or $C_1$—$C_6$-alkyl, or, when $R^2$ is hydrogen, $R^3$ also may be $C_2$—$C_6$-alkenyl, $C_2$—$C_6$-alkynyl, phenyl-$C_1$—$C_6$-alkyl, cyclo-$C_3$—$C_7$-alkyl-$C_1$—$C_6$-alkyl, pyridylmethyl or

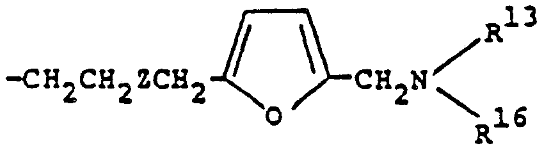 ;

$R^{16}$ is methyl and $R^{13}$ is hydrogen or methyl, or $R^{16}$ and $R^{13}$, taken together with the nitrogen atom to which they are attached, may be piperidino; or a nontoxic pharmaceutically acceptable salt, hydrate or solvate thereof.

6. A composition of Claim 1 wherein the compound of Formula I has the structure

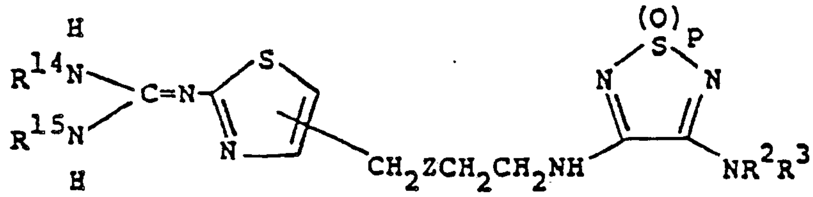

wherein p is 1 or 2; Z is sulfur or methylene; $R^{14}$ and $R^{15}$ independently are hydrogen or methyl, or, $R^{14}$ and $R^{15}$, taken together, may be ethylene; and $R^2$ and $R^3$ each are independently hydrogen or $C_1$—$C_6$-alkyl, or, when $R^2$ is hydrogen, $R^3$ also may be $C_2$—$C_6$-alkenyl, $C_2$—$C_6$-alkynyl, pyridylmethyl,

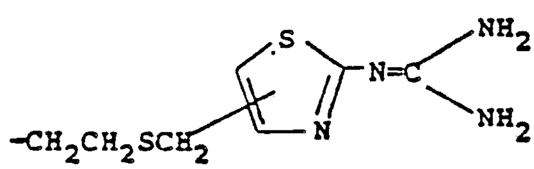 or 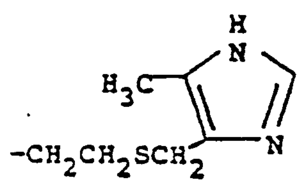 ;

or a nontoxic, pharmaceutically acceptable salt, hydrate or solvate thereof.

7. A composition of Claim 1 wherein the compound of Formula I has the structure

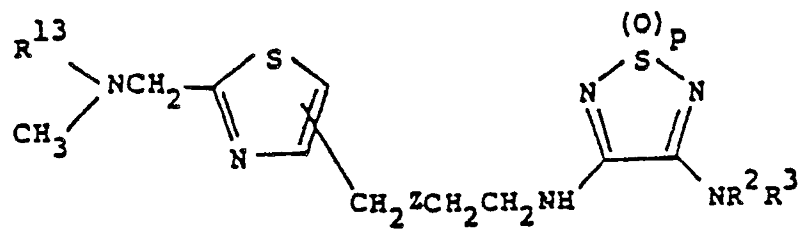

wherein p is 1 or 2; Z is sulfur or methylene; $R^2$ and $R^3$ each are independently hydrogen or $C_1$—$C_6$-alkyl, or when $R^2$ is hydrogen, $R^3$ also may be $C_2$—$C_6$-alkenyl, $C_2$—$C_6$-alkynyl or

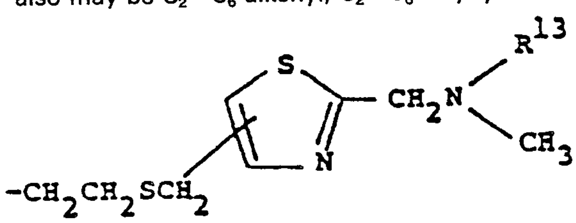 ;

and $R^{13}$ is hydrogen or methyl; or a nontoxic pharmaceutically acceptable salt, hydrate or solvate thereof.

8. A composition of Claim 1 wherein the compound of Formula I has the structure

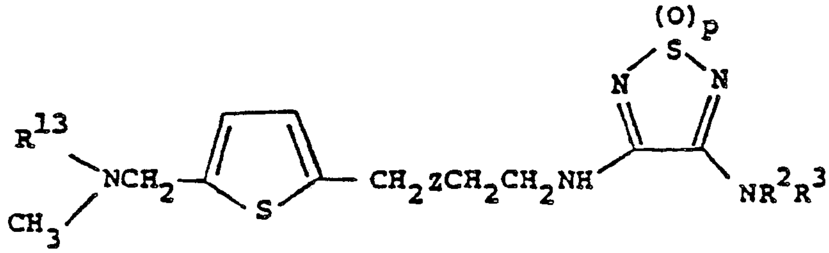

wherein p is 1 or 2; Z is sulfur or methylene; $R^2$ and $R^3$ each are independently hydrogen or $C_1$—$C_6$-alkyl, or, when $R^2$ is hydrogen, $R^3$ also may be $C_2$—$C_6$-alkenyl, $C_2$—$C_6$-alkynyl, phenyl-$C_1$—$C_6$-alkyl, pyridylmethyl, 3,4-methylenedioxybenzyl or

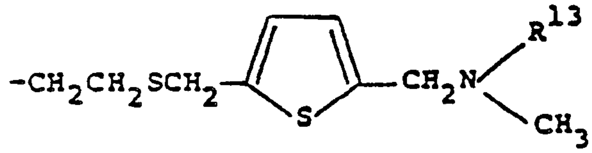 ;

and $R^{13}$ is hydrogen or methyl; or a nontoxic pharmaceutically acceptable salt, hydrate or solvate thereof.

9. A composition of Claim 1 wherein the compound of Formula I has the structure

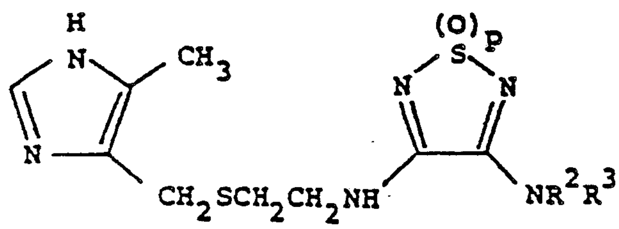

wherein p is 1 or 2; and $R^2$ and $R^3$ each are independently hydrogen or $C_1$—$C_6$-alkyl, or, when $R^2$ is hydrogen, $R^3$ also may be $C_2$—$C_6$-alkenyl, $C_2$—$C_6$-alkynyl or

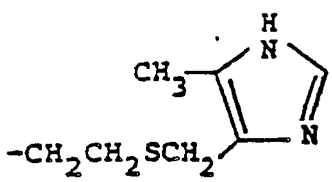 ;

or a nontoxic, pharmaceutically acceptable salt, hydrate or solvate thereof.

10. A composition of Claim 1 wherein the compound of Formula I has the structure

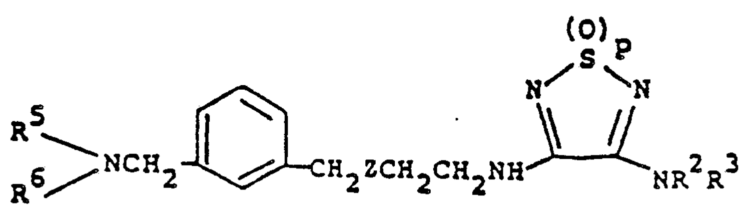

wherein p is 1 or 2; Z is sulfur or methylene; $R^2$ and $R^3$ each are independently hydrogen or $C_1$—$C_6$-alkyl, or, when $R^2$ is hydrogen, $R^3$ also may be $C_2$—$C_6$-alkenyl, $C_2$—$C_6$-alkynyl or

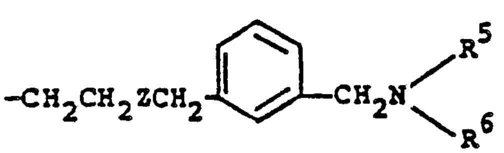 ;

and $R^5$ and $R^6$ each are independently hydrogen or $C_1$—$C_6$-alkyl, or, $R^5$ and $R^6$, taken together with the nitrogen atom to which they are attached, may be piperidino; or a nontoxic, pharmaceutically acceptable salt, hydrate or solvate thereof.

11. A composition of Claim 1 wherein the compound of Formula I has the structure

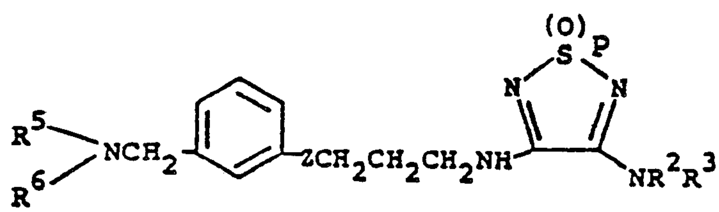

wherein p is 1 or 2; Z is oxygen or sulfur; $R^2$ and $R^3$ each are independently hydrogen or $C_1$—$C_6$-alkyl, or, when $R^2$ is hydrogen, $R^3$ also may be $C_2$—$C_6$-alkenyl, $C_2$—$C_6$-alkynyl, pyridylmethyl or

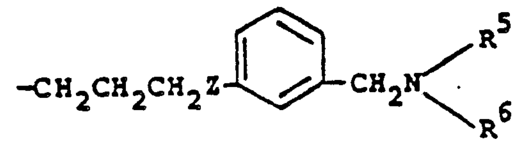

;

and $R^5$ and $R^6$ each are independently hydrogen or $C_1$—$C_6$-alkyl, or, when $R^5$ is hydrogen, $R^6$ also may be $C_2$—$C_6$-alkenyl or $C_2$—$C_6$-alkynyl; or $R^5$ and $R^6$, taken together with the nitrogen to which they are attached, may be pyrrolidino, methylpyrrolidino, morpholino, thiomorpholino, piperidino, methylpiperidino, dimethylpiperidino, homopiperidino or heptamethyleneimino; or a nontoxic, pharmaceutically acceptable salt, hydrate or solvate thereof.

12. A composition of claim 1 wherein the compound of formula I has the structure

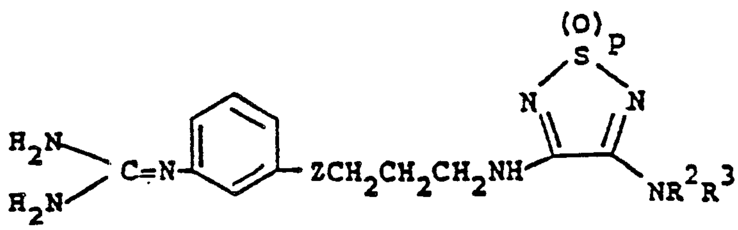

wherein p is 1 or 2; Z is oxygen or sulfur; $R^2$ and $R^3$ each are independently hydrogen or $C_1$—$C_6$-alkyl, or, when $R^2$ is hydrogen, $R^3$ may be $C_2$—$C_6$-alkenyl, $C_2$—$C_6$-alkynyl, pyridylmethyl or

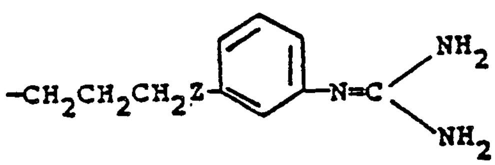

or a nontoxic pharmaceutically acceptable salt, hydrate or solvate thereof.

13. A composition of any one of Claims 1—12 wherein the dosage of pepstatin is from 100 to 175 mg and the dosage of the compound of Formula I is from 2 to 100 mg.

14. A composition of Claim 13 wherein the dosage of the compound of Formula I is from about 4 to 50 mg.

15. A pharmaceutical composition of claim 13 which comprises from 100 to 175 mg of pepstatin and from 2 to 15 mg of 3-amino-4-{2-[(5-dimethylaminomethyl-2-furyl)-methylthio]-ethylamino}-1,2,5-thiadiazole 1-oxid or a non-toxic pharmaceutically acceptable acid addition salt, hydrate or solvate thereof.

16. A pharmaceutical composition of claim 13 which comprises from 100 to 175 mg of pepstatin and from 2 to 15 mg of 3-amino-4-{2-[(2-guanidinothiazol-4-yl)methylthio]ethylamino}-1,2,5-thiadiazole 1-oxide or a nontoxic pharmaceutically acceptable acid addition salt, hydrate or solvate thereof.

17. A composition of Claims 13 to 16 wherein the pepstatin is in the form of pepstatin floating minicapsules.

18. A composition of any one of claims 1—17 in unit dosage form.

19. A composition of any one of the preceding claims comprising two parts, the first part comprising at least one compound of formula I as defined in claims 1 to 12, 15 and 16 and the second part comprising pepstatin.

20. Use of at least one compound of the formula I as defined in claims 1 to 12, 15 and 16 in combination with pepstatin for preparing a pharmaceutical composition for the treatment of peptic ulcers.

21. A process for preparing a composition of any one of the claims 1 to 19 which comprises providing an effective anti-ulcerogenic amount of at least one compound of the formula I in combination with a peptic activity inhibiting amount of pepstatin.

**Patentansprüche für die Vertragsstaaten: BE CH DE FR GB IT LI LU NL SE**

1. Pharmazeutisches Mittel zur Behandlung peptischer Ulcera, das eine die peptische Aktivität inhibierende Menge an Pepstatin und eine wirksame antiulcerogene Menge mindestens einer Verbindung der Formel I:

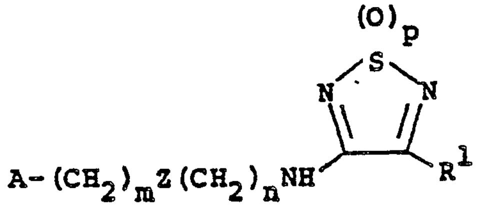

I

umfaßt, wobei in obiger Formel

p für 1 oder 2 steht;

$R^1$ für Hydroxy oder $NR^2R^3$ steht, wobei

$R^2$ und $R^3$ unabhängig voneinander für Wasserstoff, $C_1$—$C_6$-Alkyl, $C_2$—$C_6$-Alkenyl, $C_2$—$C_6$-Alkinyl, Cyclo-$C_3$—$C_7$-alkyl-$C_1$—$C_6$-alkyl, Hydroxy-$C_1$—$C_6$-alkyl, $C_1$—$C_6$-Alkoxy-$C_1$—$C_6$-alkyl, $C_1$—$C_6$-alkylthio-$C_1$—$C_6$-alkyl, 2-Fluorethyl, 2,2,2-Trifluorethyl oder Cyano-$C_1$—$C_6$-alkyl stehen, oder, wenn $R^2$ für Wasserstoff steht, $R^3$ auch für Cyclo-$C_3$—$C_7$-alkyl, Amino-$C_1$—$C_6$-alkyl, $C_1$—$C_6$-alkylamino-$C_1$—$C_6$-alkyl, Di-$C_1$—$C_6$-alkylamino-$C_1$—$C_6$-alkyl, Pyrrolidino-$C_1$—$C_6$-alkyl, Piperidino-$C_1$—$C_6$-alkyl, Morpholino-$C_1$—$C_6$-alkyl, Piperazino-$C_1$—$C_6$-alkyl, Pyridyl-$C_1$—$C_6$-alkyl, substituiertes Pyridyl-$C_1$—$C_6$-alkyl, wobei der Pyridylring einen Substituenten haben kann, der unter $C_1$—$C_6$-Alkyl, $C_1$—$C_6$-Alkoxy, Hydroxy, Amino und Halogen ausgewählt ist, Amino, $C_1$—$C_6$-Alkylamino, Di-$C_1$—$C_6$-alkylamino, Hydroxy, $C_1$—$C_6$-Alkoxy, 2,3-Dihydroxypropyl, Cyano, Amidino, $C_1$—$C_6$-Alkylamidino, $A'$—$(CH_2)_{m'}Z'(CH_2)_{n'}$—, Phenyl, Phenyl-$C_1$—$C_6$-alkyl, substituiertes Phenyl oder substituiertes Phenyl-$C_1$—$C_6$-alkyl stehen kann, wobei der Phenylring einen oder zwei Substituenten aufweisen kann, die unabhängig voneinander ausgewählt sind unter $C_1$—$C_6$-Alkyl, Hydroxy, $C_1$—$C_6$-Alkoxy und Halogen, oder einen Substituenten aufweisen kann, der ausgewählt ist unter Methylendioxy, Trifluormethyl und Di-$C_1$—$C_6$-alkylamino, oder $R^2$ und $R^3$ zusammen für —$CH_2CH_2X(CH_2)_r$— stehen können, wobei

r für eine ganze Zahl von 1 bis einschließlich 3 steht;

X für Methylen, Schwefel, Sauerstoff oder N—$R^4$ unter der Bedingung steht, daß, wenn r für 1 steht, X für Methylen steht, wobei

$R^4$ für Wasserstoff, $C_1$—$C_6$-Alkyl, $C_2$—$C_6$-Alkenyl, $C_2$—$C_6$-Alkinyl, $C_1$—$C_6$-Alkanoyl oder Benzoyl steht;

m und m' unabhängig voneinander für eine ganze Zahl von 0 bis einschließlich 2 stehen;

n und n' unabhängig voneinander für eine ganze Zahl von 2 bis einschließlich 4 stehen;

Z und Z' unabhängig voneinander für Schwefel, Sauerstoff oder Methylen stehen;

A und A' unabhängig voneinander für Phenyl, Imidazolyl, Thiazolyl, Isothiazolyl, Oxazolyl, Isoxazolyl, Triazolyl, Thiadiazolyl, Oxadiazolyl, Furyl, Thienyl oder Pyridyl stehen, wobei A und A' unabhängig voneinander einen oder zwei Substituenten aufweisen können, wobei der erste Substituent ausgewählt ist unter $C_1$—$C_6$-Alkyl, Hydroxy, Trifluormethyl, Halogen, Amino, Hydroxymethyl, $C_1$—$C_6$-Alkoxy,

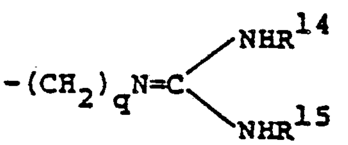

und

$(CH_2)_qNR^5R^6$,

und der zweite Substituent ausgewählt ist unter $C_1$—$C_6$-Alkyl, Hydroxy, Trifluoromethyl, Halogen, Amino, Hydroxymethyl und $C_1$—$C_6$-Alkoxy;

q für ein ganze Zahl von 0 bis einschließlich 6 steht;

$R^{14}$ und $R^{15}$ unabhängig voneinander für Wasserstoff oder $C_1$—$C_6$-Alkyl stehen, oder, wenn $R^{14}$ für Wasserstoff steht, $R^{15}$ auch für $C_1$—$C_6$-Alkanoyl oder Benzoyl stehen kann, oder, $R^{14}$ und $R^{15}$ zusammen für Ethylen stehen können; und

$R^5$ und $R^6$ unabhängig voneinander für Wasserstoff, $C_1$—$C_6$-Alkyl, $C_2$—$C_6$-Alkenyl, $C_2$—$C_6$-Alkinyl, $C_1$—$C_6$-Alkoxy-$C_1$—$C_6$-alkyl, Cyclo-$C_3$—$C_7$-alkyl, Phenyl oder Phenyl-$C_1$—$C_6$-alkyl unter der Bedingung stehen, daß $R^5$ und $R^6$ nicht beide für Cyclo-$C_3$—$C_7$-alkyl oder Phenyl stehen; oder $R^5$ und $R^6$ zusammen mit dem Stickstoffatom, an das sie gebunden sind, für Pyrrolidino, Methylpyrrolidino, Dimethylpyrrolidino, Morpholino, Thiomorpholino, Piperidino, Methylpiperidino, Dimethylpiperidino, Hydroxypiperidino, N-Methylpiperazino, Homopiperidino, Heptamethylenimino oder Octamethylenimino stehen, oder ein nicht-toxisches, pharmazeutisch verträgliches Salz, Hydrat oder Solvat davon.

2. Mittel nach Anspruch 1, wobei in Formel I $R^1$ für $NR^2R^3$ steht und $R^2$ und $R^3$ wie in Anspruch 1 definiert sind.

3. Mittel nach Anspruch 1, worin die Verbindung der Formel I die Struktur

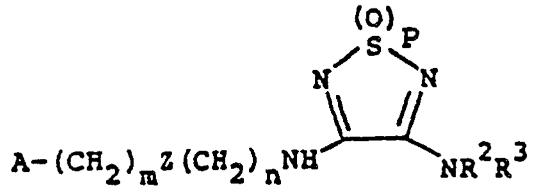

hat, worin

p für 1 oder 2 steht;

$R^2$ und $R^3$ unabhängig voneinander für Wasserstoff, $C_1$—$C_6$-Alkyl, $C_2$—$C_6$-Alkenyl, $C_2$—$C_6$-Alkinyl, Cyclo-$C_3$—$C_7$-alkyl-$C_1$—$C_6$-alkyl, Hydroxy-$C_1$—$C_6$-alkyl, $C_1$—$C_6$-alkoxy-$C_1$—$C_6$-alkyl, 2-Fluoroethyl oder 2,2,2-Trifluorethyl stehen, oder, wenn $R^2$ für Wasserstoff steht, $R^3$ auch für Pyrrolidino-$C_1$—$C_6$-alkyl, Piperidino-$C_1$—$C_6$-alkyl, Morpholino-$C_1$—$C_6$-alkyl, Piperazino-$C_1$—$C_6$-alkyl, Pyridyl-$C_1$—$C_6$-alkyl, substituiertes Pyridyl-$C_1$—$C_6$-alkyl, worin der Pyridylring einen Substituenten aufweisen kann, der unter $C_1$—$C_6$-Alkyl, $C_1$—$C_6$-Alkoxy, Hydroxy, Amino und Halogen ausgewählt ist, Hydroxy, $A'$—$(CH_2)_{m'}Z'(CH_2)_{n'}$—, Phenyl-$C_1$—$C_6$-alkyl oder substituiertes Phenyl-$C_1$—$C_6$-alkyl stehen kann, wobei der Phenylring einen oder zwei Substituenten haben kann, die unabhängig voneinander ausgewählt sind unter $C_1$—$C_6$-Alkyl, Hydroxy, $C_1$—$C_6$-Alkoxy und Halogen, oder einen Substituenten aufweisen kann, der ausgewählt ist unter Methylendioxy, Trifluormethyl und Di-$C_1$—$C_6$-alkylamino;

m und m' unabhängig voneinander für eine ganze Zahl von 0 bis einschließlich 2 stehen;

n und n' unabhängig voneinander für eine ganze Zahl von 2 bis einschließlich 4 stehen;

Z und Z' unabhängig voneinander für Schwefel, Sauerstoff oder Methylen stehen;

A und A' unabhängig voneinander für Phenyl, Imidazolyl, Thiazolyl, Oxazolyl, Thiadiazolyl, Oxadiazolyl, Furyl, Thienyl oder Pyridyl stehen, wobei A und A' unabhängig voneinander einen oder zwei Substituenten aufweisen können, wobei der erste Substituent ausgewählt ist unter $C_1$—$C_6$-Alkyl, Hydroxy, Trifluormethyl, Halogen, Amino, Hydroxymethyl, $C_1$—$C_6$-Alkoxy,

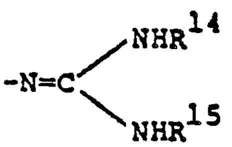 and $-CH_2NR^5R^6$,

und der zweite Substituent ausgewählt ist unter $C_1$—$C_6$-Alkyl, Hydroxy, Trifluoromethyl, Halogen, Amino, Hydroxymethyl und $C_1$—$C_6$-Alkoxy;

q für eine ganze Zahl von 0 bis einschließlich 6 steht;

$R^{14}$ und $R^{15}$ unabhängig voneinander für Wasserstoff oder $C_1$—$C_6$-Alkyl stehen, oder $R^{14}$ und $R^{15}$ zusammen für Ethylen stehen können; und

$R^5$ und $R^6$ unabhängig voneinander für Wasserstoff, $C_1$—$C_6$-Alkyl, $C_2$—$C_6$-Alkenyl oder $C_2$—$C_6$-Alkinyl stehen, oder $R^5$ und $R^6$ zusammen mit dem Stickstoffatom, an das sie gebunden sind, für Pyrrolidino, Methylpyrrolidino, Dimethylpyrrolidino, Morpholino, Thiomorpholino, Piperidino, Methylpiperidino, Dimethylpiperidino, Hydroxypiperidino, N-Methylpiperazino, Homopiperidino, Heptamethylenimino oder Octamethylenimino stehen können,

oder ein nicht-toxisches, pharmazeutisch verträgliches Salz, Hydrat oder Solvat davon.

4. Mittel nach Anspruch 1, worin die Verbindung der Formel I die Struktur

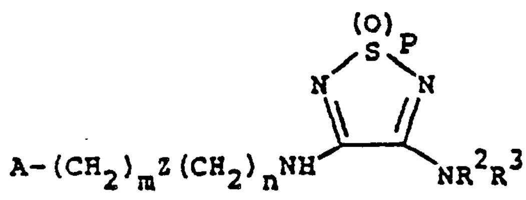

hat, worin

p für 1 oder 2 steht;

$R^2$ und $R^3$ unabhängig voneinander für Wasserstoff, $C_1$—$C_6$-Alkyl, $C_2$—$C_6$-Alkenyl, $C_2$—$C_6$-Alkinyl oder Cyclo-$C_3$—$C_7$-alkyl-$C_1$—$C_6$-alkyl stehen, oder, wenn $R^2$ für Wasserstoff steht, $R^3$ auch für Pyridyl-$C_1$—$C_6$-alkyl, substituiertes Pyridyl-$C_1$—$C_6$-alkyl, worin der Pyridylring einen Substituenten aufweisen kann, der unter $C_1$—$C_6$-Alkyl, $C_1$—$C_6$-Alkoxy, Hydroxy, Amino und Halogen ausgewählt ist, $A'$—$(CH_2)_{m'}Z'(CH_2)_{n'}$—, Phenyl-$C_1$—$C_6$-alkyl oder 3,4-Methylendioxybenzyl stehen kann;

m und m' unabhängig voneinander für 0 oder 1 stehen;

n und n' unabhängig voneinander für 2 oder 3 stehen;

Z und Z' unabhängig voneinander für Schwefel, Sauerstoff oder Methylen stehen;

A und A' unabhängig voneinander für Phenyl, Imidazolyl, Thiazolyl, Furyl, Thienyl oder Pyridyl stehen, wobei A und A' unabhängig voneinander einen oder zwei Substituenten aufweisen können, wobei der erste Substituent ausgewählt ist unter $C_1$—$C_6$-Alkyl,

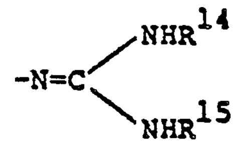

und $-CH_2NR^5R^6$,

und der zweite Substituent ausgewählt ist unter $C_1$—$C_6$-Alkyl;

$R^{14}$ und $R^{15}$ unabhängig voneinander für Wasserstoff oder $C_1$—$C_6$-Alkyl stehen, oder $R^{14}$ und $R^{15}$ zusammen für Ethylen stehen können; und

$R^5$ und $R^6$ unabhängig voneinander für Wasserstoff oder $C_1$—$C_6$-Alkyl stehen, oder $R^5$ und $R^6$ zusammen mit dem Stickstoffatom, an das sie gebunden sind, für Pyrrolidino, Methylpyrrolidino, Dimethylpyrrolidino, Morpholino, Thiomorpholino, Piperidino, Methylpiperidino, Dimethylpiperidino, Hydroxypiperidino, N-Methylpiperazino, Homopiperidino, Heptamethylenimino oder Octamethylenimino stehen können,

und ein nicht-toxisches, pharmazeutisch verträgliches Salz, Hydrat oder Solvat davon.

5. Mittel nach Anspruch 1, worin die Verbindung der Formel I die Struktur

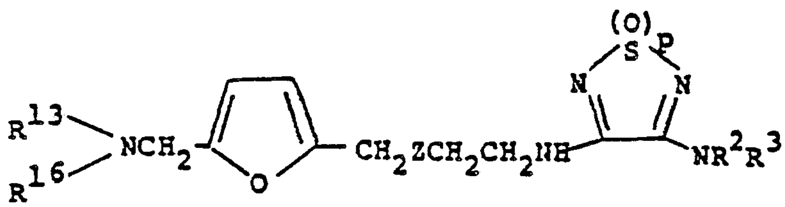

hat, worin

p für 1 oder 2 steht;

Z für Schwefel oder Methylen steht;

$R^2$ und $R^3$ unabhängig voneinander für Wasserstoff oder $C_1$—$C_6$-Alkyl stehen, oder, wenn $R^2$ für Wasserstoff steht, $R^3$ auch für $C_2$—$C_6$-Alkenyl, $C_2$—$C_6$-Alkinyl, Phenyl-$C_1$—$C_6$-alkyl, Cyclo-$C_3$—$C_7$-alkyl-$C_1$—$C_6$-alkyl, Pyridylmethyl oder

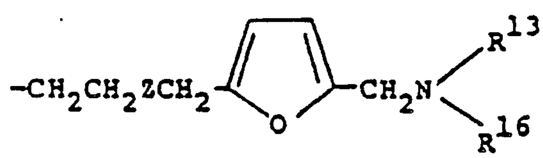

stehen kann;

$R^{16}$ für Methyl und

$R^{13}$ für Wasserstoff oder Methyl stehen, oder $R^{16}$ und $R^{13}$ zusammen mit dem Stickstoffatom, an das sie gebunden sind, für Piperidino stehen können;

oder ein nicht-toxisches, pharmazeutisch verträgliches Salz, Hydrat oder Solvat davon.

6. Mittel nach Anspruch 1, worin die Verbindung der Formel I die Struktur

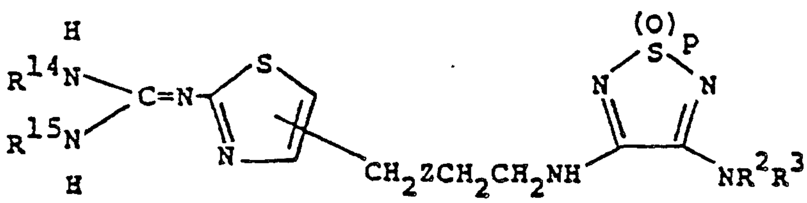

hat, worin

p für 1 oder 2 steht;

Z für Schwefel oder Methylen steht;

$R^{14}$ und $R^{15}$ unabhängig voneinander für Wasserstoff oder Methyl stehen, oder $R^{14}$ und $R^{15}$ zusammen für Ethylen stehen können; und

$R^2$ und $R^3$ unabhängig voneinander für Wasserstoff oder $C_1$—$C_6$-Alkyl stehen, oder, wenn $R^2$ für Wasserstoff steht, $R^3$ auch für $C_2$—$C_6$-Alkenyl, $C_2$—$C_6$-Alkinyl, Pyridylmethyl,

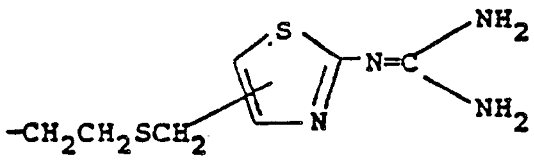

oder

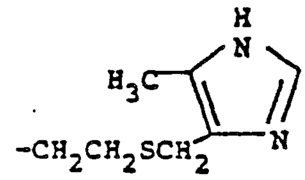

stehen kann;
oder ein nicht-toxisches, pharmazeutisch verträgliches Salz, Hydrat oder Solvat davon.

7. Mittel nach Anspruch 1, worin die Verbindung der Formel I die Struktur

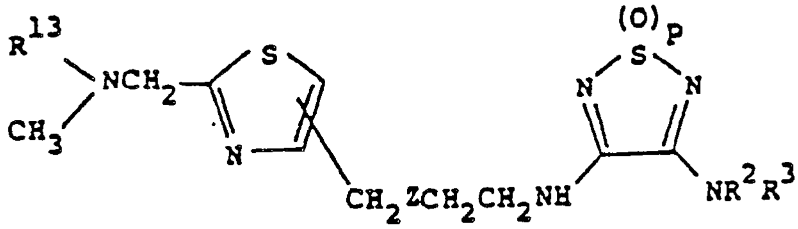

hat, worin

p für 1 oder 2 steht;

Z für Schwefel oder Methylen steht;

$R_2$ und $R_3$ unabhängig voneinander für Wasserstoff oder $C_1$—$C_6$-Alkyl stehen, oder, wenn $R^2$ für Wasserstoff steht, $R^3$ auch für $C_2$—$C_6$-Alkenyl, $C_2$—$C_6$-Alkinyl oder

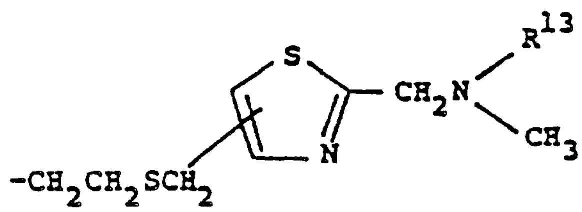

stehen kann; und

$R^{13}$ für Wasserstoff oder Methyl steht,

oder ein nicht-toxisches, pharmazeutisch verträgliches Salz, Hydrat oder Solvat davon.

8. Mittel nach Anspruch 1, worin die Verbindung der Formel I die Struktur

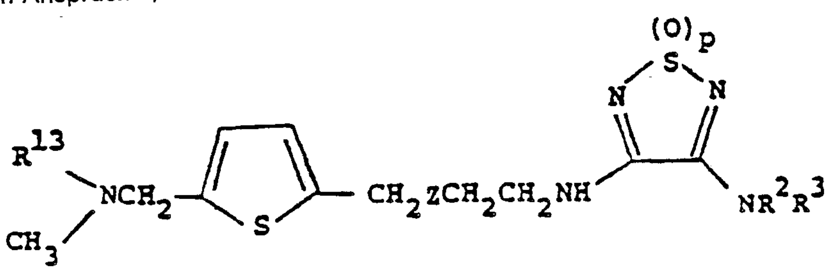

hat, worin

p für 1 oder 2 steht;

Z für Schwefel oder Methylen steht;

$R^2$ und $R^3$ jeweils unabhängig voneinander für Wasserstoff oder $C_1$—$C_6$-Alkyl stehen, oder, wenn $R^2$ für Wasserstoff steht, $R^3$ auch für $C_2$—$C_6$-Alkenyl, $C_2$—$C_6$-Alkinyl, Phenyl-$C_1$—$C_6$-alkyl, Pyridylmethyl, 3,4-Methylendioxybenzyl oder

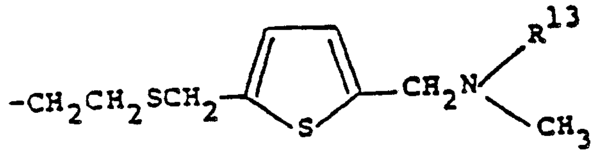

steht, und

$R^{13}$ für Wasserstoff oder Methyl steht,

oder ein nicht-toxisches, pharmazeutisch verträgliches Salz, Hydrat oder Solvat davon.

9. Mittel nach Anspruch 1, worin die Verbindung der Formel I die Struktur

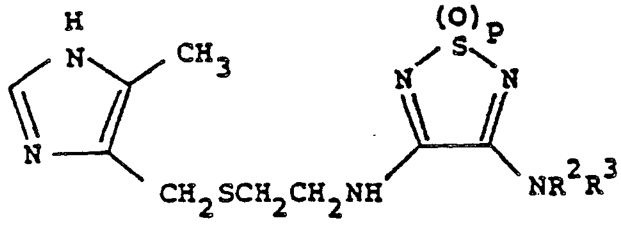

hat, worin

p für 1 oder 2 steht; und

$R^2$ und $R^3$ jeweils unabhängig voneinander für Wasserstoff oder $C_1$—$C_6$-Alkyl stehen, oder, wenn $R^2$ für Wasserstoff steht, $R^3$ auch für $C_2$—$C_6$-Alkenyl, $C_2$—$C_6$-Alkinyl oder

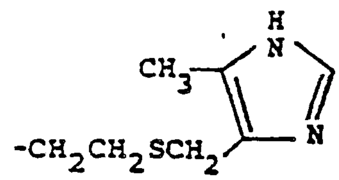

stehen kann,

oder ein nicht-toxisches, pharmazeutisch verträgliches Salz, Hydrat oder Solvat davon.

10. Mittel nach Anspruch 1, worin die Verbindung der Formel I die Struktur

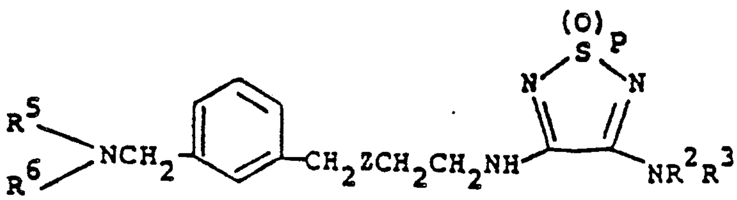

hat, worin

p für 1 oder 2 steht;

Z für Schwefel oder Methylen steht;

$R^2$ und $R^3$ jeweils unabhängig voneinander für Wasserstoff oder $C_1$—$C_6$-Alkyl stehen, oder, wenn $R^2$ für Wasserstoff steht, $R^3$ auch für $C_2$—$C_6$-Alkenyl, $C_2$—$C_6$-Alkinyl oder

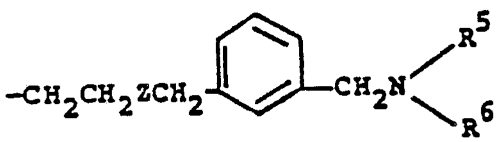

stehen kann, und

$R^5$ und $R^6$ jeweils unabhängig voneinander für Wasserstoff oder $C_1$—$C_6$-Alkyl stehen, oder $R^5$ und $R^6$ zusammen mit dem Stickstoffatom, an das sie gebun-sind, für Piperidino stehen können,

oder ein nicht-toxisches, pharmazeutisch verträgliches Salz, Hydrat oder Solvat davon.

11. Mittel nach Anspruch 1, worin die Verbindung der Formel I die Struktur

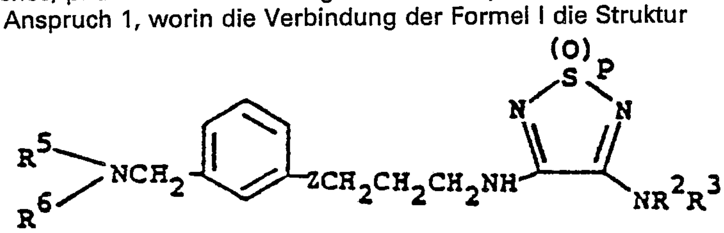

hat, worin

p für 1 oder 2 steht;

Z für Sauerstoff oder Schwefel steht;

$R^2$ und $R^3$ jeweils unabhängig voneinander für Wasserstoff oder $C_1$—$C_6$-Alkyl stehen, oder, wenn $R^2$ für Wasserstoff steht, $R^3$ auch für $C_2$—$C_6$-Alkenyl, $C_2$—$C_6$-Alkinyl, Pyridylmethyl oder

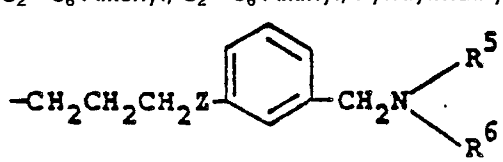

stehen kann, und

$R^5$ und $R^6$ jeweils unabhängig voneinander für Wasserstoff oder $C_1$—$C_6$-Alkyl stehen, oder, wenn $R^5$ für Wasserstoff steht, $R^6$ auch für $C_2$—$C_6$-Alkenyl oder $C_2$—$C_6$-Alkinyl stehen kann, oder $R^5$ und $R^6$ zusammen mit dem Stickstoffatom, an das sie gebunden sind, für Pyrrolidino, Methylpyrrolidino, Morpholino, Thiomorpholino, Piperidino, Methylpiperidino, Dimethylpiperidino, Homopiperidino oder Heptamethylenimino stehen können,
oder ein nicht-toxisches, pharmazeutisch verträgliches Salz, Hydrat oder Solvat davon.

12. Mittel nach Anspruch 1, worin die Verbindung der Formel I die Struktur

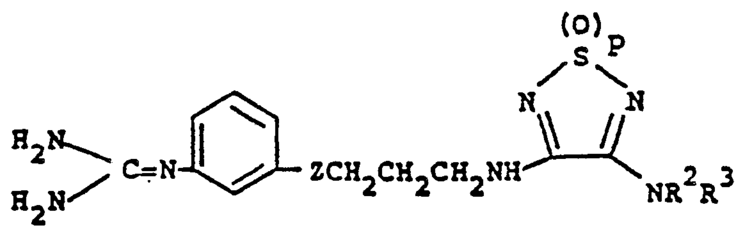

hat, worin
p für 1 oder 2 steht;
Z für Sauerstoff oder Schwefel steht;
$R^2$ und $R^3$ jeweils unabhängig voneinander für Wasserstoff oder $C_1$—$C_6$-Alkyl stehen, oder, wenn $R^2$ für Wasserstoff steht, $R^3$ auch für $C_2$—$C_6$-Alkenyl, $C_2$—$C_6$-Alkinyl, Pyridylmethyl oder

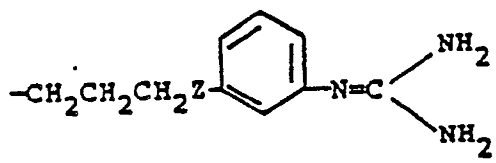

stehen kann,
oder ein nicht-toxisches, pharmazeutisch verträgliches Salz, Hydrat oder Solvat davon.

13. Mittel nach einem der Ansprüche 1 bis 12, worin die Pepstatin-Dosis 100 bis 175 mg und die Dosis der Verbindung der Formel I 2 bis 100 mg beträgt.

14. Mittel nach Anspruch 13, worin die Dosis der Verbindung der Formel I ca. 4 bis 50 mg beträgt.

15. Pharmazeutisches Mittel nach Anspruch 13, das 100 bis 175 mg Pepstatin und 2 bis 15 mg 3-Amino-4-{2-[(5-dimethylaminomethyl-2-furyl)-methylthio]-ethylamino}-1,2,5-thiadiazol-1-oxid oder ein nicht-toxisches, pharmazeutisch verträgliches Säureadditionssalz, Hydrat oder Solvat davon umfaßt.

16. Pharmazeutisches Mittel nach Anspruch 13, das 100 bis 175 mg Pepstatin und 2 bis 15 mg 3-Amino-4-{2-[(2-Guanidinothiazol-4-yl)methylthio]ethylamino}-1,2,5-thiadiazol-1-oxid oder ein nicht-toxisches, pharmazeutisch verträgliches Säureadditionssalz, Hydrat oder Solvat davon umfaßt.

17. Mittel nach den Ansprüchen 13 bis 16, worin Pepstatin in Form von flotierenden Pepstatinminikapseln vorhanden ist.

18. Mittel nach einem der Ansprüche 1—17 in Form von Dosiseinheiten.

19. Mittel nach einem der vorhergehenden Ansprüche, das zwei Teile umfaßt, wobei der erste Teil mindestens eine Verbindung der Formel I, definiert wie in den Ansprüchen 1 bis 12, 15 und 16, umfaßt und der zweite Teil Pepstatin umfaßt.

20. Verwendung von mindestens einer Verbindung der Formel I, definiert wie in den Ansprüchen 1 bis 12, 15 und 16, in Kombination mit Pepstatin zur Herstellung eines pharmazeutischen Mittels für die Behandlung peptischer Ulcera.

**Patentansprüche für den Vertragsstaat: AT**

1. Pharmazeutisches Mittel zur Behandlung peptischer Ulcera, das eine die peptische Aktivität inhibierende Menge an Pepstatin und eine wirksame antiulcerogene Menge mindestens einer Verbindung der Formel I:

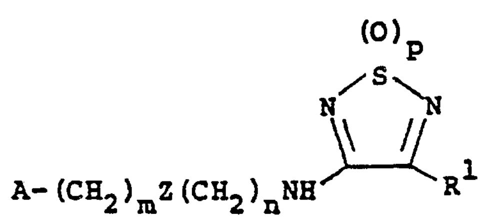

I

umfaßt, wobei in obiger Formel
p für 1 oder 2 steht;

$R^1$ für Hydroxy oder $NR^2R^3$ steht, wobei

$R^2$ und $R^3$ unabhängig voneinander für Wasserstoff, $C_1$—$C_6$-Alkyl, $C_2$—$C_6$-Alkenyl, $C_2$—$C_6$-Alkinyl, Cyclo-$C_3$—$C_7$-alkyl-$C_1$—$C_6$-alkyl, Hydroxy-$C_1$—$C_6$-alkyl, $C_1$—$C_6$-Alkoxy-$C_1$—$C_6$-alkyl, $C_1$—$C_6$-alkylthio-$C_1$—$C_6$-alkyl, 2-Fluorethyl, 2,2,2-Trifluorethyl oder Cyano-$C_1$—$C_6$-alkyl stehen, oder, wenn $R^2$ für Wasserstoff steht, $R^3$ auch für Cyclo-$C_3$—$C_7$-alkyl, Amino-$C_1$—$C_6$-alkyl, $C_1$—$C_6$-alkylamino-$C_1$—$C_6$-alkyl, Di-$C_1$—$C_6$-alkylamino-$C_1$—$C_6$-alkyl, Pyrrolidino-$C_1$—$C_6$-alkyl, Piperidino-$C_1$—$C_6$-alkyl, Morpholino-$C_1$—$C_6$-alkyl, Piperazino-$C_1$—$C_6$-alkyl, Pyridyl-$C_1$—$C_6$-alkyl, substituiertes Pyridyl-$C_1$—$C_6$-alkyl, wobei der Pyridylring einen Substituenten haben kann, der unter $C_1$—$C_6$-Alkyl, $C_1$—$C_6$-Alkoxy, Hydroxy, Amino und Halogen ausgewählt ist, Amino, $C_1$—$C_6$-Alkylamino, Di-$C_1$—$C_6$-alkylamino, Hydroxy, $C_1$—$C_6$-Alkoxy, 2,3-Dihydroxypropyl, Cyano, Amidino, $C_1$—$C_6$-Alkylamidino, A'—$(CH_2)_{m'}$Z'$(CH_2)_{n'}$—, Phenyl, Phenyl-$C_1$—$C_6$-alkyl, substituiertes Phenyl oder substituiertes Phenyl-$C_1$—$C_6$-alkyl stehen kann, wobei der Phenylring einen oder zwei Substituenten aufweisen kann, die unabhängig voneinander ausgewählt sind unter $C_1$—$C_6$-Alkyl, Hydroxy, $C_1$—$C_6$-Alkoxy und Halogen, oder einen Substituenten aufweisen kann, der ausgewählt ist unter Methylendioxy, Trifluormethyl und Di-$C_1$—$C_6$-alkylamino, oder $R^2$ und $R^3$ zusammen für —$CH_2CH_2X(CH_2)_r$— stehen können, wobei

r für eine ganze Zahl von 1 bis einschließlich 3 steht;

X für Methylen, Schwefel, Sauerstoff oder N—$R^4$ unter der Bedingung steht, daß, wenn r für 1 steht, X für Methylen steht, wobei

$R^4$ für Wasserstoff, $C_1$—$C_6$-Alkyl, $C_2$—$C_6$-Alkenyl, $C_2$—$C_6$-Alkinyl, $C_1$—$C_6$-Alkanoyl oder Benzoyl steht;

m und m' unabhängig voneinander für eine ganze Zahl von 0 bis einschließlich 2 stehen;

n und n' unabhängig voneinander für eine ganze Zahl von 2 bis einschließlich 4 stehen;

Z und Z' unabhängig voneinander für Schwefel, Sauerstoff oder Methylen stehen;

A und A' unabhängig voneinander für Phenyl, Imidazolyl, Thiazolyl, Isothiazolyl, Oxazolyl, Isoxazolyl, Triazolyl, Thiadiazolyl, Oxadiazolyl, Furyl, Thienyl oder Pyridyl stehen, wobei A und A' unabhängig voneinander einen oder zwei Substituenten aufweisen können, wobei der erste Substituent ausgewählt ist unter $C_1$—$C_6$-Alkyl, Hydroxy; Trifluormethyl, Halogen, Amino, Hydroxymethyl, $C_1$—$C_6$-Alkoxy,

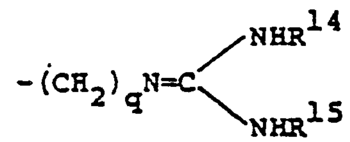

und $-(CH_2)_qNR^5R^6$,

und der zweite Substituent ausgewählt ist unter $C_1$—$C_6$-Alkyl, Hydroxy, Trifluoromethyl, Halogen, Amino, Hydroxymethyl und $C_1$—$C_6$-Alkoxy;

q für ein ganze Zahl von 0 bis einschließlich 6 steht;

$R^{14}$ und $R^{15}$ unabhängig voneinander für Wasserstoff oder $C_1$—$C_6$-Alkyl stehen, oder, wenn $R^{14}$ für Wasserstoff steht, $R^{15}$ auch für $C_1$—$C_6$-Alkanoyl oder Benzoyl stehen kann, oder, $R^{14}$ und $R^{15}$ zusammen für Ethylen stehen können; und

$R^5$ und $R^6$ unabhängig voneinander für Wasserstoff, $C_1$—$C_6$-Alkyl, $C_2$—$C_6$-Alkenyl, $C_2$—$C_6$-Alkinyl, $C_1$—$C_6$-Alkoxy-$C_1$—$C_6$-alkyl, Cyclo-$C_3$—$C_7$-alkyl, Phenyl oder Phenyl-$C_1$—$C_6$-alkyl unter der Bedingung stehen, daß $R^5$ und $R^6$ nicht beide für Cyclo-$C_3$—$C_7$-alkyl oder Phenyl stehen; oder $R^5$ und $R^6$ zusammen mit dem Stickstoffatom, an das sie gebunden sind, für Pyrrolidino, Methylpyrrolidino, Dimethylpyrrolidino, Morpholino, Thiomorpholino, Piperidino, Methylpiperidino, Dimethylpiperidino, Hydroxypiperidino, N-Methylpiperazino, Homopiperidino, Heptamethylenimino oder Octamethylenimino stehen, oder ein nichttoxisches, pharmazeutisch verträgliches Salz, Hydrat oder Solvat davon.

2. Mittel nach Anspruch 1, wobei in Formel I $R^1$ für $NR^2R^3$ steht und $R^2$ und $R^3$ wie in Anspruch 1 definiert sind.

3. Mittel nach Anspruch 1, worin die Verbindung der Formel I die Struktur

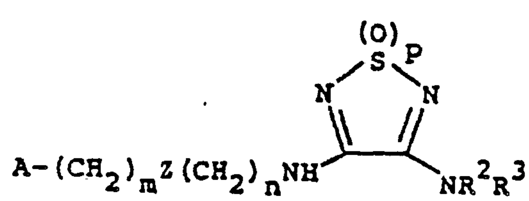

hat, worin

p für 1 oder 2 steht;

$R^2$ und $R^3$ unabhängig voneinander für Wasserstoff, $C_1$—$C_6$-Alkyl, $C_2$—$C_6$-Alkenyl, $C_2$—$C_6$-Alkinyl, Cyclo-$C_3$—$C_7$-alkyl-$C_1$—$C_6$-alkyl, Hydroxy-$C_1$—$C_6$-alkyl, $C_1$—$C_6$-alkoxy-$C_1$—$C_6$-alkyl, 2-Fluorethyl oder 2,2,2-Trifluorethyl stehen, oder, wenn $R^2$ für Wasserstoff steht, $R^3$ auch für Pyrrolidino-$C_1$—$C_6$-alkyl, Piperidino-$C_1$—$C_6$-alkyl, Morpholino-$C_1$—$C_6$-alkyl, Piperazino-$C_1$—$C_6$-alkyl, Pyridyl-$C_1$—$C_6$-alkyl, substituiertes Pyridyl-$C_1$—$C_6$-alkyl, worin der Pyridylring einen Substituenten aufweisen kann, der unter $C_1$—$C_6$-Alkyl, $C_1$—$C_6$-Alkoxy, Hydroxy, Amino und Halogen ausgewählt ist, Hydroxy, A'—$(CH_2)_{m'}$Z'$(CH_2)_{n'}$—, Phenyl-$C_1$—$C_6$-alkyl oder substituiertes Phenyl-$C_1$—$C_6$-alkyl stehen kann, wobei der Phenylring einen oder zwei Substituenten

haben kann, die unabhängig voneinander ausgewählt sind unter $C_1$—$C_6$-Alkyl, Hydroxy, $C_1$—$C_6$-Alkoxy und Halogen, oder einen Substituenten aufweisen kann, der ausgewählt ist unter Methylendioxy, Trifluormethyl und Di-$C_1$—$C_6$-alkylamino;

m und m' unabhängig voneinander für eine ganze Zahl von 0 bis einschließlich 2 stehen;

n und n' unabhängig voneinander für eine ganze Zahl von 2 bis einschließlich 4 stehen;

Z und Z' unabhängig voneinander für Schwefel, Sauerstoff oder Methylen stehen;

A und A' unabhängig voneinander für Phenyl, Imidazolyl, Thiazolyl, Oxazolyl, Thiadiazolyl, Oxadiazolyl, Furyl, Thienyl oder Pyridyl stehen, wobei A und A' unabhängig voneinander einen oder zwei Substituenten aufweisen können, wobei der erste Substituent ausgewählt ist unter $C_1$—$C_6$-Alkyl, Hydroxy, Trifluormethyl, Halogen, Amino, Hydroxymethyl, $C_1$—$C_6$-Alkoxy,

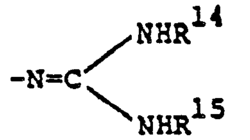 and $-CH_2NR^5R^6$,

und der zweite Substituent ausgewählt ist unter $C_1$—$C_6$-Alkyl, Hydroxy, Trifluoromethyl, Halogen, Amino, Hydroxymethyl und $C_1$—$C_6$-Alkoxy;

q für eine ganze Zahl von 0 bis einschließlich 6 steht;

$R^{14}$ und $R^{15}$ unabhängig voneinander für Wasserstoff oder $C_1$—$C_6$-Alkyl stehen, oder $R^{14}$ und $R^{15}$ zusammen für Ethylen stehen können; und

$R^5$ und $R^6$ unabhängig voneinander für Wasserstoff, $C_1$—$C_6$-Alkyl, $C_2$—$C_6$-Alkenyl oder $C_2$—$C_6$-Alkinyl stehen, oder $R^5$ und $R^6$ zusammen mit dem Stickstoffatom, an das sie gebunden sind, für Pyrrolidino, Methylpyrrolidino, Dimethylpyrrolidino, Morpholino, Thiomorpholino, Piperidino, Methylpiperidino, Dimethylpiperidino, Hydroxypiperidino, N-Methylpiperazino, Homopiperidino, Heptamethylenimino oder Octamethylenimino stehen können,

oder ein nicht-toxisches, pharmazeutisch verträgliches Salz, Hydrat oder Solvat davon.

4. Mittel nach Anspruch 1, worin die Verbindung der Formel I die Struktur

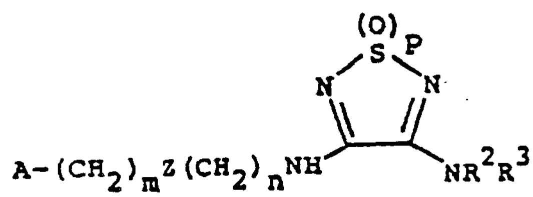

hat, worin

p für 1 oder 2 steht;

$R^2$ und $R^3$ unabhängig voneinander für Wasserstoff, $C_1$—$C_6$-Alkyl, $C_2$—$C_6$-Alkenyl, $C_2$—$C_6$-Alkinyl oder Cyclo-$C_3$—$C_7$-alkyl-$C_1$—$C_6$-alkyl stehen, oder, wenn $R^2$ für Wasserstoff steht, $R^3$ auch für Pyridyl-$C_1$—$C_6$-alkyl, substituiertes Pyridyl-$C_1$—$C_6$-alkyl, worin der Pyridylring einen Substituenten aufweisen kann, der unter $C_1$—$C_6$-Alkyl, $C_1$—$C_6$-Alkoxy, Hydroxy, Amino und Halogen ausgewählt ist, $A'—(CH_2)_{m'}Z'(CH_2)_{n'}—$, Phenyl-$C_1$—$C_6$-alkyl oder 3,4-Methylendioxybenzyl stehen kann;

m und m' unabhängig voneinander für 0 oder 1 stehen;

n und n' unabhängig voneinander für 2 oder 3 stehen;

Z und Z' unabhängig voneinander für Schwefel, Sauerstoff oder Methylen stehen;

A und A' unabhängig voneinander für Phenyl, Imidazolyl, Thiazolyl, Furyl, Thienyl oder Pyridyl stehen, wobei A und A' unabhängig voneinander einen oder zwei Substituenten aufweisen können, wobei der erste Substituent ausgewählt ist unter $C_1$—$C_6$-Alkyl,

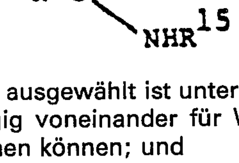 und $-CH_2NR^5R^6$,

und der zweite Substituent ausgewählt ist unter $C_1$—$C_6$-Alkyl;

$R^{14}$ und $R^{15}$ unabhängig voneinander für Wasserstoff oder $C_1$—$C_6$-Alkyl stehen, oder $R^{14}$ und $R^{15}$ zusammen für Ethylen stehen können; und

$R^5$ und $R^6$ unabhängig voneinander für Wasserstoff oder $C_1$—$C_6$-Alkyl stehen, oder $R^5$ und $R^6$ zusammen mit dem Stickstoffatom, an das sie gebunden sind, für Pyrrolidino, Methylpyrrolidino, Dimethylpyrrolidino, Morpholino, Thiomorpholino, Piperidino, Methylpiperidino, Dimethylpiperidino, Hydroxypiperidino, N-Methylpiperazino, Homopiperidino, Heptamethylenimino oder Octamethylenimino stehen können,

und ein nicht-toxisches, pharmazeutisch verträgliches Salz, Hydrat oder Solvat davon.

5. Mittel nach Anspruch 1, worin die Verbindung der Formel I die Struktur

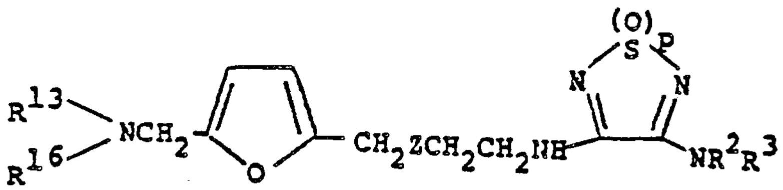

hat, worin

p für 1 oder 2 steht;

Z für Schwefel oder Methylen steht;

$R^2$ und $R^3$ unabhängig voneinander für Wasserstoff oder $C_1$—$C_6$-Alkyl stehen, oder, wenn $R^2$ für Wasserstoff steht, $R^3$ auch für $C_2$—$C_6$-Alkenyl, $C_2$—$C_6$-Alkinyl, Phenyl-$C_1$—$C_6$-alkyl, Cyclo-$C_3$—$C_7$-alkyl-$C_1$—$C_6$-alkyl, Pyridylmethyl oder

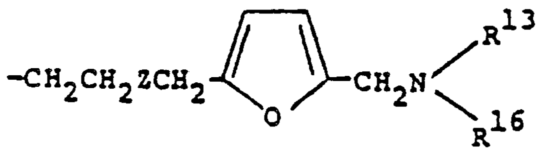

stehen kann;

$R^{16}$ für Methyl und

$R^{13}$ für Wasserstoff oder Methyl stehen, oder $R^{16}$ und $R^{13}$ zusammen mit dem Stickstoffatom, an das sie gebunden sind, für Piperidino stehen können;

oder ein nicht-toxisches, pharmazeutisch verträgliches Salz, Hydrat oder Solvat davon.

6. Mittel nach Anspruch 1, worin die Verbindung der Formel I die Struktur

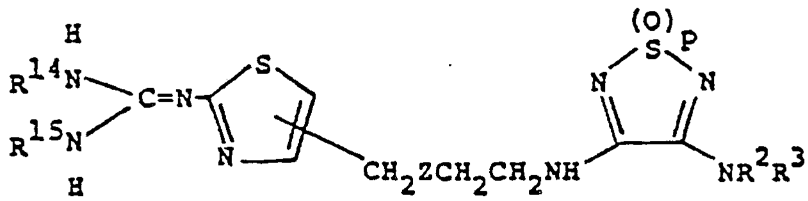

hat, worin

p für 1 oder 2 steht;

Z für Schwefel oder Methylen steht;

$R^{14}$ und $R^{15}$ unabhängig voneinander für Wasserstoff oder Methyl stehen, oder $R^{14}$ und $R^{15}$ zusammen für Ethylen stehen können; und

$R^2$ und $R^3$ unabhängig voneinander für Wasserstoff oder $C_1$—$C_6$-Alkyl stehen, oder, wenn $R^2$ für Wasserstoff steht, $R^3$ auch für $C_2$—$C_6$-Alkenyl, $C_2$—$C_6$-Alkinyl, Pyridylmethyl,

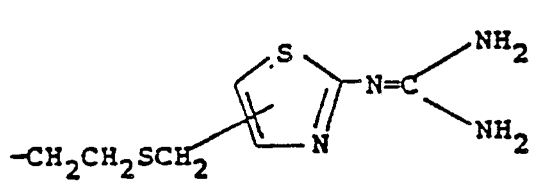

oder

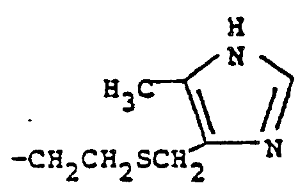

stehen kann;

oder ein nicht-toxisches, pharmazeutisch verträgliches Salz, Hydrat oder Solvat davon.

7. Mittel nach Anspruch 1, worin die Verbindung der Formel I die Struktur

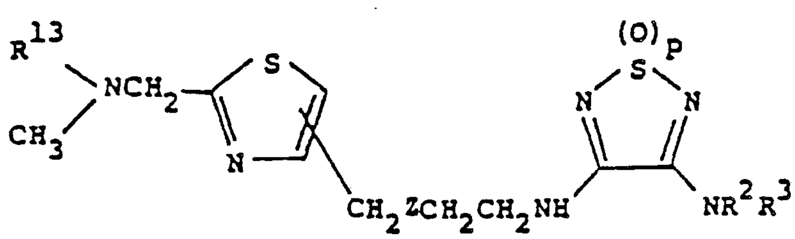

hat, worin

p für 1 oder 2 steht;

Z für Schwefel oder Methylen steht;

$R_2$ und $R_3$ unabhängig voneinander für Wasserstoff oder $C_1$—$C_6$-Alkyl stehen, oder, wenn $R^2$ für Wasserstoff steht, $R^3$ auch für $C_2$—$C_6$-Alkenyl, $C_2$—$C_6$-Alkinyl oder

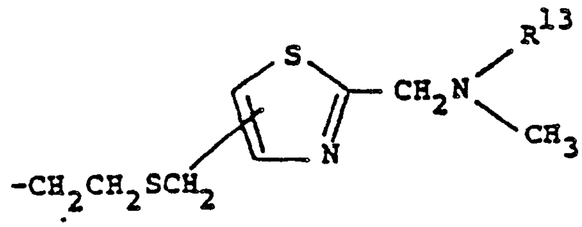

stehen kann; und

$R^{13}$ für Wasserstoff oder Methyl steht,

oder ein nicht-toxisches, pharmazeutisch verträgliches Salz, Hydrat oder Solvat davon.

8. Mittel nach Anspruch 1, worin die Verbindung der Formel I die Struktur

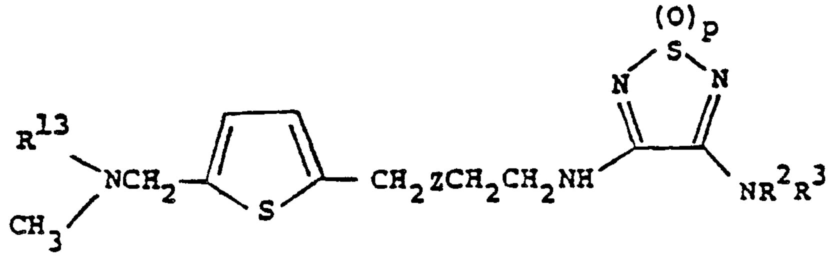

hat, worin

p für 1 oder 2 steht;

Z für Schwefel oder Methylen steht;

$R^2$ und $R^3$ jeweils unabhängig voneinander für Wasserstoff oder $C_1$—$C_6$-Alkyl stehen, oder, wenn $R^2$ für Wasserstoff steht, $R^3$ auch für $C_2$—$C_6$-Alkenyl, $C_2$—$C_6$-Alkinyl, Phenyl-$C_1$—$C_6$-alkyl, Pyridylmethyl, 3,4-Methylendioxybenzyl oder

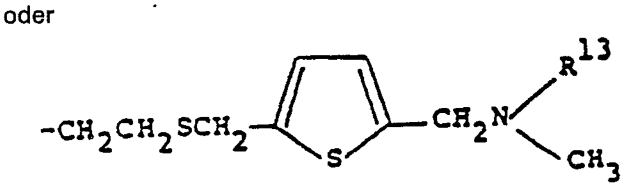

steht, und

$R^{13}$ für Wasserstoff oder Methyl steht,

oder ein nicht-toxisches, pharmazeutisch verträgliches Salz, Hydrat oder Solvat davon.

9. Mittel nach Anspruch 1, worin die Verbindung der Formel I die Struktur

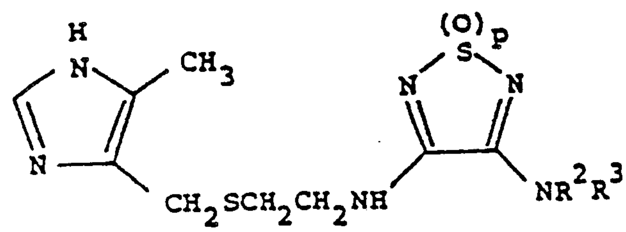

hat, worin

p für 1 oder 2 steht; und

$R^2$ und $R^3$ jeweils unabhängig voneinander für Wasserstoff oder $C_1$—$C_6$-Alkyl stehen, oder, wenn $R^2$ für Wasserstoff steht, $R^3$ auch für $C_2$—$C_6$-Alkenyl, $C_2$—$C_6$-Alkinyl oder

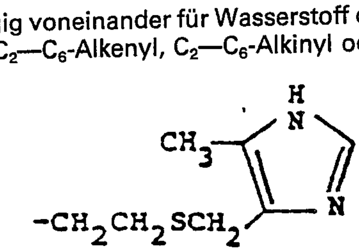

stehen kann,

oder ein nicht-toxisches, pharmazeutisch verträgliches Salz, Hydrat oder Solvat davon.

10. Mittel nach Anspruch 1, worin die Verbindung der Formel I die Struktur

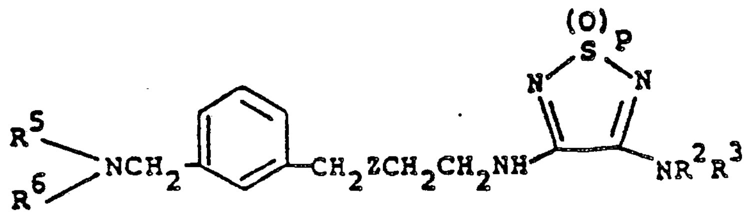

hat, worin

p für 1 oder 2 steht;

Z für Schwefel oder Methylen steht;

$R^2$ und $R^3$ jeweils unabhängig voneinander für Wasserstoff oder $C_1$—$C_6$-Alkyl stehen, oder, wenn $R^2$ für Wasserstoff steht, $R^3$ auch für $C_2$—$C_6$-Alkenyl, $C_2$—$C_6$-Alkinyl oder

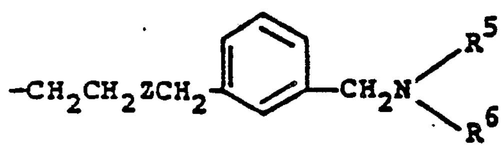

stehen kann, und

$R^5$ und $R^6$ jeweils unabhängig voneinander für Wasserstoff oder $C_1$—$C_6$-Alkyl stehen, oder $R^5$ und $R^6$ zusammen mit dem Stickstoffatom, an das sie gebunsind, für Piperidino stehen können,

oder ein nicht-toxisches, pharmazeutisch verträgliches Salz, Hydrat oder Solvat davon.

11. Mittel nach Anspruch 1, worin die Verbindung der Formel I die Struktur

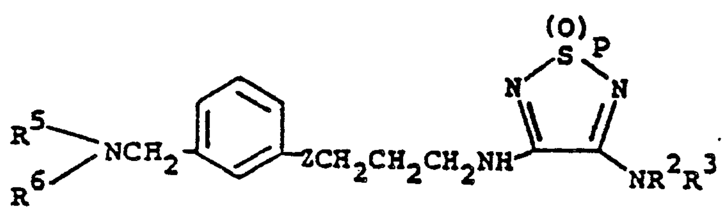

hat, worin

p für 1 oder 2 steht;

Z für Sauerstoff oder Schwefel steht;

$R^2$ und $R^3$ jeweils unabhängig voneinander für Wasserstoff oder $C_1$—$C_6$-Alkyl stehen, oder, wenn $R^2$ für Wasserstoff steht, $R^3$ auch für $C_2$—$C_6$-Alkenyl, $C_2$—$C_6$-Alkinyl, Pyridylmethyl oder

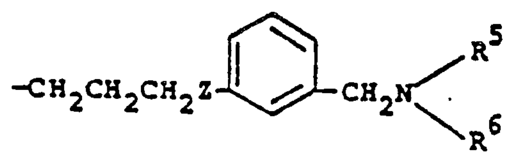

stehen kann, und

$R^5$ und $R^6$ jeweils unabhängig voneinander für Wasserstoff oder $C_1$—$C_6$-Alkyl stehen, oder, wenn $R^5$ für Wasserstoff steht, $R^6$ auch für $C_2$—$C_6$-Alkenyl oder $C_2$—$C_6$-Alkinyl stehen kann, oder $R^5$ und $R^6$ zusammen mit dem Stickstoffatom, an das sie gebunden sind, für Pyrrolidino, Methylpyrrolidino, Morpholino, Thiomorpholino, Piperidino, Methylpiperidino, Dimethylpiperidino, Homopiperidino oder Heptamethylenimino stehen können,

oder ein nicht-toxisches, pharmazeutisch verträgliches Salz, Hydrat oder Solvat davon.

12. Mittel nach Anspruch 1, worin die Verbindung der Formel I die Struktur

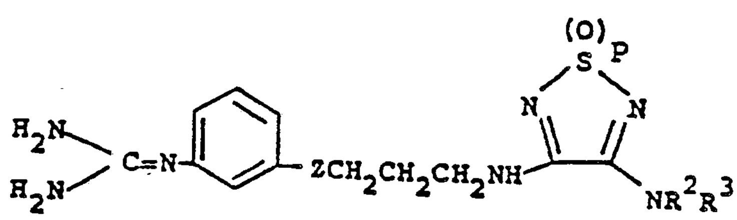

hat, worin

p für 1 oder 2 steht;
Z für Sauerstoff oder Schwefel steht;
$R^2$ und $R^3$ jeweils unabhängig voneinander für Wasserstoff oder $C_1$—$C_6$-Alkyl stehen, oder, wenn $R^2$ für Wasserstoff steht, $R^3$ auch für $C_2$—$C_6$-Alkenyl, $C_2$—$C_6$-Alkinyl, Pyridylmethyl oder

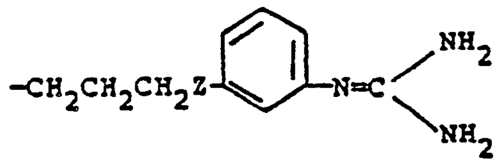

stehen kann,
oder ein nicht-toxisches, pharmazeutisch verträgliches Salz, Hydrat oder Solvat davon.

13. Mittel nach einem der Ansprüche 1 bis 12, worin die Pepstatin-Dosis 100 bis 175 mg und die Dosis der Verbindung der Formel I 2 bis 100 mg beträgt.

14. Mittel nach Anspruch 13, worin die Dosis der Verbindung der Formel I ca. 4 bis 50 mg beträgt.

15. Pharmazeutisches Mittel nach Anspruch 13, das 100 bis 175 mg Pepstatin und 2 bis 15 mg 3-Amino-4-{2-[(5-dimethylaminomethyl-2-furyl)-methylthio]-ethylamino}-1,2,5-thiadiazol-1-oxid oder ein nicht-toxisches, pharmazeutisch verträgliches Säureadditionssalz, Hydrat oder Solvat davon umfaßt.

16. Pharmazeutisches Mittel nach Anspruch 13, das 100 bis 175 mg Pepstatin und 2 bis 15 mg 3-Amino-4-{2-[(2-Guanidinothiazol-4-yl)methylthio]ethylamino}-1,2,5-thiadiazol-1-oxid oder ein nicht-toxisches, pharmazeutisch verträgliches Säureadditionssalz, Hydrat oder Solvat davon umfaßt.

17. Mittel nach den Ansprüchen 13 bis 16, worin Pepstatin in Form von flotierenden Pepstatinminikapseln vorhanden ist.

18. Mittel nach einem der Ansprüche 1—17 in Form von Dosiseinheiten.

19. Mittel nach einem der vorhergehenden Ansprüche, das zwei Teile umfaßt, wobei der erste Teil mindestens eine Verbindung der Formel I, definiert wie in den Ansprüchen 1 bis 12, 15 und 16, umfaßt und der zweite Teil Pepstatin umfaßt.

20. Verwendung von mindestens einer Verbindung der Formel I, definiert wie in den Ansprüchen 1 bis 12, 15 und 16, in Kombination mit Pepstatin zur Herstellung eines pharmazeutischen Mittels für die Behandlung peptischer Ulcera.

21. Verfahren zur Herstellung eines Mittels nach einem der Ansprüche 1 bis 19, wobei man eine wirksame antiulcerogene Menge mindestens einer Verbindung der Formel I in Kombination mit einer die peptische Aktivität inhibierenden Menge an Pepstatin zur Verfügung stellt.

**Revendications pour les Etats contractants: BE CH LI FR IT NL SE DE GB LU**

1. Composition pharmaceutique utile pour le traitement des ulcères de l'estomac qui comprend une quantité de pepstatine inhibitrice de l'activité peptique et une quantité antiulcérogène efficace d'au moins un dérivé de formule I:

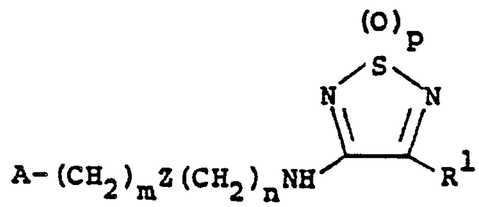

I

dans laquelle:
p est égal à 1 ou à 2;
$R^1$ représentent un groupe hydroxy ou $NR^2R^3$;
$R^2$ et $R^3$ représentent chacun indépendamment un atome d'hydrogène, un radical alkyle en $C_1$ à $C_6$, alkényle en $C_2$ à $C_6$, alkynyle en $C_2$ à $C_6$, cycloalkyle en $C_3$ à $C_7$-alkyle en $C_1$ à $C_6$, hydroxyalkyle en $C_1$ à $C_6$, alkoxy en $C_1$ à $C_6$-alkyle en $C_1$ à $C_6$, thioalkyle en $C_1$ à $C_6$-alkyle en $C_1$ à $C_6$, 2-fluoroéthyle, 2,2,2-trifluoroéthyle ou cyanoalkyle en $C_1$ à $C_6$, ou, lorsque $R^2$ représente un atome d'hydrogène, $R^3$ peut également représenter un radical cycloalkyle en $C_3$ à $C_7$, aminoalkyle en $C_1$ à $C_6$, aminoalkyle en $C_1$ à $C_6$-alkyle en $C_1$ à $C_6$, diaminoalkyle en $C_1$ à $C_6$-alkyle en $C_1$ à $C_6$, pyrrolidinoalkyle en $C_1$ à $C_6$, pipéridinoalkyle en $C_1$ à $C_6$, morpholinoalkyle en $C_1$ à $C_6$, pipérazinoalkyle en $C_1$ à $C_6$, pyridylalkyle en $C_1$ à $C_6$, pyridylalkyle en $C_1$ à $C_6$ substitué dans lequel le cycle de la pyridyle peut également contenir un substituant choisi parmi les suivants: alkyle en $C_1$ à $C_6$, alkoxy en $C_1$ à $C_6$, hydroxy, amino et halogène, amino, aminoalkyle en $C_1$ à $C_6$, diaminoalkyle en $C_1$ à $C_6$, hydroxy, alkoxy en $C_1$ à $C_6$, 2,3-dihydroxypropyle, cyano, amidino, amidinoalkyle en $C_1$ à $C_6$, $A'—(CH_2)_{m'}Z'(CH_2)_{n'}—$, phényle, phénylalkyle en $C_1$ à $C_6$, phényle substitué ou phénylalkyle en $C_1$ à $C_6$ substitué dans lequel le cycle phénylique peut contenir un ou deux substituants indépendamment choisis parmi les suivants: alkyle en $C_1$ à $C_6$, hydroxy, alkoxy en $C_1$ à $C_6$ et un atome d'halogène ou un substituant choisi parmi les suivants: dioxyméthylène, trifluorométhyle et diaminoalkyle en $C_1$ à $C_6$; ou

$R^2$ et $R^3$, lorsqu'ils sont pris ensemble, peuvent représenter le groupe $—CH_2CH_2X(CH_2)_r—$;

r représente un nombre entier égal de 1 à 3, bornes comprises;

X représente le radical méthylène, un atome de soufre, d'oxygène ou $N—R^4$ pourvu que, lorsque r est égal à 1, X représente le radical méthylène;

$R^4$ représente un atome d'hydrogène, un radical alkyle en $C_1$ à $C_6$, alkényle en $C_2$ à $C_6$, alkynyle en $C_2$ à $C_6$, alkanoyle en $C_1$ à $C_6$ ou benzoyle;

m et m' représentent chacun indépendamment un nombre entier égal de 0 à 2, bornes comprises;

n et n' représentent chacun un nombre entier égal de 2 à 4, bornes comprises;

Z et Z' représentent chacun indépendamment un atome de soufre, d'oxygène, ou le radical méthylène;

A et A' représentent chacun indépendamment un radical phényle, imidazolyle, thiazolyle, isothiazolyle, oxazolyle, isoxazolyle, triazolyle, thiadiazolyle, oxadiazolyle, furyle, thiényle ou pyridyle; pourvu que A et A' puissent contenir indépendamment un ou deux substituants, le premier substituant étant choisi parmi les suivants: un radical alkyle en $C_1$ à $C_6$, hydroxy, trifluorométhyle, un atome d'halogène, un groupe amino, le radical hydroxyméthyle, un radical alkoxy en $C_1$ à $C_6$,

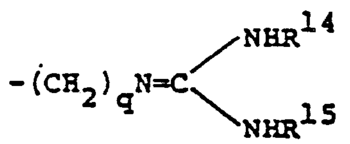

et

$-(CH_2)_qNR^5R^6$,

et le deuxième substituant étant choisi parmi un radical alkyle en $C_1$ à $C_6$, un groupe hydroxy, le radical trifluorométhyle, un halogène, un groupe amino, un radical hydroxyméthyle, et un alkoxy en $C_1$ à $C_6$;

q représente un nombre entier compris entre 0 et 6, bornes comprises;

$R^{14}$ et $R^{15}$ représentent chacun indépendamment un atome d'hydrogène ou un radical alkyle en $C_1$ à $C_6$, ou, si $R^{14}$ représente un atome d'hydrogène, $R^{15}$ peut également représenter un radical alkanoyle en $C_1$ à $C_6$, ou benzoyle; ou $R^{14}$ et $R^{15}$ pris ensemble peuvent représenter un radical éthylène; et

$R^5$ et $R^6$ représentent chacun indépendamment un atome d'hydrogène, un radical alkyle en $C_1$ à $C_6$, alkényle en $C_2$ à $C_6$, alkynyle en $C_2$ à $C_6$, alkoxy en $C_1$ à $C_6$-alkyle en $C_1$ à $C_6$, cycloalkyle en $C_3$ à $C_7$, phényle ou phénylalkyle en $C_1$ à $C_6$, pourvu que $R^5$ et $R^6$ ne soient pas en même temps un radical cycloalkyle en $C_3$ à $C_7$ ou phényle; ou $R^5$ et $R^6$, pris ensemble avec l'atome d'azote auquel ils sont liés, peuvent représenter: pyrrolidino, méthylpyrrolidino, diméthylpyrrolidino, morpholino, thiomorpholino, pipéridino, méthylpipéridino, diméthylpipéridino, hydroxypipéridino, N-méthylpipérazino, homopipéridino, heptaméthylèneimino ou octaméthylèneimino; ou un de leurs sels, hydratés ou solvatòs, non toxiques, pharmaceutiquement acceptables.

2. Une composition selon la revendication 1, dans laquelle, dans la formule I, $R^1$ représente le groupe $NR^2R^3$ et $R^2$ et $R^3$ sont tels que définis dans la revendication 1.

3. Une composition selon la revendication 1, dans laquelle le dérivé de formule I présente la structure:

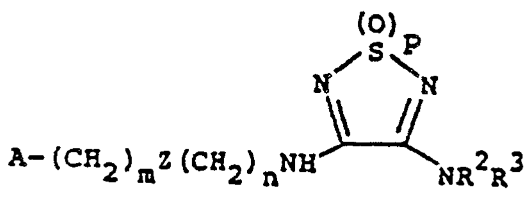

dans laquelle:

p est égal à 1 ou à 2;

$R^2$ et $R^3$ représentent chacun indépendamment un atome d'hydrogène, un radical alkyle en $C_1$ à $C_6$-alkényle en $C_2$ à $C_6$, alkynyle en $C_2$ à $C_6$, cycloalkyle en $C_3$ à $C_7$-alkyle en $C_1$ à $C_6$, hydroxyalkyle en $C_1$ à $C_6$, alkoxy en $C_1$ à $C_6$-alkyle en $C_1$ à $C_6$, 2-fluoroéthyle ou 2,2,2-trifluoroéthyle, ou, lorsque $R^2$ représente un atome d'hydrogène, $R^3$ peut également représenter un radical pyrrolidinoalkyle en $C_1$ à $C_6$, pipéridinoalkyle en $C_1$ à $C_6$, morpholinoalkyle en $C_1$ à $C_6$, pipérazinoalkyle en $C_1$ à $C_6$, pyridylalkyle en $C_1$ à $C_6$, pyridylalkyle en $C_1$ à $C_6$ substitué dans lequel le cycle pyridyle peut contenir un substituant choisi parmi les suivants: alkyle en $C_1$ à $C_6$, alkoxy en $C_1$ à $C_6$, hydroxy, amino et halogène, hydroxy, $A'—(CH_2)_{m'}Z'(CH_2)_{n'}—$, phénylalkyle en $C_1$ à $C_6$, ou phénylalkyle en $C_1$ à $C_6$ substitué dans lequel le cycle phényle peut contenir un ou deux substituants indépendamment choisis parmi les suivants: alkyle en $C_1$ à $C_6$, hydroxy, alkoxy en $C_1$ à $C_6$, un halogène, ou un substituant choisi parmi les suivants: dioxyméthylène, trifluorométhyle et diaminoalkyle en $C_1$ à $C_6$;

m et m' représentent chacun indépendamment un nombre entier égal de 0 à 2, bornes comprises;

n et n' représentent chacun un nombre entier égal de 2 à 4, bornes comprises;

Z et Z' représentent chacun indépendamment un atome de soufre, d'oxygène, ou le radical méthylène;

A et A' représentent chacun indépendamment un radical phenyle, imidazolyle, thiazolyle, oxazolyle, thiadiazolyle, oxadiazolyle, furyle, thiényle ou pyridyle; pourvu que A et A' puissent contenir indépendamment un ou deux substituants, le premier substituant étant choisi parmi les suivants: un

radical alkyle en $C_1$ à $C_6$, hydroxy, trifluorométhyle, un atome d'halogène, un groupe amino, le radical hydroxyméthyle, un radical alkoxy en $C_1$ à $C_6$,

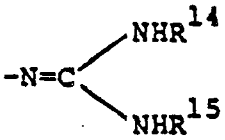

and $-CH_2NR^5R^6$,

et le deuxième substituant étant choisi parmi un radical alkyle en $C_1$ à $C_6$, un groupe hydroxy, le radical trifluorométhyle, un atome d'halogène, un groupe amino, un radical hydroxyméthyle et un groupe alkoxy en $C_1$ à $C_6$;

q représente un nombre entier compris entre 0 et 6, bornes comprises;

$R^{14}$ et $R^{15}$ représentent chacun indépendamment un atome d'hydrogène ou un radical alkyle en $C_1$ à $C_6$, ou $R^{14}$ et $R^{15}$ pris ensemble peuvent représenter un groupe éthylène; et

$R^5$ et $R^6$ représentent chacun indépendamment un atome d'hydrogène, un radical alkyle en $C_1$ à $C_6$, alkényle en $C_2$ à $C_6$, alkynyle en $C_2$ à $C_6$; ou $R^5$ et $R^6$, pris ensemble avec l'atome d'azote auquel ils sont liés, peuvent représenter: pyrrolidino, méthylpyrrolidino, diméthylpyrrolidino, morpholino, thiomorpholino, pipéridino, méthylpipéridino, diméthylpipéridino, hydroxypipéridino, N-méthylpipérazino, homopipéridino, heptaméthylèneimino ou octaméthylèneimino; ou un de leurs sels, hydratés ou solvatés, non toxiques, pharmaceutiquement acceptables.

4. Une composition selon la revendication 1, dans laquelle le dérivé de formule I présente la structure:

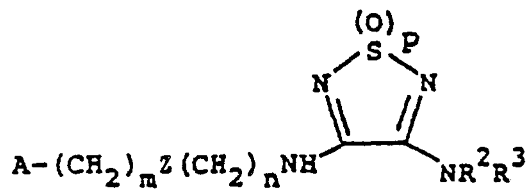

dans laquelle:

p est égal à 1 ou à 2;

$R^2$ et $R^3$ représentent chacun indépendamment un atome d'hydrogène, un radical alkyle en $C_1$ à $C_6$, alkényle en $C_2$ à $C_6$, alkynyle en $C_2$ à $C_6$ ou cycloalkyle en $C_3$ à $C_7$-alkyle en $C_1$ à $C_6$, ou, lorsque $R^2$ représente un atome d'hydrogène, $R^3$ peut également représenter un radical pyridylalkyle en $C_1$ à $C_6$, pyridylalkyle en $C_1$ à $C_6$ substitué dans lequel le cycle pyridyle peut contenir un substituant choisi parmi: alkyle en $C_1$ à $C_6$, alkoxy en $C_1$ à $C_6$, hydroxy, amino et halogène, $A'—(CH_2)_{m'}Z'(CH_2)_{n'}—$, phénylalkyle en $C_1$ à $C_6$ ou 3,4-méthylènedioxybenzyle;

m et m' peuvent chacun indépendamment être égaux à 0 ou à 1;

n et n' peuvent chacun indépendamment être égaux à 2 ou à 3;

Z et Z' représentent chacun indépendamment un atome de soufre, d'oxygène ou le groupe méthylène;

A et A' représentent chacun indépendamment: phényle, imidazolyle, thiazolyle, furyle, thiényle ou pyridyle; pourvu que A et A' puissent contenir indépendamment un ou deux substituants, le premier substituant étant choisi parmi un radical alkyle en $C_1$ à $C_6$,

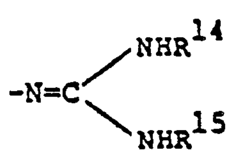

et $-CH_2NR^5R^6$,

et le deuxième substituant étant choisi parmi un radical alkyle en $C_1$ à $C_6$;

$R^{14}$ et $R^{15}$ représentent chacun indépendamment un atome d'hydrogène ou un radical alkyle en $C_1$ à $C_6$, ou $R^{14}$ et $R^{15}$, pris ensemble, peuvent représenter un groupe éthylène; et

$R^5$ et $R^6$ représentent chacun indépendamment un atome d'hydrogène ou un radical alkyle en $C_1$ à $C_6$, ou $R^5$ et $R^6$, lorsqu'ils sont pris ensemble en même temps que l'atome d'azote auquel ils sont attachés, peuvent représenter un groupe pyrrolidino, méthylpyrrolidino, diméthylpyrrolidino, morpholino, thiomorpholino, pipéridino, méthylpipéridino, diméthylpipéridino, hydroxypipéridino, N-méthylpipérazino, homopipéridino, heptaméthylèneimino ou octaméthylèneimino; ou un de leurs sels, hydratés ou solvatés, non toxiques, pharmaceutiquement acceptables.

5. Une composition selon la revendication 1, dans laquelle le dérivé de formule I présente la structure:

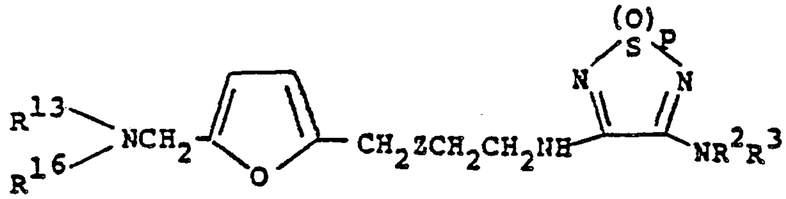

dans laquelle:

p est égal à 1 ou à 2;

Z représente un atome de soufre ou le groupe méthylène;

$R^2$ et $R^3$ représentent chacun indépendamment un atome d'hydrogène ou un radical alkényle en $C_1$ à $C_6$ ou, lorsque $R^2$ représente un atome d'hydrogène, $R^3$ peut également représenter: alkényle en $C_2$ à $C_6$, alkynyle en $C_2$ à $C_6$, phénylalkyle en $C_1$ à $C_6$, cycloalkyle en $C_3$ à $C_7$-alkyle en $C_1$ à $C_6$, pyridylméthyle ou

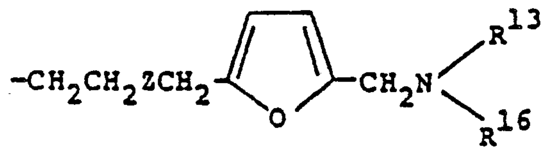 ;

$R^{16}$ représente un radical méthyle; et

$R^{13}$ représente un atome d'hydrogène ou le radical méthyle, ou $R^{16}$ et $R^{13}$, pris ensemble avec l'atome d'azote auquel ils sont attachés, peuvent former le groupe pipéridino; ou un de leurs sels, hydratés ou solvatés, non toxiques, pharmaceutiquement acceptables.

6. Une composition selon la revendication 1, dans laquelle le dérivé de formule I présente la structure:

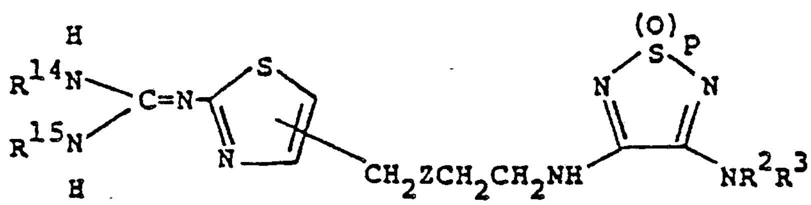

dans laquelle:

p est égal à 1 ou à 2;

Z représente un atome de soufre ou le groupe méthylène;

$R^{14}$ et $R^{15}$ représentent chacun indépendamment un atome d'hydrogène ou le radical méthyle; ou $R^{14}$ et $R^{15}$, lorsqu'ils sont pris ensemble, peuvent former le groupe éthylène; et

$R^2$ et $R^3$ représentent chacun indépendamment un atome d'hydrogène ou le radical alkyle en $C_1$ à $C_6$ ou, lorsque $R^2$ représente un atome d'hydrogène, $R^3$ peut également représenter le groupe alkényle en $C_2$ à $C_6$, alkynyle en $C_2$ à $C_6$, pyridylméthyle, ou

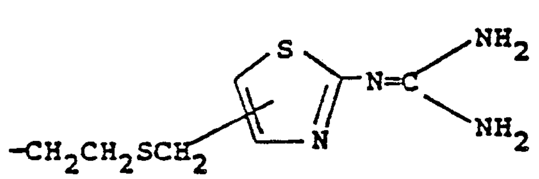 ou 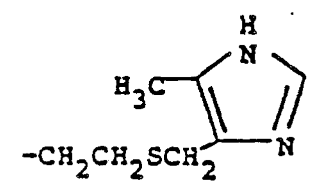 ;

ou un de leurs sels, hydratés ou solvatés, non toxiques, pharmaceutiquement acceptables.

7. Une composition selon la revendication 1, dans laquelle le dérivé de formule I présente la structure:

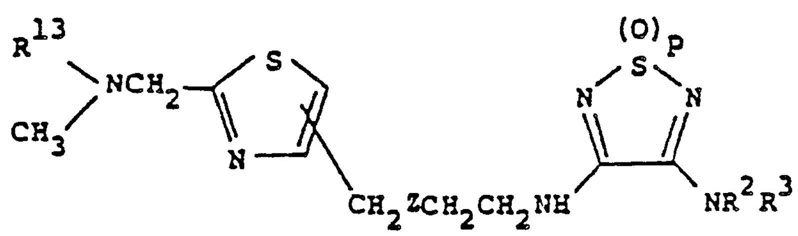

dans laquelle:

p est égal à 1 ou à 2;

Z représente un atome de soufre ou le groupe méthylène;

$R^2$ et $R^3$ représentent chacun indépendamment un atome d'hydrogène ou un radical alkyle en $C_1$ à $C_6$ ou, lorsque $R^2$ représente un atome d'hydrogène, $R^3$ peut également représenter un alkényle en $C_2$ à $C_6$, alkynyle en $C_2$ à $C_6$ ou

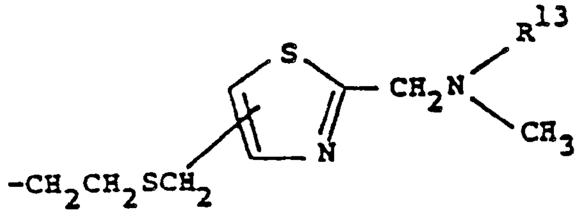

;

et

$R^{13}$ représente un atome d'hydrogène ou le radical méthyle; ou un de leurs sels, hydratés ou solvatés, non toxiques, pharmaceutiquement acceptables.

8. Une composition selon la revendication 1, dans laquelle le dérivé de formule I présente la structure:

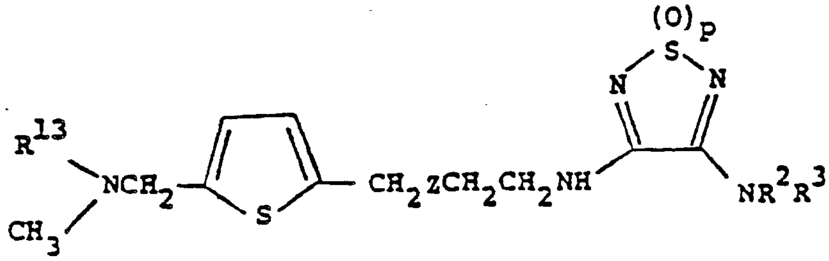

dans laquelle:

p est égal à 1 ou à 2;

Z représente un atome de soufre ou le groupe méthylène;

$R^2$ et $R^3$ représentent chacun indépendamment un atome d'hydrogène ou un radical alkyle en $C_1$ à $C_6$ ou, lorsque $R^2$ représente un atome d'hydrogène, $R^3$ peut également représenter un alkényle en $C_2$ à $C_6$, alkynyle en $C_2$ à $C_6$, phénylalkyle en $C_1$ à $C_6$, pyridylméthyle, 3,4-méthylènedioxybenzyle ou

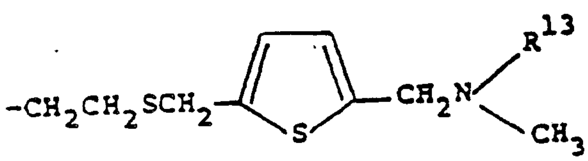

;

et

$R^{13}$ représente un atome d'hydrogène ou le radical méthyle; ou un de leurs sels, hydratés ou solvatés, non toxiques, pharmaceutiquement acceptables.

9. Une composition selon la revendication 1, dans laquelle le dérivé de formule I présente la structure:

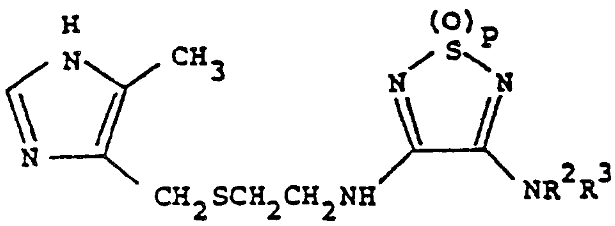

dans laquelle:

p est égal à 1 ou à 2;

$R^2$ et $R^3$ représentent chacun indépendamment un atome d'hydrogène ou un radical alkyle en $C_1$ à $C_6$ ou, lorsque $R^2$ représente un atome d'hydrogène, $R^3$ peut également représenter un alkényle en $C_2$ à $C_6$, alkynyle en $C_2$ à $C_6$ ou

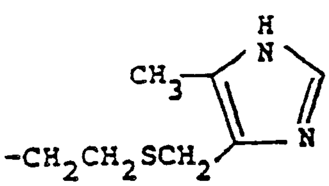

ou un de leurs sels, hydratés ou solvatés, non toxiques, pharmaceutiquement acceptables.

10. Une composition selon la revendication 1, dans laquelle le dérivé de formule I présente la structure:

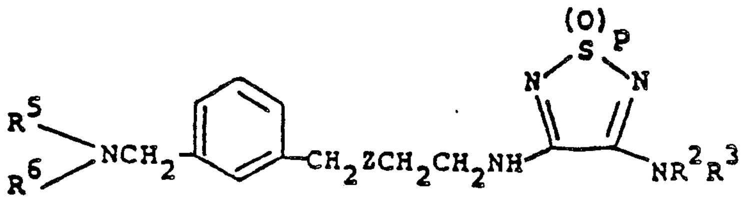

dans laquelle:
p est égal à 1 ou à 2;
Z représente un atome de soufre ou le groupe méthylène;
$R^2$ et $R^3$ représentent chacun indépendamment un atome d'hydrogène ou un radical alkyle en $C_1$ à $C_6$ ou, lorsque $R^2$ représente un atome d'hydrogène, $R^3$ peut également représenter un alkényle en $C_2$ à $C_6$, alkynyle en $C_2$ à $C_6$ ou

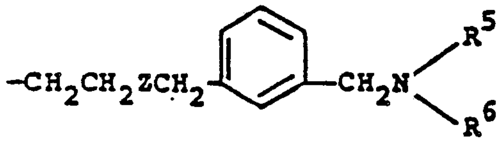 ;

et
$R^5$ et $R^6$ représentent chacun indépendamment un atome d'hydrogène ou un radical alkyle en $C_1$ à $C_6$ ou, $R^5$ et $R^6$, pris ensemble avec l'atome d'azote auquel ils sont attachés, peuvent représenter le groupe pipéridino; ou un de leurs sels, hydratés ou solvatés, non toxiques, pharmaceutiquement acceptables.

11. Une composition selon la revendication 1, dans laquelle le dérivé de formule I présente la structure:

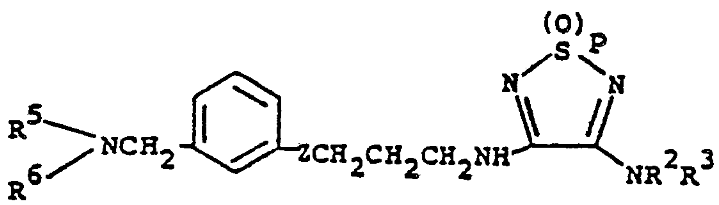

dans laquelle:
p est égal à 1 ou à 2;
Z représente un atome d'oxygène ou de soufre;
$R^2$ et $R^3$ représentent chacun indépendamment un atome d'hydrogène ou un radical alkyle en $C_1$ à $C_6$ ou, lorsque $R^2$ représente un atome d'hydrogène, $R^3$ peut également représenter un alkényle en $C_2$ à $C_6$, alkynyle en $C_2$ à $C_6$, pyridylméthyle ou

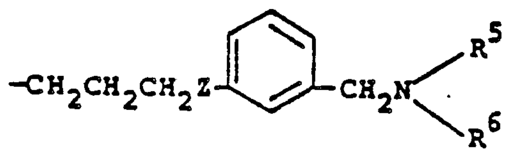 ;

et
$R^5$ et $R^6$ représentent chacun indépendamment un atome d'hydrogène ou un radical alkyle en $C_1$ à $C_6$ ou, lorsque $R^5$ représente un atome d'hydrogène, $R^6$ peut représenter un alkényle en $C_2$ à $C_6$ ou alkynyle en $C_2$ à $C_6$, ou, $R^5$ et $R^6$, pris ensemble avec l'atome d'azote auquel ils sont attachés, peuvent représenter un radical pyrrolidino, méthylpyrrolidino, morpholino, thiomorpholino, pipéridino, méthylpipéridino, diméthylpipéridino, homopipéridino ou heptaméthylèneimino; ou un de leurs sels, hydratés ou solvatés, non toxiques, pharmaceutiquement acceptables.

12. Une composition selon la revendication 1, dans laquelle le dérivé de formule I présente la structure:

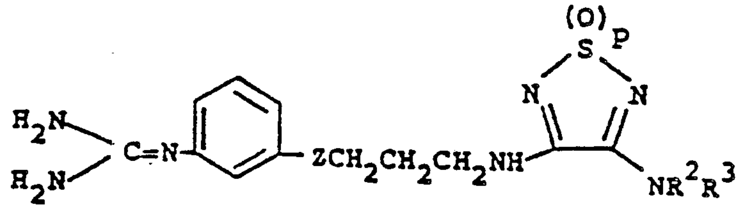

dans laquelle:
p est égal à 1 ou à 2;
Z représente un atome d'oxygène ou de soufre;
$R^2$ et $R^3$ représentent chacun indépendamment un atome d'hydrogène ou un radical alkyle en $C_1$ à $C_6$

ou, lorsque $R^2$ représente un atome d'hydrogène, $R^3$ peut représenter un alkényle en $C_2$ à $C_6$, alkynyle en $C_2$ à $C_6$, pyridylméthyle ou

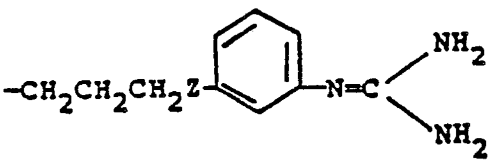

ou un de leurs sels, hydratés ou solvatés, non toxiques, pharmaceutiquement acceptables.

13. Une composition selon l'une quelconque des revendications 1 à 12, dans laquelle la dose de pepstatine est comprise entre 100 et 175 mg et la dose du dérivé de formule I est comprise entre 2 et 100 mg.

14. Une composition selon la revendication 13, dans laquelle la dose du dérivé de formule I est comprise entre environ 4 et 50 mg.

15. Une composition pharmaceutique selon la revendication 13, qui comprend de 100 à 175 mg de pepstatine et de 2 à 15 mg 1-oxyde de 3-amino-4-{2-[(5-diméthylaminométhyl-2-furyl)thiométhyl]aminoéthyl}-1,2,5-thiadiazol ou un de leurs sels d'addition d'acide, hydratés ou solvatés, non toxiques, pharmaceutiquement acceptables.

16. Une composition pharmaceutique selon la revendication 13, qui comprend de 100 à 175 mg de pepstatine et de 2 à 15 mg de 1-oxyde de 3-amino-4-{2-[(2-guanidinothiazol-4-yl)thiométhyl]aminoéthyl}-1,2,5-thiadiazol ou un de leurs sels d'addition d'acide, hydratés ou solvatés, non toxiques, pharmaceutiquement acceptables.

17. Une composition selon les revendications 13 à 16, dans laquelle la pepstatine se trouve sous la forme de minicapsules flottantes de pepstatine.

18. Une composition selon l'une quelconque des revendications 1 à 17, sous forme de dosage unitaire.

19. Une composition selon l'une quelconque des revendications précédentes, comprenant deux parties, la première partie étant constituée d'au moins un dérivé de formule I selon les revendications 1 à 12, 15 et 16, et la deuxième partie comprenant de la pepstatine.

20. Utilisation d'au moins un dérivé de formule I tel que défini dans les revendications 1 à 12, 15 et 16, en association avec de la pepstatine, pour la préparation d'une composition pharmaceutique de traitement des ulcères peptiques.

**Revendications pour l'Etat contractant: AT**

1. Composition pharmaceutique utile pour le traitement des ulcères de l'estomac qui comprend une quantité de pepstatine inhibitrice de l'activité peptique et une quantité antiulcérogène efficace d'au moins un dérivé de formule I:

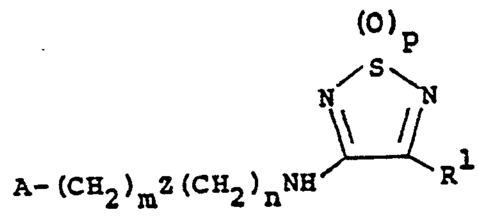

I

dans laquelle:

p est égal à 1 ou à 2;

$R^1$ représente un groupe hydroxy ou $NR^2R^3$;

$R^2$ et $R^3$ représentent chacun indépendamment un atome d'hydrogène, un radical alkyle en $C_1$ à $C_6$, alkényle en $C_2$ à $C_6$, alkynyle en $C_2$ à $C_6$, cycloalkyle en $C_3$ à $C_7$-alkyle en $C_1$ à $C_6$, hydroxyalkyle en $C_1$ à $C_6$, alkoxy en $C_1$ à $C_6$-alkyle en $C_1$ à $C_6$, thioalkyle en $C_1$ à $C_6$-alkyle en $C_1$ à $C_6$, 2-fluoroéthyle, 2,2,2-trifluoroéthyle ou cyan-alkyle en $C_1$ à $C_6$, ou, lorsque $R^2$ représente un atome d'hydrogène, $R^3$ peut également représenter un radical cycloalkyle en $C_3$ à $C_7$, aminoalkyle en $C_1$ à $C_6$, aminoalkyle en $C_1$ à $C_6$-alkyle en $C_1$ à $C_6$, diaminoalkyle en $C_1$ à $C_6$-alkyle en $C_1$ à $C_6$, pyrrolidinoalkyle en $C_1$ à $C_6$, pipéridinoalkyle en $C_1$ à $C_6$, morpholinoalkyle en $C_1$ à $C_6$, pipérazinoalkyle en $C_1$ à $C_6$, pyridylalkyle en $C_1$ à $C_6$, pyridylalkyle en $C_1$ à $C_6$ substitué dans lequel le cycle de la pyridyle peut également contenir un substituant choisi parmi les suivants: alkyle en $C_1$ à $C_6$, alkoxy en $C_1$ à $C_6$, hydroxy, amino et halogène, amino, aminoalkyle en $C_1$ à $C_6$, diaminoalkyle en $C_1$ à $C_6$, hydroxy, alkoxy en $C_1$ à $C_6$, 2,3-dihydroxypropyle, cyano, amidino, amidinoalkyle en $C_1$ à $C_6$, $A'-(CH_2)_{m'}Z'(CH_2)_{n'}-$, phényle, phénylalkyle en $C_1$ à $C_6$, phényle substitué ou phénylalkyle en $C_1$ à $C_6$ substitué dans lequel le cycle phénylique peut contenir un ou deux substituants indépendamment choisis parmi les suivants: alkyle en $C_1$ à $C_6$, hydroxy, alkoxy en $C_1$ à $C_6$ et un atome d'halogène ou un substituant choisi parmi les suivants: dioxyméthylène, trifluorométhyle et diaminoalkyle en $C_1$ à $C_6$; ou

$R^2$ et $R^3$, lorsqu'ils sont pris ensemble, peuvent représenter le groupe $-CH_2CH_2X(CH_2)_r-$;

r représente un nombre entier égal de 1 à 3, bornes comprises;

X représente le radical méthylène, un atome de soufre, d'oxygène ou N—$R^4$ pourvu que, lorsque r est égal à 1, X représente le radical méthylène;

$R^4$ représente un atome d'hydrogène, un radical alkyle en $C_1$ à $C_6$, alkényle en $C_2$ à $C_6$, alkynyle en $C_2$ à $C_6$, alkanoyle en $C_1$ à $C_6$ ou benzoyle;

m et m' représentent chacun indépendamment un nombre entier égal de 0 à 2, bornes comprises;

n et n' représentent chacun un nombre entier égal de 2 à 4, bornes comprises;

Z et Z' représentent chacun indépendamment un atome de soufre, d'oxygène, ou le radical méthylène;

A et A' représentent chacun indépendamment un radical phényle, imidazolyle, thiazolyle, isothiazolyle, oxazolyle, isoxazolyle, triazolyle, thiadiazolyle, oxadiazolyle, furyle, thiényle ou pyridyle; pourvu que A et A' puissent contenir indépendamment un ou deux substituants, le premier substituant étant choisi parmi les suivants: un radical alkyle en $C_1$ à $C_6$, hydroxy, trifluorométhyle, un atome d'halogène, un groupe amino, le radical hydroxyméthyle, un radical alkoxy en $C_1$ à $C_6$,

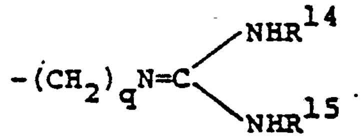 et $-(CH_2)_qNR^5R^6$,

et le deuxième substituant étant choisi parmi un radical alkyle en $C_1$ à $C_6$, un groupe hydroxy, le radical trifluorométhyle, un halogène, un groupe amino, un radical hydroxyméthyle, et un alkoxy en $C_1$ à $C_6$;

q représente un nombre entier compris entre 0 et 6, bornes comprises;

$R^{14}$ et $R^{15}$ représentent chacun indépendamment un atome d'hydrogène ou un radical alkyle en $C_1$ à $C_6$, ou, si $R^{14}$ représente un atome d'hydrogène, $R^{15}$ peut également représenter un radical alkanoyle en $C_1$ à $C_6$, ou benzoyle; ou $R^{14}$ et $R^{15}$ pris ensemble peuvent représenter un radical éthylène; et

$R^5$ et $R^6$ représentent chacun indépendamment un atome d'hydrogène, un radical alkyle en $C_1$ à $C_6$, alkényle en $C_2$ à $C_6$, alkynyle en $C_2$ à $C_6$, alkoxy en $C_1$ à $C_6$-alkyle en $C_1$ à $C_6$, cycloalkyle en $C_3$ à $C_7$, phényle ou phénylalkyle en $C_1$ à $C_6$, pourvu que $R^5$ et $R^6$ ne soient pas en même temps un radical cycloalkyle en $C_3$ à $C_7$ ou phényle; ou $R^5$ et $R^6$, pris ensemble avec l'atome d'azote auquel ils sont liés, peuvent représenter: pyrrolidino, méthylpyrrolidino, diméthylpyrrolidino, morpholino, thiomorpholino, pipéridino, méthylpipéridino, diméthylpipéridino, hydroxypipéridino, N-méthylpipérazino, homopipéridino, heptaméthylèneimino ou octaméthylèneimino; ou un de leurs sels, hydratés ou solvatòs, non toxiques, pharmaceutiquement acceptables.

2. Une composition selon la revendication 1, dans laquelle, dans la formule I, $R^1$ représente le groupe $NR^2R^3$ et $R^2$ et $R^3$ sont tels que définis dans la revendication 1.

3. Une composition selon la revendication 1, dans laquelle le dérivé de formule I présente la structure:

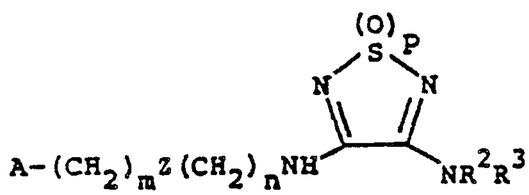

dans laquelle:

p est égal à 1 ou à 2;

$R^2$ et $R^3$ représentent chacun indépendamment un atome d'hydrogène, un radical alkyle en $C_1$ à $C_6$-alkényle en $C_2$ à $C_6$, alkynyle en $C_2$ à $C_6$, cycloalkyle en $C_3$ à $C_7$-alkyle en $C_1$ à $C_6$, hydroxyalkyle en $C_1$ à $C_6$, alkoxy en $C_1$ à $C_6$-alkyle en $C_1$ à $C_6$, 2-fluoroéthyle ou 2,2,2-trifluoroéthyle, ou, lorsque $R^2$ représente un atome d'hydrogène, $R^3$ peut également représenter un radical pyrrolidinoalkyle en $C_1$ à $C_6$, pipéridinoalkyle en $C_1$ à $C_6$, morpholinoalkyle en $C_1$ à $C_6$, pipérazinoalkyle en $C_1$ à $C_6$, pyridylalkyle en $C_1$ à $C_6$, pyridylalkyle en $C_1$ à $C_6$ substitué dans lequel le cycle pyridyle peut contenir un substituant choisi parmi les suivants: alkyle en $C_1$ à $C_6$, alkoxy en $C_1$ à $C_6$, hydroxy, amino et halogène, hydroxy, A'—$(CH_2)_{m'}Z'(CH_2)_{n'}$—, phénylalkyle en $C_1$ à $C_6$, ou phénylalkyle en $C_1$ à $C_6$ substitué dans lequel le cycle phényle peut contenir un ou deux substituants indépendamment choisis parmi les suivants: alkyle en $C_1$ à $C_6$, hydroxy, alkoxy en $C_1$ à $C_6$, un halogène, ou un substituant choisi parmi les suivants: dioxyméthylène, trifluorométhyle et diaminoalkyle en $C_1$ à $C_6$;

m et m' représentent chacun indépendamment un nombre entier égal de 0 à 2, bornes comprises;

n et n' représentent chacun un nombre entier égal de 2 à 4, bornes comprises;

Z et Z' représentent chacun indépendamment un atome de soufre, d'oxygène, ou le radical méthylène;

A et A' représentent chacun indépendamment un radical phenyle, imidazolyle, thiazolyle, oxazolyle, thiadiazolyle, oxadiazolyle, furyle, thiényle ou pyridyle; pourvu que A et A' puissent contenir indépendamment un ou deux substituants, le premier substituant étant choisi parmi les suivants: un radical alkyle en $C_1$ à $C_6$, hydroxy, trifluorométhyle, un atome d'halogène, un groupe amino, le radical hydroxyméthyle, un radical alkoxy en $C_1$ à $C_6$,

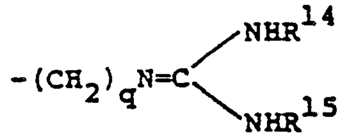

et $-(CH_2)_qNR^5R^6$,

et le deuxième substituant étant choisi parmi un radical alkyle en $C_1$ à $C_6$, un groupe hydroxy, le radical trifluorométhyle, un atome d'halogène, un groupe amino, un radical hydroxyméthyle et un groupe alkoxy en $C_1$ à $C_6$;

q représente un nombre entier compris entre 0 et 6, bornes comprises;

$R^{14}$ et $R^{15}$ représentent chacun indépendamment un atome d'hydrogène ou un radical alkyle en $C_1$ à $C_6$, ou $R^{14}$ et $R^{15}$ pris ensemble peuvent représenter un groupe éthylène; et

$R^5$ et $R^6$ représentent chacun indépendamment un atome d'hydrogène, un radical alkyle en $C_1$ à $C_6$, alkényle en $C_2$ à $C_6$, alkynyle en $C_2$ à $C_6$; ou $R^5$ et $R^6$, pris ensemble avec l'atome d'azote auquel ils sont liés, peuvent représenter: pyrrolidino, méthylpyrrolidino, diméthylpyrrolidino, morpholino, thiomorpholino, pipéridino, méthylpipéridino, diméthylpipéridino, hydroxypipéridino, N-méthylpipérazino, homopipéridino, heptaméthylèneimino ou octaméthylèneimino; ou un de leurs sels, hydratés ou solvatés, non toxiques, pharmaceutiquement acceptables.

4. Une composition selon la revendication 1, dans laquelle le dérivé de formule I présente la structure:

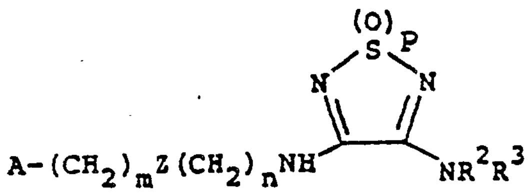

dans laquelle:

p est égal à 1 ou à 2;

$R^2$ et $R^3$ représentent chacun indépendamment un atome d'hydrogène, un radical alkyle en $C_1$ à $C_6$, alkényle en $C_2$ à $C_6$, alkynyle en $C_2$ à $C_6$ ou cycloalkyle en $C_3$ à $C_7$-alkyle en $C_1$ à $C_6$, ou, lorsque $R^2$ représente un atome d'hydrogène, $R^3$ peut également représenter un radical pyridylalkyle en $C_1$ à $C_6$, pyridylalkyle en $C_1$ à $C_6$ substitué dans lequel le cycle pyridyle peut contenir un substituant choisi parmi: alkyle en $C_1$ à $C_6$, alkoxy en $C_1$ à $C_6$, hydroxy, amino et halogène, $A'-(CH_2)_{m'}Z'(CH_2)_{n'}-$, phénylalkyle en $C_1$ à $C_6$ ou 3,4-méthylènedioxybenzyle;

m et m' peuvent chacun indépendamment être égaux à 0 ou à 1;

n et n' peuvent chacun indépendamment être égaux à 2 ou à 3;

Z et Z' représentent chacun indépendamment un atome de soufre, d'oxygène ou le groupe méthylène;

A et A' représentent chacun indépendamment: phényle, imidazolyle, thiazolyle, furyle, thiényle ou pyridyle; pourvu que A et A' puissent contenir indépendamment un ou deux substituants, le premier substituant étant choisi parmi un radical alkyle en $C_1$ à $C_6$,

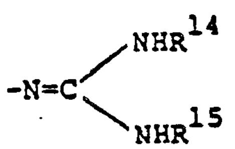

et $-CH_2NR^5R^6$,

et le deuxième substituant étant choisi parmi un radical alkyle en $C_1$ à $C_6$;

$R^{14}$ et $R^{15}$ représentent chacun indépendamment un atome d'hydrogène ou un radical alkyle en $C_1$ à $C_6$, ou $R^{14}$ et $R^{15}$, pris ensemble, peuvent représenter un groupe éthylène; et

$R^5$ et $R^6$ représentent chacun indépendamment un atome d'hydrogène ou un radical alkyle en $C_1$ à $C_6$, ou $R^5$ et $R^6$, lorsqu'ils sont pris ensemble en même temps que l'atome d'azote auquel ils sont attachés, peuvent représenter un groupe pyrrolidino, méthylpyrrolidino, diméthylpyrrolidino, morpholino, thiomorpholino, pipéridino, méthylpipéridino, diméthylpipéridino, hydroxypipéridino, N-méthylpipérazino, homopipéridino, heptaméthylèneimino ou octaméthylèneimino; ou un de leurs sels, hydratés ou solvatés, non toxiques, pharmaceutiquement acceptables.

5. Une composition selon la revendication 1, dans laquelle le dérivé de formule I présente la structure:

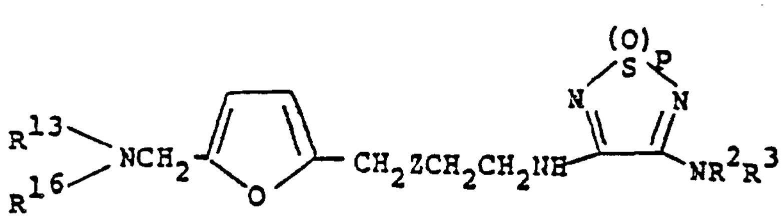

dans laquelle:

p est égal à 1 ou à 2;

Z représente un atome de soufre ou le groupe méthylène;

$R^2$ et $R^3$ représentent chacun indépendamment un atome d'hydrogène ou un radical alkényle en $C_1$ à $C_6$ ou, lorsque $R^2$ représente un atome d'hydrogène, $R^3$ peut également représenter: alkényle en $C_2$ à $C_6$, alkynyle en $C_2$ à $C_6$, phénylalkyle en $C_1$ à $C_6$, cycloalkyle en $C_3$ à $C_7$-alkyle en $C_1$ à $C_6$, pyridylméthyle ou

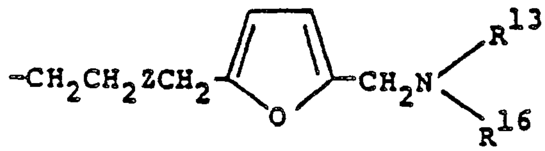

;

$R^{16}$ représente un radical méthyle; et

$R^{13}$ représente un atome d'hydrogène ou le radical méthyle, ou $R^{16}$ et $R^{13}$, pris ensemble avec l'atome d'azote auquel ils sont attachés, peuvent former le groupe pipéridino; ou un de leurs sels, hydratés ou solvatés, non toxiques, pharmaceutiquement acceptables.

6. Une composition selon la revendication 1, dans laquelle le dérivé de formule I présente la structure:

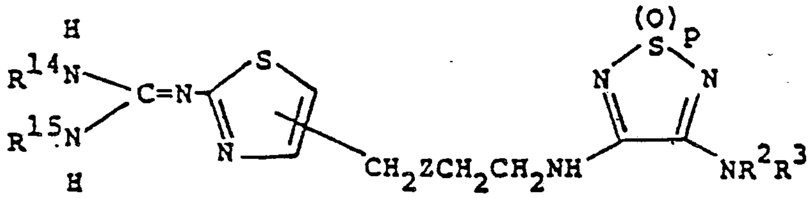

dans laquelle:

p est égal à 1 ou à 2;

Z représente un atome de soufre ou le groupe méthylène;

$R^{14}$ et $R^{15}$ représentent chacun indépendamment un atome d'hydrogène ou le radical méthyle; ou $R^{14}$ et $R^{15}$, lorsqu'ils sont pris ensemble, peuvent former le groupe éthylène; et

$R^2$ et $R^3$ représentent chacun indépendamment un atome d'hydrogène ou le radical alkyle en $C_1$ à $C_6$ ou, lorsque $R^2$ représente un atome d'hydrogène, $R^3$ peut également représenter le groupe alkényle en $C_2$ à $C_6$, alkynyle en $C_2$ à $C_6$, pyridylméthyle, ou

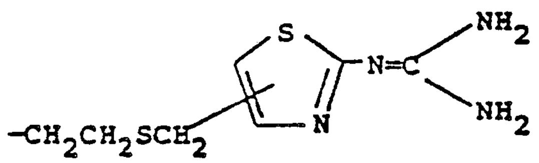

ou

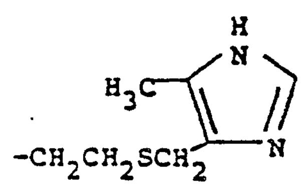

;

ou un de leurs sels, hydratés ou solvatés, non toxiques, pharmaceutiquement acceptables.

7. Une composition selon la revendication 1, dans laquelle le dérivé de formule I présente la structure:

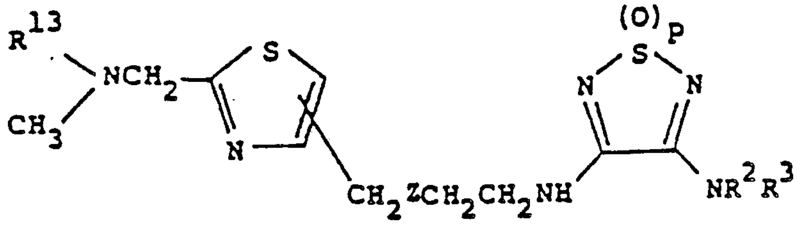

dans laquelle:

p est égal à 1 ou à 2;

Z représente un atome de soufre ou le groupe méthylène;

$R^2$ et $R^3$ représentent chacun indépendamment un atome d'hydrogène ou un radical alkyle en $C_1$ à $C_6$ ou, lorsque $R^2$ représente un atome d'hydrogène, $R^3$ peut également représenter un alkényle en $C_2$ à $C_6$, alkynyle en $C_2$ à $C_6$ ou

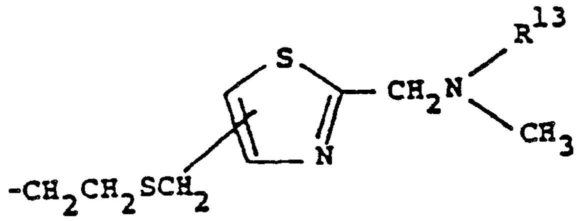 ;

et

$R^{13}$ représente un atome d'hydrogène ou le radical méthyle;

ou un de leurs sels, hydratés ou solvatés, non toxiques, pharmaceutiquement acceptables.

8. Une composition selon la revendication 1, dans laquelle le dérivé de formule I présente la structure:

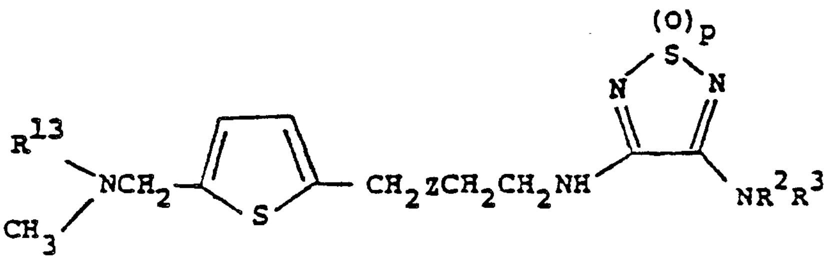

dans laquelle:

p est égal à 1 ou à 2;

Z représente un atome de soufre ou le groupe méthylène;

$R^2$ et $R^3$ représentent chacun indépendamment un atome d'hydrogène ou un radical alkyle en $C_1$ à $C_6$ ou, lorsque $R^2$ représente un atome d'hydrogène, $R^3$ peut également représenter un alkényle en $C_2$ à $C_6$, alkynyle en $C_2$ à $C_6$, phénylalkyle en $C_1$ à $C_6$, pyridylméthyle, 3,4-méthylènedioxybenzyle ou

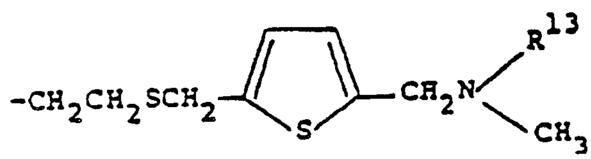 ;

et

$R^{13}$ représente un atome d'hydrogène ou le radical méthyle; ou un de leurs sels, hydratés ou solvatés, non toxiques, pharmaceutiquement acceptables.

9. Une composition selon la revendication 1, dans laquelle le dérivé de formule I présente la structure:

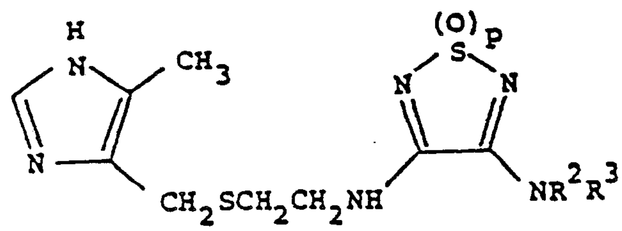

dans laquelle:

p est égal à 1 ou à 2;

$R^2$ et $R^3$ représentent chacun indépendamment un atome d'hydrogène ou un radical alkyle en $C_1$ à $C_6$ ou, lorsque $R^2$ représente un atome d'hydrogène, $R^3$ peut également représenter un alkényle en $C_2$ à $C_6$, alkynyle en $C_2$ à $C_6$ ou

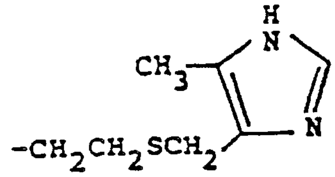

ou un de leurs sels, hydratés ou solvatés, non toxiques, pharmaceutiquement acceptables.

10. Une composition selon la revendication 1, dans laquelle le dérivé de formule I présente la structure:

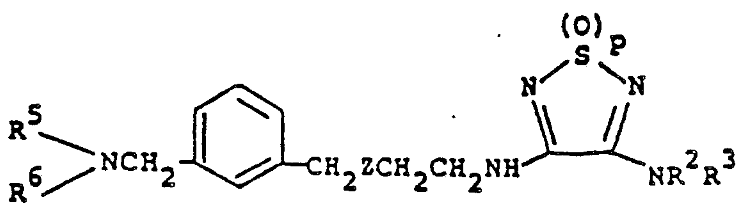

dans laquelle:

p est égal à 1 ou à 2;

Z représente un atome de soufre ou le groupe méthylène;

$R^2$ et $R^3$ représentent chacun indépendamment un atome d'hydrogène ou un radical alkyle en $C_1$ à $C_6$ ou, lorsque $R^2$ représente un atome d'hydrogène, $R^3$ peut également représenter un alkényle en $C_2$ à $C_6$, alkynyle en $C_2$ à $C_6$ ou

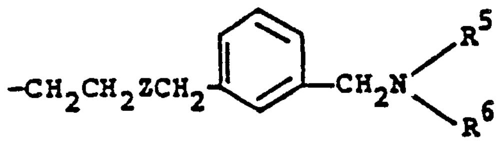 ;

et

$R^5$ et $R^6$ représentent chacun indépendamment un atome d'hydrogène ou un radical alkyle en $C_1$ à $C_6$ ou, $R^5$ et $R^6$, pris ensemble avec l'atome d'azote auquel ils sont attachés, peuvent représenter le groupe pipéridino; ou un de leurs sels, hydratés ou solvatés, non toxiques, pharmaceutiquement acceptables.

11. Une composition selon la revendication 1, dans laquelle le dérivé de formule I présente la structure:

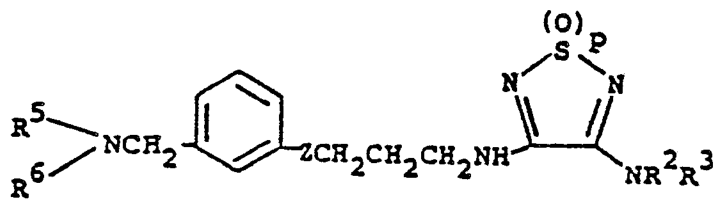

dans laquelle:

p est égal à 1 ou à 2;

Z représente un atome d'oxygène ou de soufre;

$R^2$ et $R^3$ représentent chacun indépendamment un atome d'hydrogène ou un radical alkyle en $C_1$ à $C_6$ ou, lorsque $R^2$ représente un atome d'hydrogène, $R^3$ peut également représenter un alkényle en $C_2$ à $C_6$, alkynyle en $C_2$ à $C_6$, pyridylméthyle ou

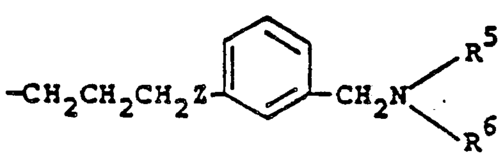 ;

et

$R^5$ et $R^6$ représentent chacun indépendamment un atome d'hydrogène ou un radical alkyle en $C_1$ à $C_6$ ou, lorsque $R^5$ représente un atome d'hydrogène, $R^6$ peut représenter un alkényle en $C_2$ à $C_6$ ou alkynyle en $C_2$ à $C_6$, ou, $R^5$ et $R^6$, pris ensemble avec l'atome d'azote auquel ils sont attachés, peuvent représenter un radical pyrrolidino, méthylpyrrolidino, morpholino, thiomorpholino, pipéridino, méthylpipéridino, diméthylpipéridino, homopipéridino ou heptaméthylèneimino; ou un de leurs sels, hydratés ou solvatés, non toxiques, pharmaceutiquement acceptables.

12. Une composition selon la revendication 1, dans laquelle le dérivé de formule I présente la structure:

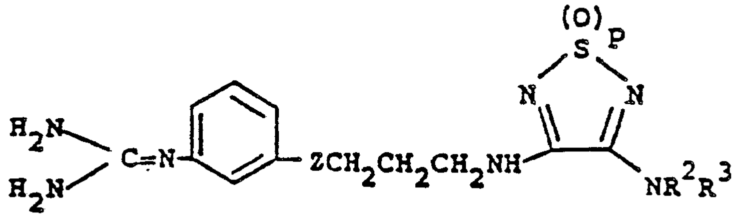

dans laquelle:

p est égal à 1 ou à 2;

Z représente un atome d'oxygène ou de soufre;

$R^2$ et $R^3$ représentent chacun indépendamment un atome d'hydrogène ou un radical alkyle en $C_1$ à $C_6$ ou, lorsque $R^2$ représente un atome d'hydrogène, $R^3$ peut représenter un alkényle en $C_2$ à $C_6$, alkynyle en $C_2$ à $C_6$, pyridylméthyle ou

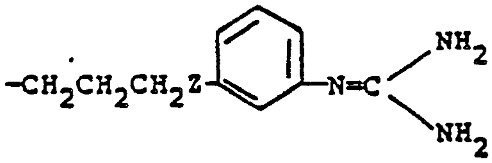

ou un de leurs sels, hydratés ou solvatés, non toxiques, pharmaceutiquement acceptables.

13. Une composition selon l'une quelconque des revendications 1 à 12, dans laquelle la dose de pepstatine est comprise entre 100 et 175 mg et la dose du dérivé de formule I est comprise entre 2 et 100 mg.

14. Une composition selon la revendication 13, dans laquelle la dose du dérivé de formule I est comprise entre environ 4 et 50 mg.

15. Une composition pharmaceutique selon la revendication 13, qui comprend de 100 à 175 mg de pepstatine et de 2 à 15 mg 1-oxyde de 3-amino-4-{2-[(5-diméthylaminométhyl-2-furyl)thiométhyl]aminoéthyl}-1,2,5-thiadiazol ou un de leurs sels d'addition d'acide, hydratés ou solvatés, non toxiques, pharmaceutiquement acceptables.

16. Une composition pharmaceutique selon la revendication 13, qui comprend de 100 à 175 mg de pepstatine et de 2 à 15 mg de 1-oxyde de 3-amino-4-{2-[(2-guanidinothiazol-4-yl)thiométhyl]aminoéthyl}-1,2,5-thiadiazol ou un de leurs sels d'addition d'acide, hydratés ou solvatés, non toxiques, pharmaceutiquement acceptables.

17. Une composition selon les revendications 13 à 16, dans laquelle la pepstatine se trouve sous la forme de minicapsules flottantes de pepstatine.

18. Une composition selon l'une quelconque des revendications 1 à 17, sous forme de dosage unitaire.

19. Une composition selon l'une quelconque des revendications précédentes, comprenant deux parties, la première partie étant constituée d'au moins un dérivé de formule I selon les revendications 1 à 12, 15 et 16, et la deuxième partie comprenant de la pepstatine.

20. Utilisation d'au moins un dérivé de formule I tel que défini dans les revendications 1 à 12, 15 et 16, en association avec de la pepstatine, pour la préparation d'une composition pharmaceutique de traitement des ulcères peptiques.

21. Un procédé de préparation d'une composition selon l'une quelconque des revendicatons 1 à 19, qui consiste à procurer une quantité anti-ulcérogène efficace d'au moins un dérivé de formule I en association avec une quantité de pepstatine inhibitrice de l'activité peptique.

FIG. 1

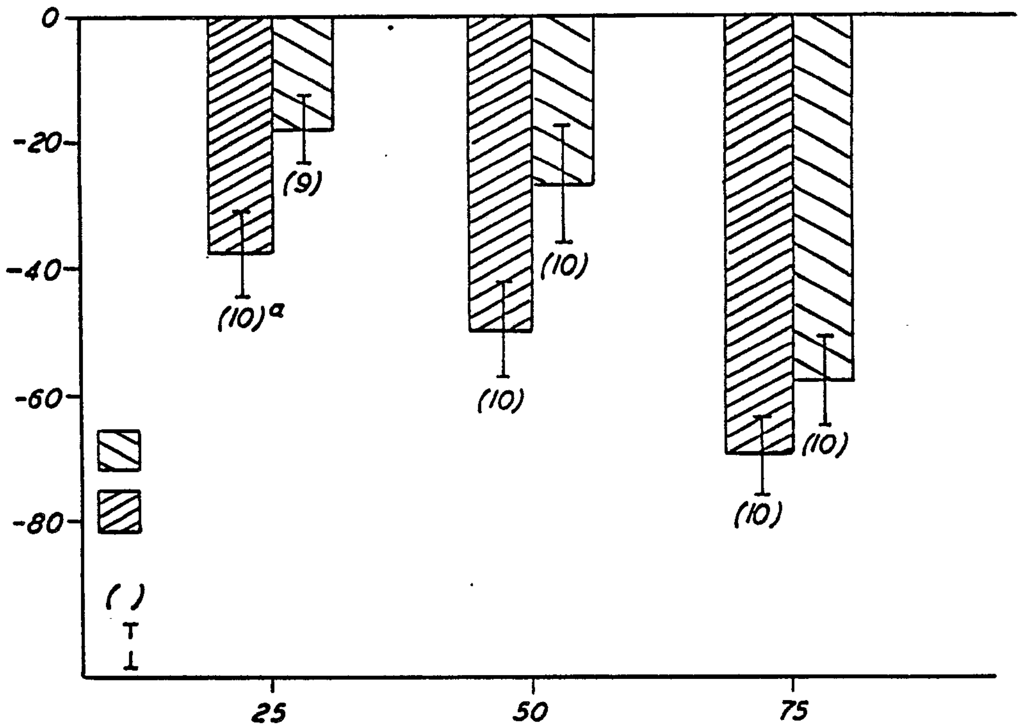

FIG. 2

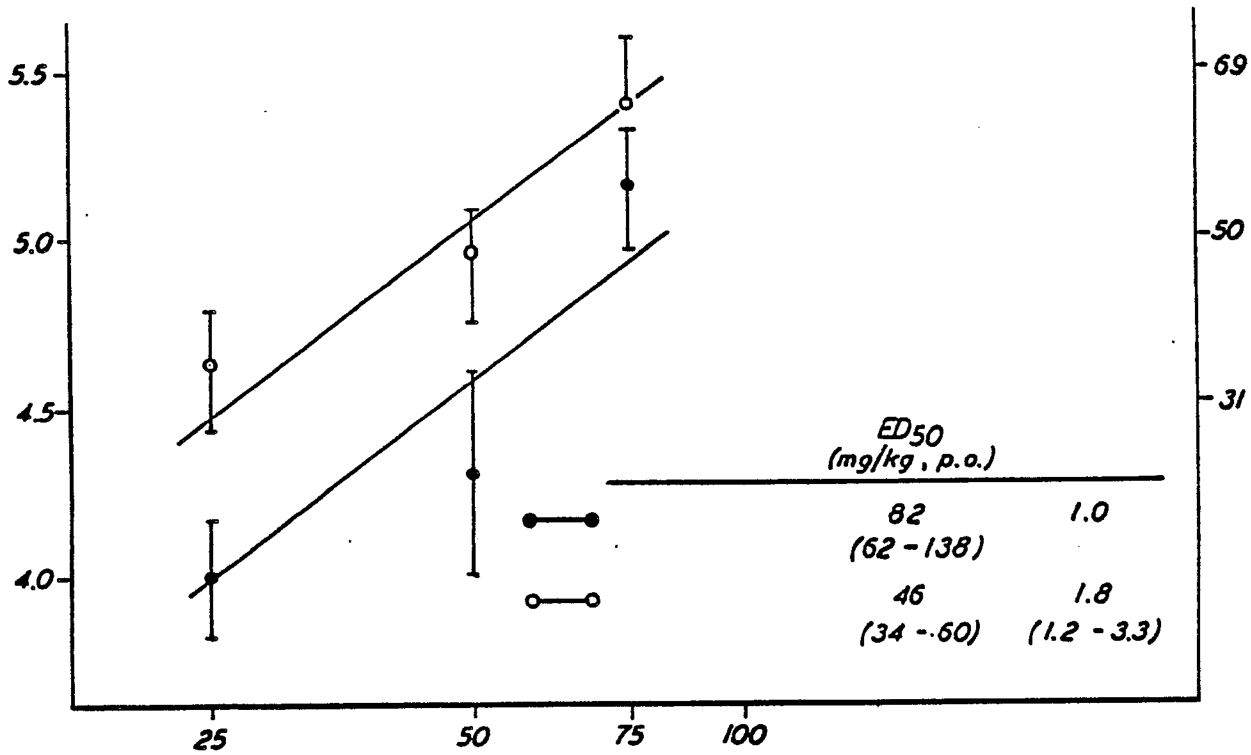
5.5
5.0
4.5
4.0
69
50
31
ED50
(mg/kg , p.o.)
82
(62 - 138)
1.0
46
(34 - 60)
1.8
(1.2 - 3.3)
25
50
75
100

FIG. 3

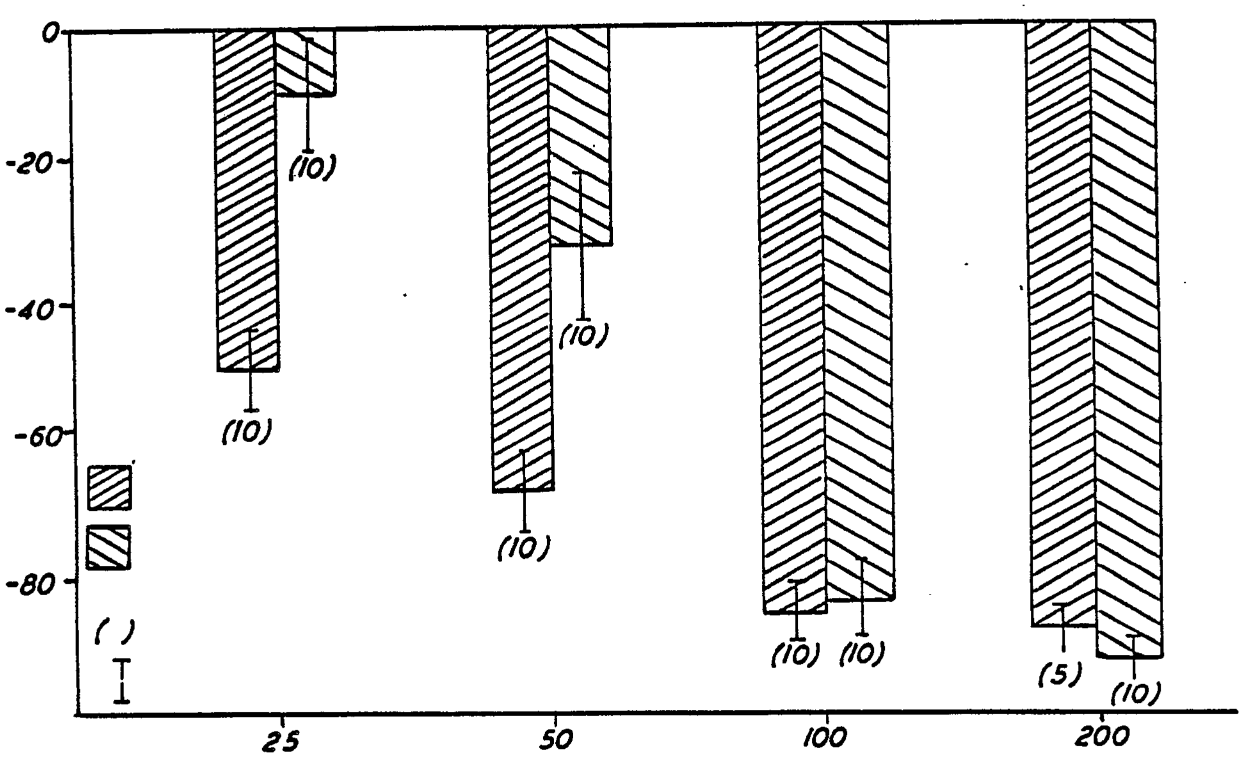

FIG. 4

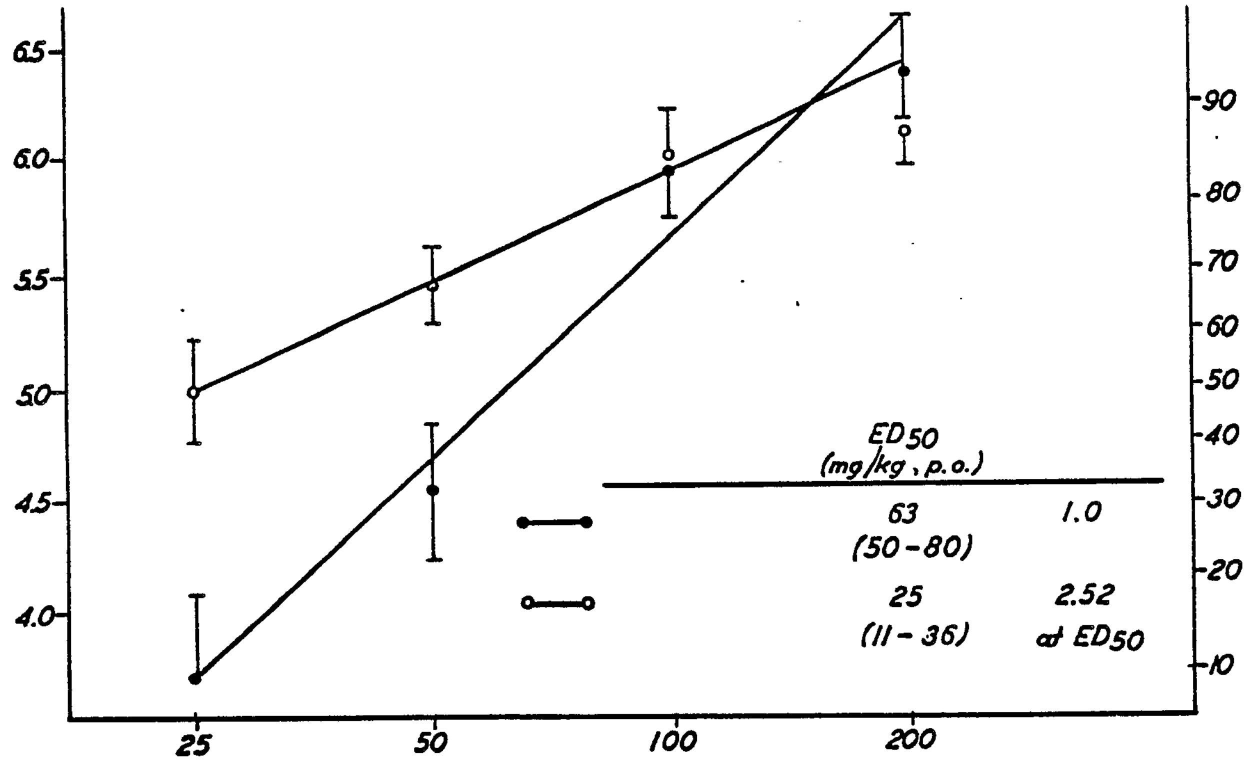
6.5
6.0
5.5
5.0
4.5
4.0
90
80
70
60
50
40
30
20
10
25
50
100
200
ED50
(mg/kg, p.o.)
63
(50-80)
1.0
25
(11-36)
2.52
at ED50

FIG. 5

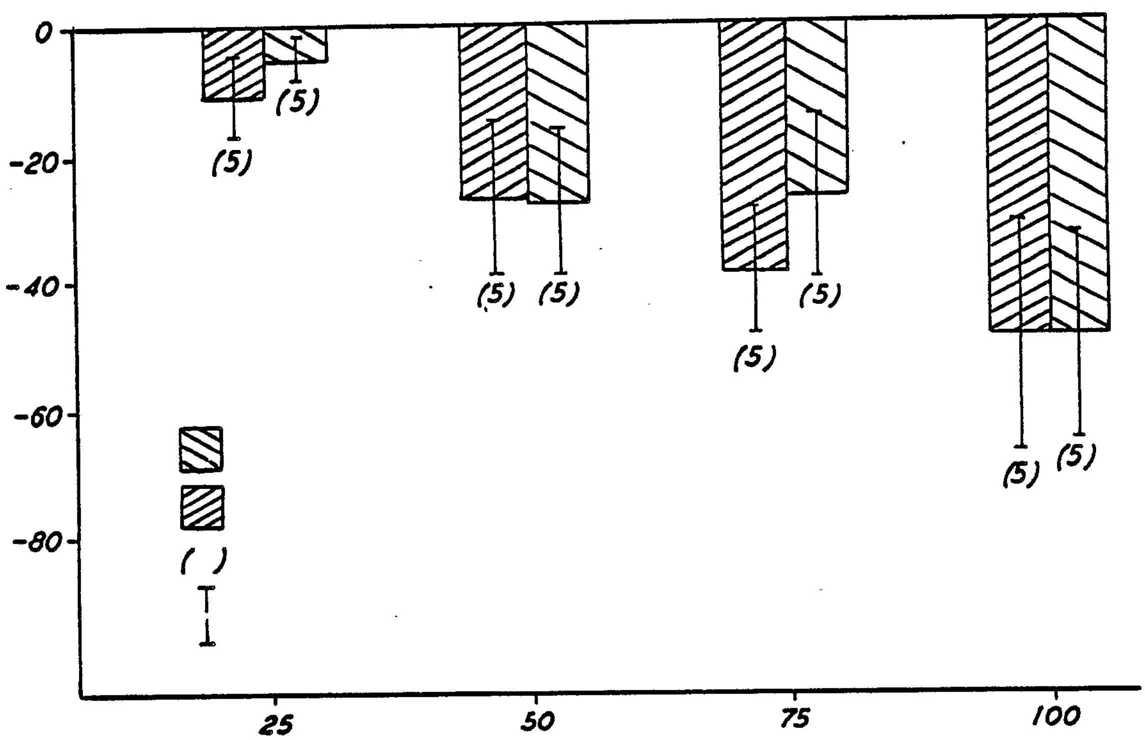

FIG. 6

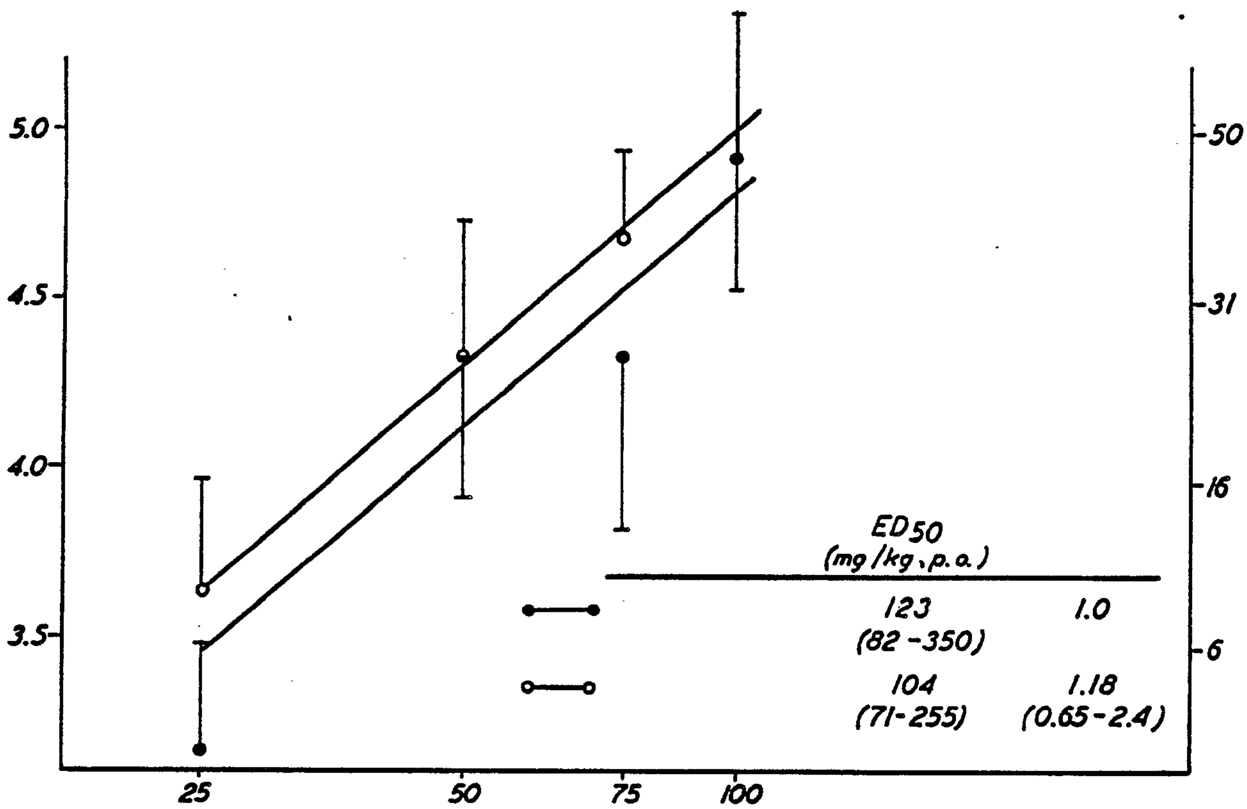
5.0
4.5
4.0
3.5
50
31
16
6
25
50
75
100
ED50
(mg/kg, p.o.)
123
(82-350)
1.0
104
(71-255)
1.18
(0.65-2.4)